(19) Europäisches Patentamt
European Patent Office
Office européen des brevets

(11) EP 2 940 063 A1

(12) **EUROPEAN PATENT APPLICATION**
published in accordance with Art. 153(4) EPC

(43) Date of publication:
**04.11.2015 Bulletin 2015/45**

(21) Application number: **13869648.9**

(22) Date of filing: **26.12.2013**

(51) Int Cl.:
***C08G 77/34*** (2006.01) ***C08G 77/38*** (2006.01)
***A61K 8/89*** (2006.01)

(86) International application number:
**PCT/JP2013/085005**

(87) International publication number:
**WO 2014/104256 (03.07.2014 Gazette 2014/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **28.12.2012 JP 2012289017**

(71) Applicant: **Dow Corning Toray Co., Ltd.**
**Tokyo 100-0004 (JP)**

(72) Inventors:
- **TAMURA Seiki**
**Ichihara-shi**
**Chiba 299-0108 (JP)**
- **SAWAYAMA Sayuri**
**Ichihara-shi**
**Chiba 299-0108 (JP)**
- **HORI Seiji**
**Ichihara-shi**
**Chiba 299-0108 (JP)**

(74) Representative: **Ede, Eric**
**Murgitroyd & Company**
**Scotland House**
**165-169 Scotland Street**
**Glasgow G5 8PL (GB)**

# (54) MANUFACTURING METHOD FOR HIGH-PURITY GLYCEROL DERIVATIVE-MODIFIED SILICONE

(57) The present invention relates to a manufacturing method for a liquid high-purity glycerin derivative-modified silicone, the method comprising:
a step of adding, to a mixture containing a glycerin derivative-modified silicone and impurities, an organic wax having affinity with the impurities and having a higher melting point than the glycerin derivative-modified silicone, melting and mixing while heating, and introducing the impurities into the melted organic wax;
a step of obtaining a solidified product of the organic wax by cooling the organic wax; and
a step of performing solid-liquid phase separation on the glycerin derivative-modified silicone and the solidified product of the organic wax.

With the present invention, it is possible to provide a useful method for stably producing a high-purity glycerin derivative-modified silicone on a commercial scale.

EP 2 940 063 A1

## Description

TECHNICAL FIELD

The present application claims priority on the basis of Patent Application No. 2012-289017, which was filed in Japan on December 28, 2012, the contents of which are incorporated herein by reference.

The present invention relates to a manufacturing method for a high-purity glycerin derivative-modified silicone. Furthermore, the present invention relates to the use of the high-purity glycerin derivative-modified silicone in external use preparations, cosmetics, and various industrial materials.

BACKGROUND ART

The compounds disclosed in Patent Documents 1 to 9 are known examples of silicones modified with glycerin derivatives, and the reaction schemes thereof are also publicly known. In general, there are almost no cases in which the reaction for introducing a glycerin derivative into the silicone backbone progresses in a chemically equivalent (molar equivalent) state, and the introduction reaction is usually completed by charging an excess amount of the glycerin derivative. Accordingly, an unreacted modifier (glycerin derivative) remains in the reaction system in addition to the glycerin derivative-modified silicone copolymer serving as a product. Glycerin derivatives often have a high boiling point, and polyglycerin is also a polymer compound, so purification by stripping is not effective. Therefore, it has been difficult to obtain a high-purity glycerin derivative-modified silicone on a commercial scale. This is due to not only the fact that stripping at an excessively high temperature causes the degeneration of the product or undesirable side reactions, but also the fact that a technique of stripping impurities having a high boiling point at an even higher temperature is inefficient in an actual production process.

Another technique for increasing the purity of an organo-modified silicone containing a residual organic modifier is an extraction (or precipitation/re-precipitation) separation method utilizing the difference in solubility between impurities and the main component. For example, when the organic modifier is a hydrophilic compound, in an extraction separation method, most impurities are first extracted and removed with a hydrophilic solvent (alternatively, the main component is conversely extracted with a lipophilic solvent). However, phase separation in the extraction process ordinarily takes time, and this does not yield clean separation. This results in an increase in waste and a decrease in yield and productivity. In addition, in the case of a glycerin derivative-modified silicone, there are many cases in which the entire system enters an emulsified state and cannot be separated, which leads to poor versatility.

On the other hand, a precipitation and re-precipitation method is a technique of dissolving an organo-modified silicone containing a residual organic modifier in an organic solvent with solubility in both the impurities and the main component, and precipitating and separating the main component by gradually adding water, for example. Patent Document 10 discloses a high-purity polypropylene glycol-modified organosiloxane polymer obtained by a precipitation and re-precipitation method. However, the total amounts of the organic solvent and water that are used in this method are ten times the amount of the organo-modified silicone each time re-precipitation is performed, and this is repeated three times to obtain a high-purity organo-modified silicone with no impurities. Accordingly, taking into consideration problems such as the low productivity in relation to the number of reactions and the large amount of waste water treatment, application to mass production on a commercial scale is difficult. In addition, when the organic modifier is a polyethylene glycol derivative or a glycerin derivative, the hydrophilicity and surface activity performance of the corresponding organo-modified silicone are increased, so separation and purification are often difficult with this method.

Patent Document 11 discloses an organosiloxane derivative having a sugar residue but not containing an unreacted starting material, which is obtained by a membrane separation method using a dialysis tube. However, a dialysis time of three days is required to obtain 10 g of a high-purity organo-modified silicone, so this method cannot be considered suitable for mass production on a commercial scale from the perspective of efficiency. In addition, in Patent Document 11, it is stated that the purification of the organopolysiloxane derivative is also possible by column chromatography. Patent Document 4 discloses a glyceryl ether-modified silicone purified by a silica gel column. However, column chromatography requires the circulation of a large amount of a solvent in order to obtain a high-purity organo-modified silicone, and there are many problems with production on a commercial scale, such as the apparatus design, the recovery of the waste solvent, the removal of the solvent from the recovered solution, and low productivity.

Another example of a technique for increasing the purity of an organo-modified silicone containing a residual organic modifier such as a glycerin derivative is an attempt to improve the transparency of a product by repeating precision filtration or adsorption agent treatment so as to reduce the amount of the residual organic modifier, which is also a cause of turbidity or phase separation. However, this residual organic modifier is ordinarily a liquid in the temperature range in which the organo-modified silicone serving as the main component is in the liquid state, so a technique of solid-liquid separation utilizing a filter aid, a cartridge filter, or the like is not only irrational, but is also mostly ineffective in actuality.

Patent Document 12 discloses a purification method for an alkyl glyceryl polysiloxane derivative by means of

ultrafiltration utilizing a diafiltration method. However, since the pore diameter is small and the film tends to become obstructed in a short amount of time, ultrafiltration needs to be performed after diluting an organo-modified silicone containing an organic modifier around ten times with a volatile solvent such as hexane. Therefore, there are problems such as the removal of the solvent from the filtrate, low productivity, and operator safety.

Patent Document 7 proposes a method for producing a branched polyglycerol-modified silicone obtained by adding/graft polymerizing a silicone having at least one functional group selected from the group consisting of hydroxy groups, carboxy groups, amino groups, imino groups, mercapto groups, and epoxy groups, with 2,3-epoxy-1-propanol in the presence of an acidic or basic catalyst. However, with this method, the siloxane backbone is severed during graft polymerization, and two or more types of components of different nature tend to be produced as a copolymer. There are also many problems from the perspectives of quality and the purification process, and it is difficult to stably obtain a high-purity polyglycerin-modified silicone on a commercial scale.

In addition, Patent Document 13 discloses hydrogenation treatment and subsequent acid treatment on a glycerin-modified polysiloxane in Working Example 5 as a method for purifying a modified silicone compound having a branched polymer consisting of a hydrophilic group. However, this technique is an odor elimination technique for stabilizing the unsaturated base portion of a residual organic modifier, which is a cause of the odor of a modified silicone composition, by means of hydrolysis and hydrogenation treatment, but cannot yield high-purity glycerin-modified silicone. In this technique, the excess glycerin derivative changes the structure thereof and continues to remain in the composition.

Recently, Patent Document 8 has proposed a novel alternating copolymer of organopolysiloxane with polyglycerine derivative, and suggests that a high molecular weight polyglycerine-modified silicone can be obtained without the problem of white turbidness, and the like, caused by the unreacted starting material occurring. However, it is clear from the chemical structure that this compound has a hydrophilic group portion incorporated on its backbone. As a result, this copolymer has properties completely different that those of conventional general-use hydrophilic silicones such as polyether-modified silicone and the like and, therefore, a high level of technical skill is necessary to stably compound this copolymer in delicate formulations such as cosmetic products and the like, leading to the problem of the field of use being limited.

As described above, there were previously practically no known useful methods for stably producing a high-purity glycerin derivative-modified silicone on a commercial scale. Furthermore, there has also been no known technique of increasing the purity of an organo-modified silicone which can be applied regardless of the type of organic modifier and can reasonably accommodate production on a commercial scale.

PRIOR ART DOCUMENT

Patent Document

Patent Document 1: Japanese Examined Patent Application Publication No. S62-34039A

Patent Document 2: Japanese Unexamined Patent Application Publication No. S62-195389A (Japanese Patent No. 2583412B)

Patent Document 3: Japanese Examined Patent Application Publication No. H06-089147 (Japanese Patent No. 1956013B)

Patent Document 4: Japanese Patent No. 2613124B (Japanese Unexamined Patent Application Publication No. H04-188795A)

Patent Document 5: Japanese Patent No. 2844453B (Japanese Unexamined Patent Application Publication No. H02-228958A)

Patent Document 6: Japanese Patent No. 3976226B (Japanese Unexamined Patent Application Publication No. 2002-179798A)

Patent Document 7: Japanese Patent No. 4485134B (Japanese Unexamined Patent Application Publication No. 2004-339244A)

Patent Document 8: Japanese Patent No. 5037782B (Japanese Unexamined Patent Application Publication No. 2005-042097A)

Patent Document 9: Japanese Patent No. 4357909B (Japanese Unexamined Patent Application Publication No. 2005-089494A)

Patent Document 10: Japanese Unexamined Patent Application Publication No. S63-202629A

Patent Document 11: Japanese Patent No. 3172787B (Japanese Unexamined Patent Application Publication No. H05-186596A)

Patent Document 12: Japanese Unexamined Patent Application Publication No. H05-156019A

Patent Document 13: WO/2002/055588

Patent Document 14: WO/2011/049248
Patent Document 15: WO/2011/049247
Patent Document 16: WO/2011/049246
Patent Document 17: Japanese Unexamined Patent Application Publication No. 2012-046507A

SUMMARY OF INVENTION

Technical Problem

The present invention was conceived in light of the problems described above, and an object thereof is to provide a technique for increasing the purity of a glycerin derivative-modified silicone which can be applied regardless of the type of the organic modifier and can reasonably accommodate production on a commercial scale.
In particular, an object of the present invention is to provide a method for stably producing a high-purity glycerin derivative-modified silicone on a commercial scale, even when the boiling point of the glycerin derivative serving as an organic modifier is high or the molecular weight of the glycerin derivative is large.
In addition, another object of the present invention is to use a high-purity glycerin derivative-modified silicone produced with such a method in external use preparations, cosmetics, or various industrial materials.

Solution to Problem

The object of the present invention can be achieved by a method of producing a liquid high-purity glycerin derivative-modified silicone, the method comprising:

a step of adding, to a mixture containing a glycerin derivative-modified silicone and impurities, an organic wax having affinity with the impurities and having a higher melting point than the glycerin derivative-modified silicone, melting and mixing while heating, and introducing the impurities into the melted organic wax; a step of obtaining a solidified product of the organic wax by cooling the organic wax; and
a step of performing solid/liquid phase separation on the glycerin derivative-modified silicone and the solidified product of the organic wax.

The impurities are preferably impurities originating from the glycerin derivative.
The glycerin derivative-modified silicone is preferably a liquid at least at a temperature of 100°C.
The organic wax preferably has a melting point of from 45°C to 150°C.
The organic wax preferably has an average molecular weight of at least 900.
The organic wax preferably has a (poly)oxyethylene site.
The organic wax is preferably a glycerin derivative containing a (poly)oxyethylene site.
The silicon atoms of the glycerin derivative-modified silicone can bond with glycerin derivative group-containing organic groups via Si-C bonds or Si-O-C bonds.
The glycerin derivative-modified silicone can be expressed by the following general formula (1):

[Formula 1]

$$R^1_aR^2_bL^1_cQ_dSiO_{(4-a-b-c-d)/2} \qquad (1)$$

(wherein $R^1$ represents a monovalent organic group (however, excluding $R^2$, L, and Q), a hydrogen atom or a hydroxyl group; and $R^2$ is a substituted or unsubstituted, straight or branched monovalent hydrocarbon group having 9 to 60 carbon atoms, or the chain organosiloxane group represented by the following general formula (2-1):

[Formula 2]

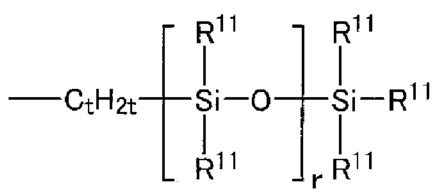
(2-1)

(wherein $R^{11}$ are each independently a substituted or unsubstituted monovalent hydrocarbon group having from 1 to 30 carbons, hydroxyl groups, or hydrogen atoms and at least one of the $R^{11}$ moieties is the monovalent hydrocarbon group; t is a number in a range of 2 to 10; and r is a number in a range of 1 to 500); or the general formula (2-2) below:

[Formula 3]

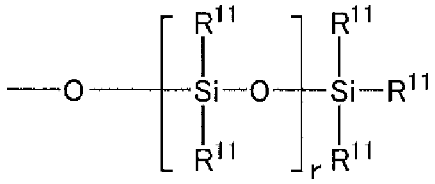

(2-2)

(wherein, $R^{11}$ and r are synonymous with those described above); and $L^1$ represents a silylalkyl group having a siloxane dendron structure expressed by the following general formula (3) when i=1;

[Formula 4]

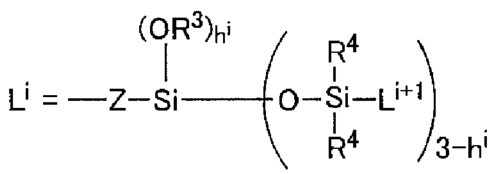

(3)

(wherein, $R^3$ each independently represents a substituted or unsubstituted, straight or branched monovalent hydrocarbon group having 1 to 30 carbons; $R^4$ each independently represents an alkyl group or phenyl group having 1 to 6 carbon atoms; Z represents a divalent organic group; i represents a generation of the silylalkyl group represented by $L^i$ and is an integer of 1 to k when k is a number of generations that is a number of repetitions of the silylalkyl group; the number of generations k is an integer from 1 to 10; $L^{i+1}$ is the silylalkyl group when i is less than k, and $R^4$ when i=k, and $h^i$ is a number in a range from 0 to 3); Q represents a glycerin derivative group-containing organic group; and a, b, c, and d are each numbers in the ranges of $1.0 \le a \le 2.5$, $0 \le b \le 1.5$, $0 \le c \le 1.5$, and $0.0001 \le d \le 1.5$.

The glycerin derivative-modified silicone may be an organo-modified silicone obtained by reacting:

(A) an organohydrogenpolysiloxane;
(B) a glycerin derivative group-containing organic compound having one or more reactive unsaturated groups in each molecule; and
(C) one or more types of organic compounds selected from the group consisting of (C1) an organic compound having a number of reactive unsaturated groups greater than 1 on average in each molecule and (C2) an organic compound having one or more reactive unsaturated groups and one or more epoxy groups in each molecule (however, the use of the component (B) is optional when the component (C) contains a glycerin derivative group-containing organic group); the glycerin derivative-modified silicone having a silicon-bonded glycerin derivative group-containing organic group and having a crosslinked structure containing a Si-C bond in a crosslinking portion.

The glycerin derivative-modified silicone may be a straight-chain glycerin derivative group-containing alternating copolymer obtained by reacting at least:

(D) an organopolysiloxane having reactive functional groups at both terminals of a molecular chain, or a derivative thereof; and
(E) an organic compound having two reactive functional groups capable of reacting with the reactive functional groups positioned at both of the molecular chain terminals of (D) in the molecule.

In the present invention, the mixture may further contain a solvent of the glycerin derivative-modified silicone.
In addition, in the present invention, the mixture containing the glycerin derivative-modified silicone and the impurities are preferably treated by an acidic aqueous solution, and water and odor-causing substances produced by treatment with the acidic aqueous solution and water are preferably removed by heating or depressurization.
In addition, the object of the present invention is also achieved by an external use preparation, a cosmetic, or

an industrial material containing a high-purity glycerin derivative-modified silicone obtained by the manufacturing method of the present invention.

ADVANTAGEOUS EFFECTS OF INVENTION

The manufacturing method for a high-purity glycerin derivative-modified silicone according to the present invention can be applied regardless of the type of the organic modifier and can reasonably accommodate production on a commercial scale.

In particular, the present invention can stably produce a high-purity glycerin derivative-modified silicone on an industrial scale even when the boiling point of the organic modifier (glycerin derivative), which is difficult to purify by distillation, is high or the organic modifier (glycerin derivative) is a polymer compound.

In addition, when the mixture contains a solvent of the glycerin derivative-modified silicone, a solution of a high-purity glycerin derivative-modified silicone can be produced easily, and the production of this solution has excellent yield and productivity, so the method is also suitable for production on a commercial scale.

The high-purity glycerin derivative-modified silicone obtained by the manufacturing method of the present invention substantially consists of a single component from which impurities - in particular, impurities originating from the organic modifier - have been removed, so phase separation, precipitation of the unreacted starting material, or the like does not occur after production. Therefore, the composition is chemically and physically stable.

In addition, a high-purity glycerin derivative-modified silicone produced by the present invention or a solution containing the same can be suitably used in external use preparations or cosmetics and can further be used widely in various industrial materials.

DETAILED DESCRIPTION OF THE INVENTION

A first aspect of the present invention is a manufacturing method for a liquid high-purity glycerin derivative-modified silicone, the method comprising:

> a step of adding, to a mixture containing a glycerin derivative-modified silicone and impurities, an organic wax having affinity with the impurities and having a higher melting point than the glycerin derivative-modified silicone, melting and mixing while heating, and introducing the impurities into the melted organic wax; a step of obtaining a solidified product of the organic wax by cooling the organic wax; and a step of performing solid/liquid phase separation on the glycerin derivative-modified silicone and the solidified product of the organic wax.

The manufacturing method of the present invention is characterized in that impurities - in particular, impurities originating from an organic modifier (glycerin derivative) - are dissolved in a heated and melted organic wax, and the organic wax is then solidified by cooling while the impurities remain inside the organic wax. On the other hand, the glycerin derivative-modified silicone is separated from impurities by utilizing the principle that the glycerin derivative-modified silicone is incompatible with the organic wax and remains as a fluid due to its low melting point.

The first aspect of the present invention will be described in detail hereinafter.

<Manufacturing method for high-purity glycerin derivative-modified silicone>

[Organic wax] Any organic wax having affinity with impurities - in particular, impurities originating from the organic modifier (glycerin derivative) - and having a higher melting point than the glycerin derivative-modified silicone can be used as the organic wax used in the present invention. The organic wax of the present invention does not contain silicon atoms in its molecular structure. When a solid-liquid separation operation such as filtration is performed at room temperature, the melting point of the organic wax is discretional but is preferably at least 45°C. Specifically, the organic wax has a melting point of preferably from 45°C to 150°C, more preferably from 50°C to 120°C, and even more preferably from 60°C to 100°C and has a number average molecular weight of preferably at least 900, more preferably at least 2,000, even more preferably at least 5,000, and particularly preferably from 6,000 to 50,000. When the melting point of the organic wax is lower than 45°C, the melting point of the solid that is produced by cooling after the impurities originating from the organic modifier are introduced becomes even lower, in particular, so in order to perform solid-liquid separation on the solid and the glycerin derivative-modified silicone serving as the main component, it is necessary to perform filtration at a temperature of at most 40°C. Filtration at such a low temperature tends to cause an increase in filtration time when the glycerin derivative-modified silicone is a high-viscosity organo-modified silicone, which leads to a risk that the production efficiency may decrease. In addition, a wax with a melting point lower than 45°C typically has a low capacity to be solidified by introducing impurities, and the solid-liquid separability also tends to be poor. In general, the filtration speed may be low in low-temperature filtration, but filtration may also be performed at around 0°C or at an even

lower temperature by diluting the composition with a solvent such as hexane so as to reduce the viscosity. In addition, the impurity removing effect may be enhanced by performing filtration at a low temperature and aggressively precipitating the solid in accordance with the desired quality, the type of impurities, and the like. On the other hand, when the melting point of the organic wax is higher than 150°C, a larger amount of energy is required to melt the wax, which is not preferable from the perspective of the environment or efficiency. In addition, at a temperature exceeding 150°C, the glycerin derivative-modified silicone itself also typically tends to be diminished, which is not preferable.

Furthermore, when the molecular weight of the organic wax is less than 900, the organic wax tends to become easily compatible with not only impurities originating from the organic modifier, for example, but also with the glycerin derivative-modified silicone serving as the main component which is modified with the organic modifier, and as a result, the added organic wax blends into the main component, which may make solid-liquid separation difficult. On the other hand, an upper limit is not particularly established for the molecular weight of the organic wax but is ordinarily at most ten million. A high-molecular-weight organic wax may require a special catalyst, equipment, or the like to produce, which may be problematic from the perspective of supply or cost. Therefore, it is preferable to use a composition with a molecular weight of at most 50,000, which is easily procured.

In particular, when the glycerin derivative-modified silicone contains a (poly)oxyethylene site in the molecule, the organic wax preferably has a (poly)oxyethylene site. In addition, even when the glycerin derivative-modified silicone does not contain a (poly)oxyethylene site in the molecule, the organic wax preferably has a (poly)oxyethylene site and particularly preferably also has a glycerin derivative site. Examples of organic waxes suitable for such cases include polyethylene glycol (PEG) or polyethylene oxide (PEO) which satisfies the conditions related to the melting point and molecular weight, or a compound having a structure of a form in which one or both of the terminal hydroxyl groups thereof are capped with a given sequestering agent. Examples of terminal capping groups include but are not limited to methyl groups, ethyl groups, propyl groups, butyl groups, pentyl groups, hexyl groups, heptyl groups, octyl groups, alkyl groups with even longer chains; cycloalkyl groups such as cyclopentyl groups and cyclohexyl groups; alkenyl groups such as vinyl groups, allyl groups, and butenyl groups; aryl groups such as phenyl groups and tolyl groups; monovalent hydrocarbon groups as typified by aralkyl groups such as benzyl groups; acyl groups such as acetyl groups and benzoyl groups; groups in which the hydrogen atoms bonded to the carbon atoms of these groups are at least partially substituted with organic groups containing hetero atoms; and trimethylsilyl groups. In addition, the organic wax may contain other (poly)oxyalkylene chains or (poly)glycerin chains in addition to the (poly)oxyethylene chain within a range that does not diminish the effect of the present invention. Furthermore, the organic wax may be a compound of a form in which multiple ethylene oxides are addition-polymerized with various polyhydric alcohols or a compound using such a compound as a base. That is, the most suitable type of organic wax is one which satisfies the conditions related to the melting point and molecular weight and has a polyoxyethylene chain and a glycerin unit. Examples include a hydrophilic wax obtained by addition-polymerizing multiple ethylene oxides with glycerin, a hydrophilic wax obtained by addition-polymerizing multiple ethylene oxides with diglycerin, and a hydrophilic wax obtained by addition-polymerizing multiple ethylene oxides with triglycerin. The next most suitable types of organic waxes are polyethylene glycol (PEG) or polyethylene oxide (PEO) of a structure in which multiple ethylene oxides are subjected to homopolymerization.

When the glycerin derivative-modified silicone contains both a (poly)oxyethylene site and a (poly)oxypropylene site in the molecule or has a structure not containing a (poly)oxyalkylene site other than the (poly)oxypropylene site, the organic wax preferably has structural units in which a (poly)oxyethylene site and a (poly)oxypropylene site are connected in blocks. These blocks may repeat or may form a non-repeating AB-type or ABA-type block copolymer. Examples of organic waxes suitable for such cases include a polyethylene glycol (PEG)/polypropylene glycol (PPG) copolymer or polyethylene oxide (PEO)/polypropylene glycol (PPG) copolymer which satisfies the conditions related to the melting point and molecular weight, or a compound having a structure of a form in which one or both of the terminal hydroxyl groups thereof are capped with a given sequestering agent. Examples of terminal capping groups include but are not limited to those described above. In addition, the organic wax may contain other (poly)oxyalkylene sites or (poly)glycerin sites in addition to the (poly)oxyethylene site and the (poly)oxypropylene site within a range that does not diminish the effect of the present invention. Furthermore, the organic wax may be a compound of a form in which ethylene oxides and propylene oxides are addition-polymerized with various polyhydric alcohols in blocks or a compound using such a compound as a base.

A suitable amount of the organic wax that is used is from 0.5 to 10 wt.% and more preferably from 1 to 5 wt.% with respect to the glycerin derivative-modified silicone serving as the main component. At less than 0.5 wt.%, the effect of removing impurities is often insufficient. When the amount used exceeds 10 wt.%, it is not only economically disadvantageous, but the filter efficiency or yield also decreases, and there is often waste from the perspective of the impurity removing effect.

A reaction mixture containing a glycerin derivative-modified silicone as a main component and impurities - in particular, components originating from a glycerin derivative (organic modifier) serving as one of the starting materials of the glycerin derivative-modified silicone - as impurities has affinity with the impurities, and the organic wax having a higher melting point than the glycerin derivative-modified silicone is added, heated, melted, and mixed. Mixing is preferably

performed using mechanical power. For example, mixing can be performed with a paddle mixer, a propeller mixer, or in a reaction vessel or a container equipped with mixing blades, and an emulsifier, a kneader, or the like may also be used as necessary. In addition, the mixing of both components needs to be performed at a temperature equal to or higher than the temperature at which the organic wax that is used melts, and from the perspective of dissolving and introducing the impurities into the melted wax, it is preferably performed sufficiently so that the entire composition is thoroughly mixed. At this time, when treatment is performed by adding a solvent which is a good solvent for the glycerin derivative-modified silicone and a poor solvent for the impurities, the viscosity of the system decreases, so the contact between the impurities and the organic wax component occurs efficiently, and as a result, the introduction of impurities by means of the organic wax (that is, the increase in the purity of the glycerin-modified silicone) can be accelerated. Ordinarily, mixing and stirring should be performed for 10 minutes to 5 hours and preferably from 30 minutes to 2 hours in a range of from 45 to 150°C and preferably from 70 to 120°. Treatment can be completed in a shorter amount of time when the capacity of the mixing stirrer is higher, but the treatment conditions can be set out of consideration of the energy cost such as the power consumption. The mixture is then left to cool or is cooled so as to be integrally solidified (preferably as solid particles) while impurities remain in the wax, whereas the glycerin derivative-modified silicone serving as the main component in the system is incompatible with the organic wax and remains as a fluid due to low melting point. In this cooling process, the stirring and mixing operation may or may not be performed. It is also possible, in principle, to perform the mixing operation and the like using human or animal power, but this is not advantageous from the perspective of stable production or efficiency on an industrial scale.

The mixture consisting of the glycerin derivative-modified silicone fluid and solid particles obtained by the treatment process described above can be subjected to liquid-solid separation by means of a common filtration operation with filter paper using diatomaceous earth, activated carbon, or the like, as a filter aid, for example. This makes it possible to easily obtain a high-purity glycerin derivative-modified silicone. When a solvent which is a good solvent for the glycerin derivative-modified silicone and a poor solvent for the impurities is used in the treatment process, a mixture consisting of the glycerin derivative-modified silicone fluid, the solid particles, and the solvent is subjected to solid-liquid separation by means of a common filtration operation with filter paper using diatomaceous earth, activated carbon, or the like, as a filter aid, for example. When the solvent can be used as an oil agent for a cosmetic, for example, the filtrate can be used as a cosmetic starting material containing a high-purity glycerin derivative-modified silicone and an oil agent and formed into a product directly. On the other hand, when a volatile substance is used as the solvent, a high-purity glycerin derivative-modified silicone can be obtained by removing the volatile solvent by means of a heating and depressurization operation or the like from the filtrate after solid-liquid separation. In general, glycerin derivative-modified silicones have high viscosity, so performing treatment with the organic wax in the presence of the solvent is advantageous for an increase in purity or a decrease in turbidity of the glycerin derivative-modified silicone.

[Glycerin derivative-modified silicone] The glycerin derivative-modified silicone to which the present invention can be applied is a silicone compound modified with a glycerin derivative and is a liquid composition, and it is preferably a liquid at least at a temperature of 100°C. The chemical structure or the like is not particularly limited as long as the composition satisfies this condition.

In the present invention, a "liquid form" or a "liquid" means that after the liquid surface of an organopolysiloxane in a prescribed container is placed horizontally and the vessel is then inclined, the liquid surface can once again become horizontal after 1 hour, preferably after 30 minutes, and more preferably after 10 minutes. Here, "horizontal" means to form a plane that intersects the direction of gravitational force at a right angle. The glycerin derivative-modified silicone is preferably a liquid at least at 100°C but more preferably also exhibits liquidity in a range from 100°C or less to room temperature. Specifically, the glycerin derivative-modified silicone is preferably a liquid at 80°C, more preferably a liquid at 40°C, and even more preferably a liquid at room temperature (25°C). Compositions with liquidity at a temperature of 100°C or higher are, of course, included in the scope of liquid organic silicon compounds, but even compounds which are in a semi-gelatinous form or a soft solid form without fluidity at room temperature (25°C) or lower but demonstrate liquidity when heated to 100°C, for example, are also included.

The glycerin derivative-modified silicone can be expressed by the following general formula (1):

[Formula 5]

$$R^1_aR^2_bL^1_cQ_dSiO_{(4-a-b-c-d)/2} \qquad (1)$$

(wherein $R^1$ represents a monovalent organic group (however, excluding $R^2$, L, and Q), a hydrogen atom or a hydroxyl group; and $R^2$ is a substituted or unsubstituted, straight or branched monovalent hydrocarbon group having 9 to 60 carbon atoms, or the chain organosiloxane group represented by the following general formula (2-1):

[Formula 6]

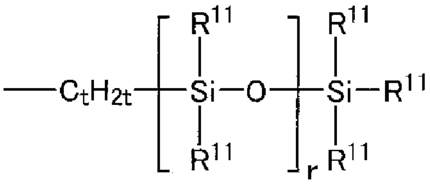
(2-1)

(wherein $R^{11}$ are each independently a substituted or unsubstituted monovalent hydrocarbon group having from 1 to 30 carbons, hydroxyl groups, or hydrogen atoms and at least one of the $R^{11}$ moieties is the monovalent hydrocarbon group;t is a number in a range of 2 to 10; and r is a number in a range of 1 to 500); or the general formula (2-2) below:

[Formula 7]

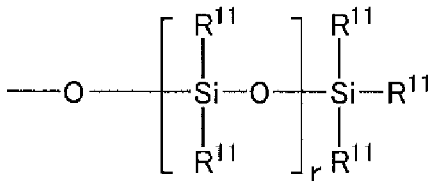
(2-2)

(wherein, $R^{11}$ and r are synonymous with those described above); and $L^1$ represents a silylalkyl group having a siloxane dendron structure expressed by the following general formula (3) when i=1;

[Formula 8]

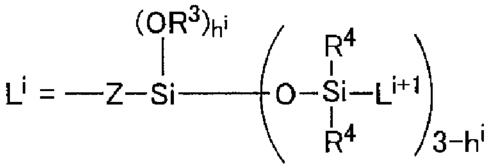
(3)

(wherein, $R^3$ each independently represents a substituted or unsubstituted, straight or branched monovalent hydrocarbon group having 1 to 30 carbon atoms; $R^4$ each independently represents an alkyl group or phenyl group having 1 to 6 carbon atoms; Z represents a divalent organic group; i represents a generation of the silylalkyl group represented by $L^i$ and is an integer of 1 to k when k is a number of generations that is a number of repetitions of the silylalkyl group; the number of generations k is an integer from 1 to 10; $L^{i+1}$ is the silylalkyl group when i is less than k, and $R^4$ when i=k, and $h^i$ is a number in a range from 0 to 3); Q represents a glycerin derivative group-containing organic group; and a, b, c, and d are each numbers in the ranges of $1.0 \le a \le 2.5$, $0 \le b \le 1.5$, $0 \le c \le 1.5$, and $0.0001 \le d \le 1.5$.

Here, when the glycerin derivative-modified silicone represented by general formula (1) has the long chain organic group or the chain organosiloxane group represented by $R^2$, b is a number greater than 0, preferably $0.0001 \le b \le 1.5$, and more preferably $0.001 \le b \le 1.5$. Similarly, when the glycerin derivative-modified silicone represented by general formula (1) has a silylalkyl group having the siloxane dendron structure represented by $L^1$, c is a number greater than 0, preferably $0.0001 \le c \le 1.5$, and more preferably $0.001 \le c \le 1.5$.

The glycerin derivative-modified silicone preferably has a long-chain organic group or a chain organosiloxane group represented by $R^2$ or a siloxane dendron structure represented by $L^1$ together with the glycerin derivative group-containing organic group serving as Q. At this time, the suitable values of b and c are expressed as follows by essential functional groups.

(1) When there is a group represented by $R^2$: $0.001 \le b \le 1.5$ and $0 \le c \le 1.5$.
(2) When there is a group represented by $L^1$: $0 \le b \le 1.5$ and $0.001 \le c \le 1.5$.
(3) When there are both a group represented by $R^2$ and a group represented by $L^1$: $0.001 \le b \le 1.5$ and $0.001 \le c \le 1.5$.

The monovalent groups represented by $R^1$ in general formula can be the same or different and are not particularly

limited as long as they are not the functional groups of $R^2$, $L^1$, and Q. However, they are preferably a substituted or unsubstituted, straight-chain or branched monovalent hydrocarbon group having from 1 to 8 carbon atoms, a (poly)oxyalkylene group represented by $-R^5O(AO)_nR^6$ (in the formula, AO represents an oxyalkylene group having from 2 to 4 carbon atoms; $R^5$ represents a substituted or unsubstituted, straight-chain or branched divalent hydrocarbon group having from 3 to 5 carbon atoms; $R^6$ represents a substituted or unsubstituted, straight-chain or branched monovalent hydrocarbon group having from 1 to 24 carbon atoms and hydrogen atoms or a substituted or unsubstituted, straight-chain or branched acyl group having from 2 to 24 carbon atoms; and n is from 1 to 100), an alkoxy group, a hydroxyl group, or a hydrogen atom. However, not all of the $R^1$ moieties are hydroxyl groups, hydrogen atoms, alkoxy groups, or (poly)oxyalkylene groups.

Examples of a monovalent hydrocarbon group having 1 to 8 carbon atoms are, for example, alkyl groups such as a methyl group, ethyl group, propyl group, butyl group, pentyl group, hexyl group, heptyl group, octyl group, and the like; cycloalkyl groups such as a cyclopentyl group, cyclohexyl group, and the like; alkenyl groups such as a vinyl group, allyl group, butenyl group, and the like; aryl groups such as a phenyl group, tolyl group, and the like; aralkyl groups such as a benzyl group; and groups wherein the hydrogen atoms bonded to the carbon atoms of these groups are substituted at least partially by fluorine or a similar halogen atom, or an organic group having an epoxy group, a glycidyl group, an acyl group, a carboxyl group, an amino group, a (meth)acryl group, a mercapto group, or the like (however, the total number of carbon atoms is from 1 to 8). The monovalent hydrocarbon group is preferably a group other than an alkenyl group, and is particularly preferably a methyl group, an ethyl group, or a phenyl group. Additionally, examples of the alkoxy group include a methoxy group, an ethoxy group, an isopropoxy group, a butoxy group, and similar lower alkoxy groups; a lauryl alkoxy group, a myristyl alkoxy group, a palmityl alkoxy group, an oleyl alkoxy group, a stearyl alkoxy group, a behenyl alkoxy group, and similar higher alkoxy groups; and the like.

Particularly, the $R^1$ moieties are preferably monovalent hydrocarbon groups having from 1 to 8 carbon atoms and that are free of unsaturated aliphatic bonds or monovalent fluorinated hydrocarbon groups. Examples of the monovalent hydrocarbon group not having unsaturated aliphatic bonds belonging to the $R^1$ moiety include methyl groups, ethyl groups, propyl groups, butyl groups, pentyl groups, hexyl groups, and similar alkyl groups; phenyl groups, tolyl groups, xylyl groups, and similar aryl groups; and aralkyl groups such as benzyl groups. Examples of the monovalent fluorinated hydrocarbon group include trifluoropropyl groups, pentafluoroethyl groups, and similar perfluoroalkyl groups. From an industrial perspective, $R^1$ is preferably a methyl group, an ethyl group, or a phenyl group, and more preferably from 90 mol% to 100 mol% of all the $R^1$ moieties are selected from methyl groups, ethyl groups, or phenyl groups.

A glycerin derivative-modified silicone aims at imparting additional functionality, and it is possible to introduce or design a modified group other than a hydrophilic group (-Q), particularly a short chain or medium chain hydrocarbon based group, as $R^1$. Specifically, when $R^1$ is a substituted monovalent hydrocarbon group, a substituent can be preferably selected in accordance with desired characteristics and uses. For example, when using the glycerin derivative-modified silicone as a cosmetic composition or a fiber treating agent starting material, it is possible to introduce an amino group, amide group, aminoethyl aminopropyl group, carboxyl group, and the like, as the substituted group of a monovalent hydrocarbon group, for the purpose of improving the sensation during use, feeling to touch, persistence, and the like.

The substituted or unsubstituted, straight or branched monovalent hydrocarbon group having 9 to 60 carbon atoms of $R^2$ of general formula (1) is a long chain hydrocarbon group or a chain organosiloxane group represented by general formula (2-1) or (2-2). By introducing this group at the main chain and/or side chain of polysiloxane, it is possible to further improve the affinity, emulsifiability, and dispersibility, and further the sensation during use of various components such as an oil agent, powder, or the like incorporated in an external use preparation or a cosmetic composition. Furthermore, because the monovalent long chain hydrocarbon group or chain organopolysiloxane group is a hydrophobic functional group, the compounding stability and the compatibility with organic oils having a high content of alkyl groups are further improved. $R^2$ may be all the monovalent long chain hydrocarbon group or all the chain organopolysiloxane group, or may be a functional group of both of these groups. In the glycerin derivative-modified silicone, it is particularly preferable that part or all of $R^2$ is a monovalent long chain hydrocarbon group, and by having such a monovalent long chain hydrocarbon group in a molecule, the glycerin derivative-modified silicone exhibits more superior compatibility not only with silicone oil, but with non silicone oil with a high alkyl group content as well. For example, it is possible to obtain an emulsion and a dispersion with superior stability over time and thermal stability, which are made of non silicone oil.

Substituted or unsubstituted, straight or branched monovalent hydrocarbon groups that are represented by $R^2$ of general formula (1), that are bonded to silicon atoms, and that have 9 to 60 carbon atoms, may be the same or different. Furthermore, the structure thereof is selected from among straight chain, branched, and partially branched. In the present invention, it is particularly preferable for R2 to be an unsubstituted straight chain monovalent hydrocarbon group. An unsubstituted monovalent hydrocarbon group can be, for example, an alkyl group, aryl group, or aralkyl group having 9 to 60 carbon atoms, preferably 9 to 30 carbon atoms, and more preferably 10 to 25 carbon atoms. On the other hand, examples of the substituted monovalent hydrocarbon group include perfluoroalkyl groups, aminoalkyl groups, amide alkyl groups, and ester groups having from 9 to 30 carbon atoms, preferably from 9 to 30 carbons atoms, and more preferably from 10 to 24 carbon atoms. Additionally, the carbon atoms of the monovalent hydrocarbon groups may

be partially substituted with alkoxy groups, and examples of said alkoxy groups include methoxy groups, ethoxy groups, and propoxy groups. This type of monovalent hydrocarbon group is particularly preferably an alkyl group having 9 to 30 carbon atoms, and an example thereof is a group represented by the general formula $-(CH_2)_v-CH_3$ (v is a number in a range of 8 to 29). Particularly, an alkyl group having 10 to 24 carbon atoms is preferable.

The chain organosiloxane group in general formula (2-1) or (2-2) has a straight chain polysiloxane chain structure, unlike a silylalkyl group, which has a siloxane dendron structure. In general formula (2-1) or (2-2), $R^{11}$ are each independently a substituted or unsubstituted monovalent hydrocarbon group having from 1 to 30 carbon atoms, a hydroxyl group, or a hydrogen atom. The substituted or unsubstituted monovalent hydrocarbon group with 1 to 30 carbon atoms is preferably an alkyl group with 1 to 30 carbon atoms, an aryl group with 6 to 30 carbon atoms, an aralkyl group with 6 to 30 carbon atoms, or a cycloalkyl group with 6 to 30 carbon atoms, and is exemplified by a methyl group, ethyl group, propyl group, butyl group, pentyl group, hexyl group, heptyl group, octyl group, decyl group, or other alkyl group; a cyclopentyl group, cyclohexyl group, or other cycloalkyl group; or a phenyl group, tolyl group, or other aryl group. The hydrogen atoms bonded to the carbon atoms of these groups may be substituted at least partially by fluorine or a similar halogen atom, or an organic group containing an epoxy group, acyl group, carboxyl group, amino group, methacryl group, mercapto group, or the like. A methyl group, a phenyl group, or a hydroxyl group is particularly preferable as $R^{11}$. A configuration in which a part of $R^{11}$ is a methyl group and another part of $R^{11}$ is a long chain alkyl group having 8 to 30 carbon atoms is also preferable.

In general formula (2-1) or (2-2), t is a number in a range from 2 to 10; r is a number in a range from 1 to 500; and r preferably is a number in a range from 2 to 500. Such a straight chain organosiloxane group is hydrophobic. From the standpoint of compatibility with various oil agents, r preferably is a number in a range from 1 to 100, and particularly preferably is a number in a range from 2 to 30.

A silylalkyl group having a siloxane dendron structure shown by general formula (3) is a functional group that includes a structure wherein a carbosiloxane unit spreads in a dendrimer shape and that exhibits high water repellence. The silylalkyl group is well-balanced when combined with hydrophilic groups, and when an external use preparation or cosmetic composition that incorporates the glycerin derivative-modified silicone is used, the silylalkyl group inhibits an unpleasant sticky feeling, and provides a refreshingly natural feeling to the touch. Additionally, the silylalkyl group having a siloxane dendron structure is chemically stable, and for this reason, the silylalkyl group is a functional group providing advantageous properties such as usability in combination with a wide range of components.

Examples of the substituted or unsubstituted, straight or branched monovalent hydrocarbon group having 1 to 30 carbon atoms (the $R^3$ moieties in general formula (3)) include methyl groups, ethyl groups, propyl groups, butyl groups, pentyl groups, hexyl groups, heptyl groups, octyl groups, and similar alkyl groups; cyclopentyl groups, cyclohexyl groups, and similar cycloalkyl groups; vinyl groups, allyl groups, butenyl groups, and similar alkenyl groups; phenyl groups, tolyl groups, and similar aryl groups; benzyl groups and similar aralkyl groups; and groups wherein the hydrogen atoms bonded to the carbon atoms of these groups are substituted at least partially by fluorine or a similar halogen atom, or an organic group containing an epoxy group, a glycidyl group, an acyl group, a carboxyl group, an amino group, a methacryl group, a mercapto group, or the like (provided that the total number of carbons is from 1 to 30).

Among the phenyl group or the alkyl group having from 1 to 6 carbons represented by $R^4$ in general formula (3), examples of the alkyl group having from 1 to 6 carbons include methyl, ethyl, n-propyl, i-propyl, n-butyl, i-butyl, s-butyl, pentyl, neopentyl, cyclopentyl, hexyl, and similar straight, branched, or cyclic alkyl groups.

In the general formula (3), in the case of i = k, $R^4$ is preferably a methyl group or a phenyl group. In particular, R4 is preferably a methyl group when i = k.

From an industrial standpoint, the number of generations k is preferably an integer from 1 to 3, and more preferably is 1 or 2. In each of the number of generations, the group represented by $L^1$ is expressed as follows. In the formulae, $R^3$, $R^4$, and Z are the same groups as described above.

When the number of generations is k=1, $L^1$ is represented by the following general formula (3-1).

[Formula 9]

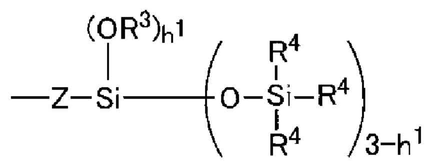

(3-1)

When the number of generations is k=2, $L^1$ is represented by the following general formula (3-2).

[Formula 10]

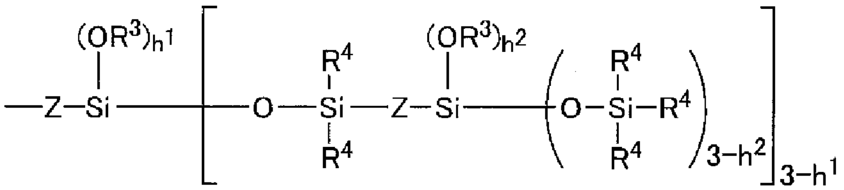

(3-2)

When the number of generations is k=3, $L^1$ is represented by the following general formula (3-3).

[Formula 11]

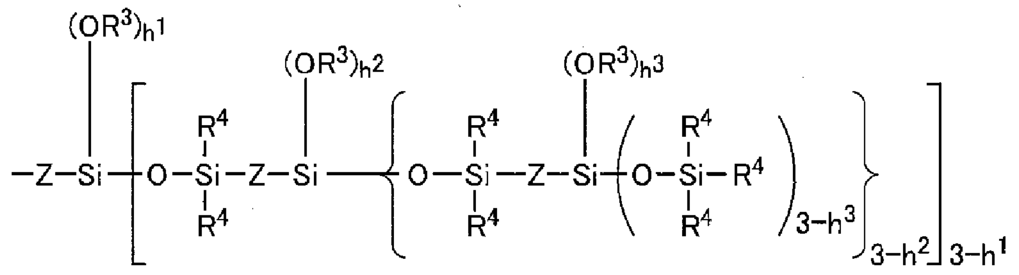

(3-3)

In the structures expressed by the general formulae (3-1) to (3-3) in the case of the number of generations is from 1 to 3, each of $h^1$, $h^2$ and $h^3$ moieties is independently a number in a range from 0 to 3. These $h^i$ moieties are preferably a number in a range from 0 to 1, and $h^i$ is, in particular, preferably 0.

In general formulae (3) and (3-1) to (3-3), Z are each independently a divalent organic group, and specific examples thereof include a divalent organic group formed by addition-reacting a silicon-bonded hydrogen atom and a functional group having an unsaturated hydrocarbon group such as an alkenyl group, an acryloxy group, a methacryloxy group, or the like at the terminal. Depending on the method for introducing the silylalkyl group having a siloxane dendron structure, the functional group can be appropriately selected and is not restricted to the functional groups described above. Preferably, Z are each independently a group selected from divalent organic groups represented by the following general formula.

$$-R^7-$$
$$-R^7-CO-$$
$$-R^7-COO-R^8-$$
$$-CO-R^7-$$
$$-R^7-OCO-R^8-$$
$$-R^7-CONH-R^8-$$
$$-R^7-R^8-$$

[Formula 12]

Of these, Z in $L^1$ is preferably a divalent organic group expressed by general formula $-R^7-$ that is introduced by a reaction between a silicon-bonded hydrogen atom and an alkenyl group. Likewise, Z is preferably a divalent organic group expressed by general formula $-R^7-COO-R^8-$that is introduced by a reaction between a silicon-bonded hydrogen atom and an unsaturated carboxylic ester group.

On the other hand, in the silylalkyl group represented by $L^i$, in which the number of generations k is 2 or more, and $L^i$ is $L^2$ to $L^k$, Z is preferably an alkylene group having from 2 to 10 carbon atoms or a divalent organic group represented by $-R^7-COO-R^8-$ and is particularly preferably a group selected from an ethylene group, a propylene group, a methylethylene group, a hexylene group, and $-CH_2C(CH_3)COO-C_3H_6-$.

In the general formula described above, $R^7$ are each independently a substituted or unsubstituted straight or

branched chain alkylene group or alkenylene group having from 2 to 22 carbons or an arylene group having from 6 to 22 carbons. More specifically, examples of $R^7$ include an ethylene group, a propylene group, a butylene group, a hexylene group, and similar straight alkylene groups; a methylmethylene group, a methylethylene group, a 1-methylpentylene group, a 1,4 -dimethylbutylene group, and similar branched alkylene groups. $R^8$ is preferably a group selected from an ethylene group, a propylene group, a methylethylene group, and a hexylene group.

In the general formula described above, $R^8$ is a group selected from divalent organic groups expressed by the following formula.

[Formula 13]

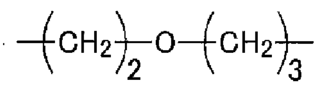
,
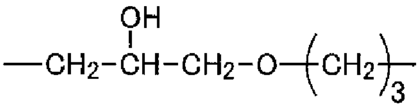

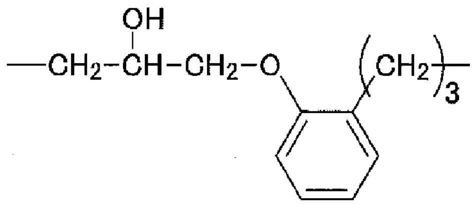
,
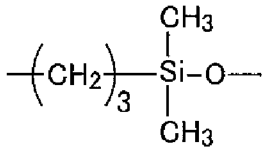

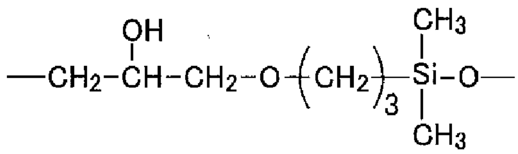
,
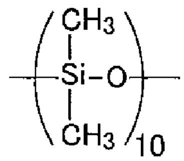

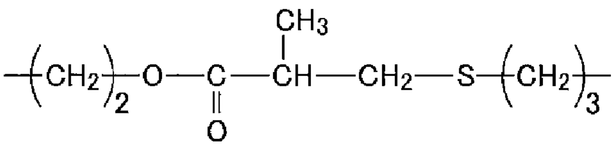

In general formula (1), Q is a glycerin derivative group-containing organic group, and forms the hydrophilic site of the glycerin derivative-modified silicone. The structure of Q is not limited provided that the structure has a glycerin derivative site, but the glycerin derivative residue is preferably bonded to the silicon atom via a divalent organic group.

Here, "glycerin derivative residue" refers to a hydrophilic group having a (poly)glycerin structure, and refers to a hydrophilic group having a monoglycerin, a diglycerin, a triglycerin, a tetraglycerin, and at least a pentaglycerin structure. Additionally, the terminal hydroxyl group may be partially capped with an alkyl group. Furthermore, the (poly)glycerin structure may be straight or branched, and may be a structure that is branched in a dendritic manner as well.

The glycerin derivative group-containing organic group (Q) described above is preferably bonded to a silicon atom via a linking group that is at least divalent and is preferably a glycerin derivative group-containing organic group comprising at least one type of hydrophilic unit selected from hydrophilic units represented by structural formulae (3-3) to (3-6) below. However, the hydrophilic units constituting Q do not consist of only the following structural formula (3-6).

[Formula 14]
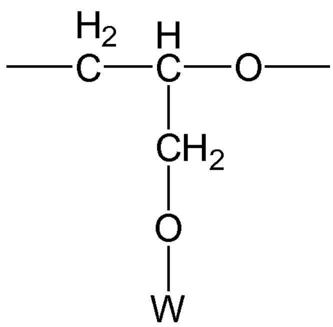
(3-3)

[Formula 15] 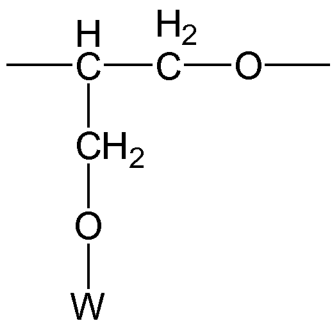 (3-4)

[Formula 16] 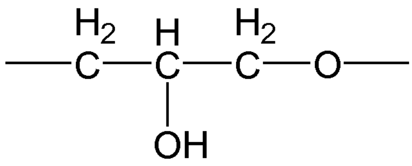 (3-5)

[Formula 17] $-C_rH_{2r}-O-$ (3-6)

In structural formula 3-1, r is a number in a range of 1 to 6.

In formulae (3-3) to (3-5), W is a hydrogen atom or an alkyl group having from 1 to 20 carbons, and preferably is a hydrogen atom. Particularly, when W is a hydrogen atom, oxidation in air does not occur easily, and aldehydes such as formaldehyde and the like, and antigenic compounds such as formate esters and the like, are not easily produced over time while in storage. Therefore, when W is a hydrogen atom, there is a benefit of high environmental compatibility.

The hydrophilic units represented by structural formulae (3-3) to (3-5) are hydrophilic units included in a hydrophilic group derived from a hydrophilic compound selected principally from polyhydric alcohols including glycerin, polyglycerins (also called "polyglycerols"), and polyglycidyl ethers or compounds in which terminal hydroxyl groups thereof are partially capped by hydrocarbon groups. Furthermore, the glycerin derivative group-containing organic group (Q) according to the present invention may be a hydrophilic group optionally comprising a hydrophilic structure (polyether structure) consisting of an oxyalkylene unit represented by the above structural formula (3-6) (for example, an oxyethylene unit or an oxypropylene unit).

In the general formula (1), Q may be, for example, a hydrophilic group that does not have a branched structure such as a monoglycerin-modified group or a diglycerin-modified group, and may also be a hydrophilic group that has a partial branched structure in the functional group such as a polyglycerol group or a polyglycidylether group.

More specifically, Q may be a glycerin derivative group-containing organic group bonded to a silicon atom via a linking group that is at least divalent, comprising at least one linearly bonded hydrophilic unit selected from hydrophilic units represented by the following structural formulae (3-3) to (3-6) (however, the hydrophilic units constituting Q do not consist of only the structural formula (3-6)). Similarly, Q may be a glycerin derivative group-containing organic group that is bonded to a silicon atom via a linking group that is at least divalent, the glycerin derivative group-containing organic group containing at least two hydrophilic units of at least one type selected from hydrophilic units represented by the above structural formulae (3-3) to (3-6) and having a branched unit selected from groups represented by the following structural formulae (3-7) to (3-9).

[Formula 18] 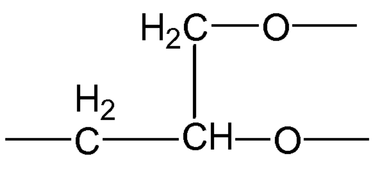 (3-7)

[Formula 19] 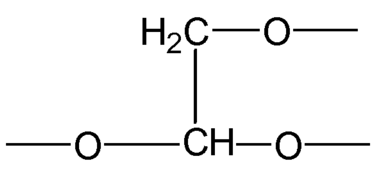 (3-8)

[Formula 20] 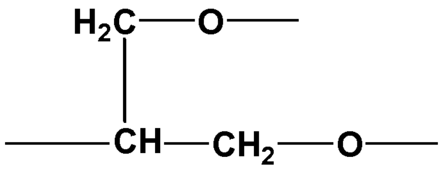 (3-9)

The at least one type of hydrophilic unit selected from the hydrophilic units represented by the general formulae (3-3) to (3-6) are each independently bonded to the two oxygen atoms of the above structural formulae (3-7) to (3-9). The hydrophilic unit may further be bonded to a branch unit selected from groups represented by structural formulae (3-7) to (3-9). Moreover, the hydrophilic unit may be formed so as to have a dendroid-shape polyether structure, a polyglycerol structure, or a polyglycidyl ether structure obtained by branching into multiple generations. For example, the structure of a hydrophilic group Q which has one branch unit represented by structural formula (3-7) and two branch units represented by structural formula

(3-9) and which is branched in a dendritic manner is shown below, but it goes without saying that dendroid-shape polyglycerol structures are not limited to this example.

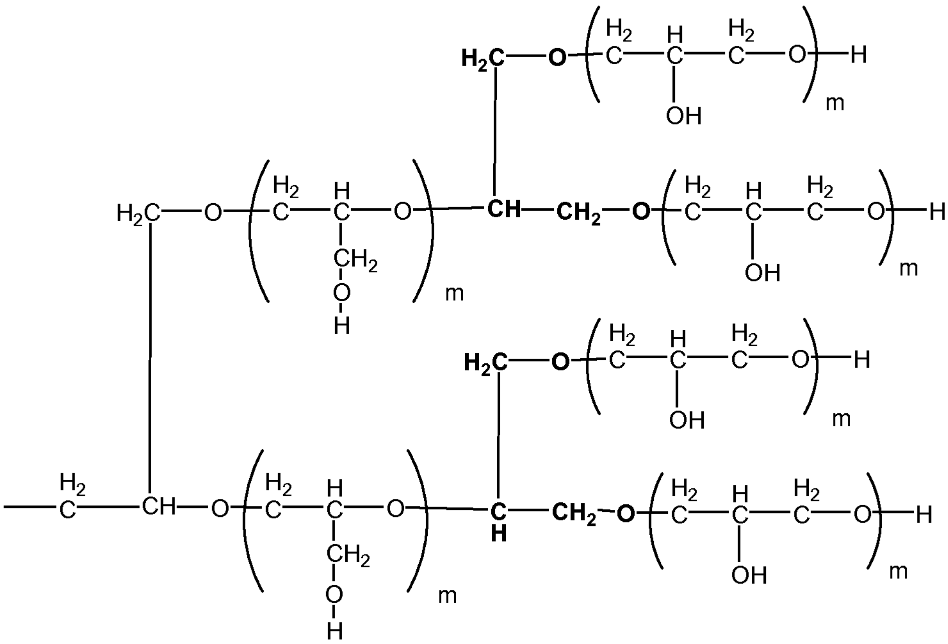

[Formula 21]

(In the formula, m is a number in a range from 0 to 50, provided that not all of the m moieties are 0).

The linking group that is at least divalent is a bonding site with respect to the silicon atom included in the hydrophilic group Q, and a structure thereof is not particularly limited. Examples thereof include, ethylene groups, propylene groups, butylene groups, hexylene groups, and similar alkylene groups; ethylene phenylene groups, propylene phenylene groups, and similar alkylene phenylene groups; ethylene benzylene groups and similar alkylene aralkylene groups; ethyleneoxy phenylene groups, propyleneoxy phenylene groups, and similar alkyleneoxy phenylene groups; methyleneoxy benzylene groups, ethyleneoxy benzylene groups, propyleneoxy benzylene groups, and similar alkyleneoxy benzylene groups; and, furthermore, groups described below. Note that there are preferably from 0 to 3 and more preferably 0 or 1 ether bonds in the linking group that is at least divalent.

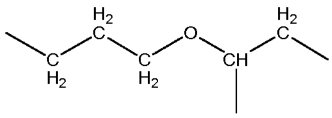

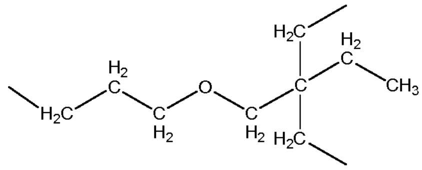

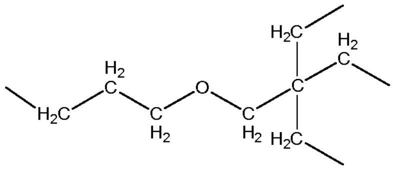

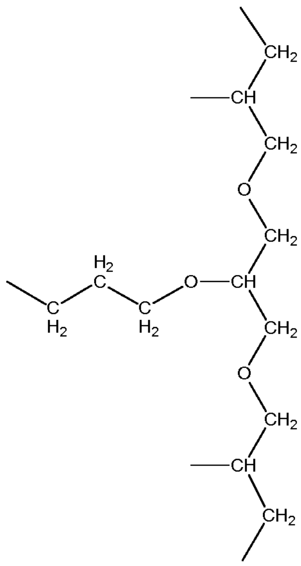

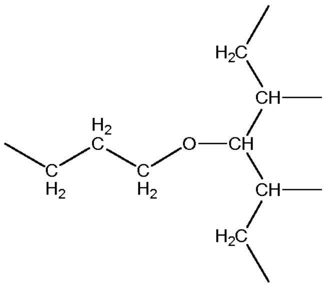

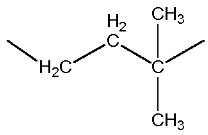

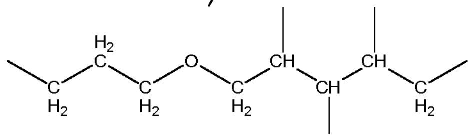

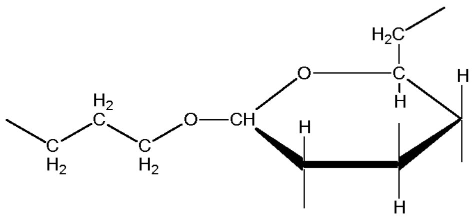

[Formula 22]

More preferably, Q is a hydrophilic group represented by structural formulae (4-1) to (4-4) below, and these are generally hydrophilic groups derived from polyglycerin-based compounds.

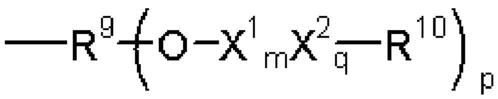

(4-1)

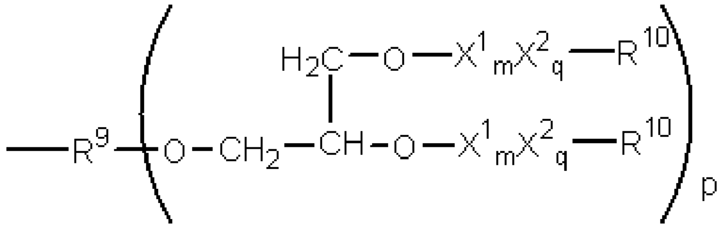

(4-2)

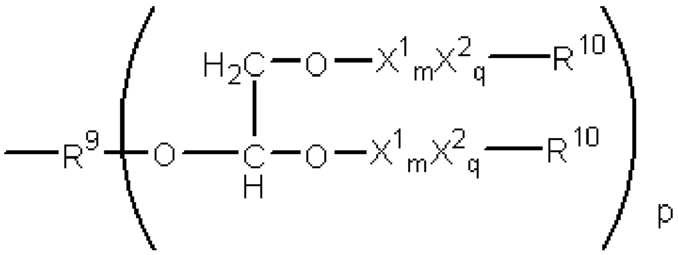

(4-3)

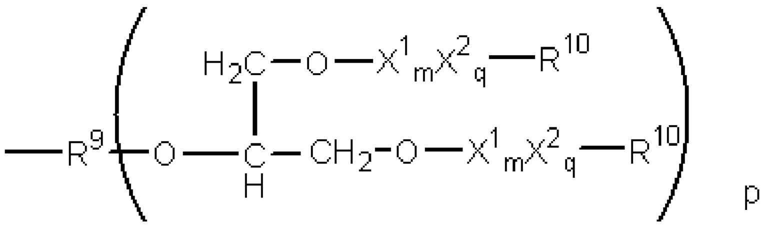

(4-4)

[Formula 23]

In formulae (4-1) to (4-4), $R^9$ is an organic group having (p+1) valence, and p is a number that is greater than or equal to 1 and less than or equal to 3. As the $R^9$, the same groups as the linking group that is at least divalent may be mentioned.

It is more preferable that p is equal to 1 and that $R^9$ is a group selected from divalent organic groups expressed by the following general formulae.

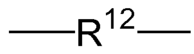

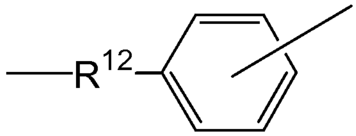

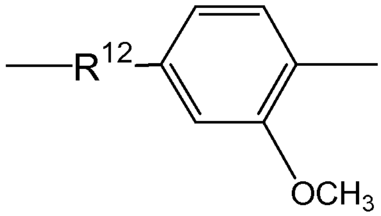

[Formula 24] 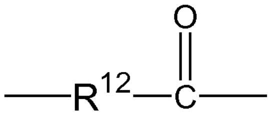

In the formulae, $R^{12}$ may have a substituent, and are each independently a straight or branched chain alkylene group or alkenylene group having from 2 to 22 carbon atoms, or an arylene group having from 6 to 22 carbon atoms.

$X^1$ are each independently at least one hydrophilic unit selected from the hydrophilic units expressed by general formulae (3-3-1) to (3-5-1) below, and m is a number in a range of 1 to 5, and is more preferably a number in a range of 1 to 4.

[Formula 25] 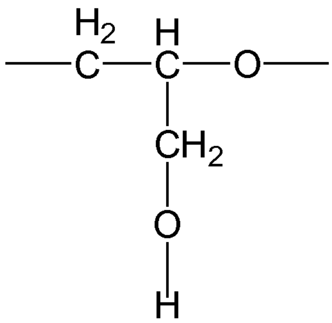 (3-3-1)

[Formula 26] 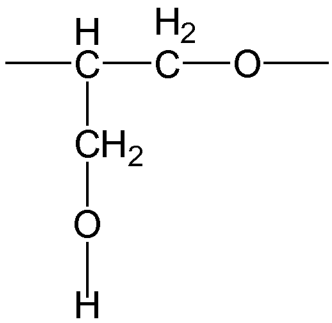 (3-4-1)

[Formula 27] 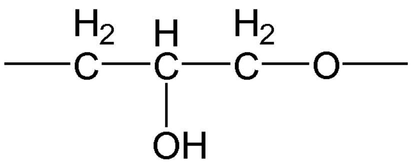 (3-5-1)

$X^2$ is an optional (poly)oxyethylene unit that Q may contain, and q is a number in a range of from 0 to 100. Here, q is preferably a number in a range of from 0 to 50 and is preferably from 0 to 30. Furthermore, $X^2$ may contain a poly(oxypropylene unit) and/or a (poly)oxybutylene unit together with the (poly)oxyethylene unit. In this case, $X^2$ may be contained in Q as a (poly)oxyalkylene unit represented by the formula:

$$-(C_2H_4O)_{t1}(C_3H_6O)_{t2}(C_4H_8O)_{t3}-$$

(in the formula, t1, t2, and t3 are numbers satisfying $0 \le t1 \le 100$, $0 \le t2 \le 100$, and $0 \le t3 \le 50$, preferably numbers satisfying $0 \le t1 \le 50$, $0 \le t2 \le 50$, and $0 \le t3 \le 30$, and more preferably numbers satisfying $0 \le t1 \le 30$, $0 \le t2 \le 30$, and $0 \le t3 \le 10$).

Here, the manner in which $X^1$ and $X^2$ are bonded can be block or random. That is, the hydrophilic group Q may be a hydrophilic group in which hydrophilic segments, which are obtained by bonding hydrophilic units expressed by general formulae (3-3-1) to (3-5-1) above in a block manner, are bonded to hydrophilic segments comprising polyoxyalkylene units, and may be a hydrophilic group in which these constituent units are bonded in a random manner. An example thereof is a bonding pattern such as $-(X^2)_{m1}-X^1-(X^2)_{m2}-X^1-$.

$R^{10}$ is a hydrogen atom or a group selected from the group consisting of glycidyl groups, acyl groups, and alkyl groups having from 1 to 20 carbon atoms.

From the perspectives of gel formability and the thickening effect with respect to the oil agent component of the glycerin derivative-modified silicone of the present invention and the perspective of the surface activity performance such as the emulsion and dispersion stability, a preferable hydrophilic group Q is a hydrophilic group derived from (poly)glycerin represented by the following structural formula (4-1-1).

[Formula 28] $-R^{9'}-O-X^1{}_m-R^{10}$ (4-1-1)

In the formula, $R^{9'}$ is a divalent organic group, and can be a group synonymous with those mentioned above. $X^1$ and $R^{10}$ are synonymous with the groups described above, and m is a number in a range of 1 to 5.

In the glycerin derivative-modified silicone of the present invention, from the perspectives of thickening effect and gel formability with respect to the oil agent component, use as a surfactant (emulsifier), a moisturizer, or various treatment agents (powder dispersing agent or surface treatment agent), and particularly use as a powder treatment agent and a cosmetic composition starting material, the hydrophilic group Q is a hydrophilic group derived from a (poly)glycerin system compound and is most preferably a hydrophilic group derived from (poly)glycerin. Specifically, the hydrophilic group Q is a (poly)glycerin monoallyl ether or a (poly)glyceryl eugenol, which are examples of hydrophilic groups derived from (poly)glycerin compounds having a monoglycerin, diglycerin, triglycerin, or tetraglycerin structure.

A particularly suitable hydrophilic group Q is a diglycerin derivative group-containing organic group in which the number of repetitions m of glycerin units in the structural formula (4-1-1) is a number in a range of from 1.5 to 2.4 on average. At this time, $R^{9'}$ is a divalent organic group, and can be a group synonymous with those mentioned above. $X^1$ and $R^{10}$ are also synonymous with the groups described above.

It is most preferable for the diglycerin derivative group-containing organic group to be a diglycerin derivative group-containing organic group represented by following general formula (5-1):

[Formula 29] 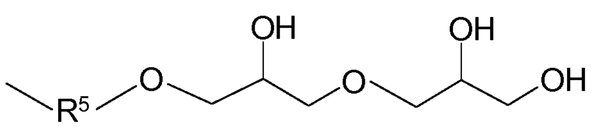 (5-1)

(In the formula, $R^5$ is a divalent organic group that does not contain an oxyalkylene structure wherein an average value of the number of repetitions of the oxyalkylene unit is two or more) or the following general formula (5-2):

[Formula 30] 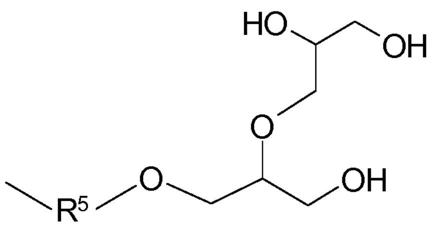 (5-2)

(wherein $R^5$ is synonymous with that described above).

The bond position of the glycerin derivative group-containing organic group (-Q) can be either the terminal or side chain of polysiloxane, which is the main chain; and the structure may have two or more glycerin derivative group-containing organic groups per molecule of glycerin derivative-modified silicone. Furthermore, the two or more glycerin derivative group-containing organic groups can be the same or different glycerin derivative group-containing organic groups. These two or more glycerin derivative group-containing organic groups can be structured such that bonding occurs only in a side chain of polysiloxane, which is the main chain, only at a terminal, or in a side chain and at a terminal.

The glycerin derivative-modified silicone having a glycerin derivative group-containing organic group (-Q) represented by general formula (1) is preferably a liquid at least at 100°C. In addition, the polysiloxane main chain may be a straight chain, a branched chain, or reticulated (including slightly crosslinked and elastomeric). With the manufacturing method of the present invention, it is possible to easily improve the opaque appearance of a composition and stabilize the composition as a translucent or transparent uniform liquid, not only in the case of a low-viscosity glycerin derivative-modified silicone, but also in the case of a glycerin derivative-modified silicone which has high viscosity and is in a solid form at room temperature (including gummy compositions having plasticity and poor fluidity).

The particularly preferable glycerin derivative-modified silicone of the present invention is a glycerin derivative-modified silicone having a straight chain polysiloxane structure represented by structural formula (1-1) below:

[Formula 31]

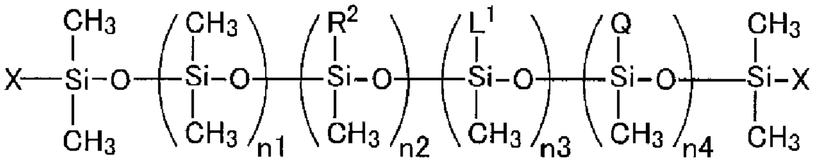

(1-1)

(In the formula,

$R^2$, $L^1$, and Q are each independently synonymous with those described above;
X is a group selected from the group consisting of a methyl group, $R^2$, $L^1$, and Q;
n1, n2, n3, and n4 are each independently a number in a range from 0 to 2,000, and
n1+n2+n3+n4 is a number in a range from 0 to 2,000;however, when n4=0, at least one X is Q.)

In formula (1-1), (n1+n2+n3+n4) preferably is a number in a range from 10 to 2,000, more preferably is in a range from 25 to 1,500, and particularly preferably is a number in a range from 50 to 1,000. n1 preferably is a number in a range from 10 to 2,000, more preferably is in a range from 25 to 1,500, and particularly preferably is in a range from 50 to 1,000. n2 preferably is a number in a range from 0 to 250, more preferably in a range from 0 to 150.

When $R^2$ is the long chain alkyl group, n2>1 is particularly preferable from the standpoint of compatibility with oil agents other than silicone and surface activity. n3 preferably is a number in a range from 0 to 250, and it is particularly preferable that 3>1, and that it has least one silylalkyl group (-$L^1$) having a siloxane dendron structure in a side chain portion. [1])n4 is a number in a range from 0 to 100, and preferably is in a range from 0 to 50. However, when n4 = 0, at least one X needs to be Q.

In the structural formula (1-1), it is preferable that Q are each independently a glycerin derivative group-containing organic group expressed by any of general formulae (4-1) through (4-4). In the glycerin derivative-modified silicone, all Qs can be one type of glycerin derivative group-containing organic group, that is expressed by any of general formulae (4-1) through (4-4). A part of the Qs in a molecule can be a glycerin derivative group-containing organic group expressed by any of general formulae (4-1) through (4-4) above. The remaining Qs may be another glycerin derivative group-containing organic group.

Furthermore, the glycerin derivative-modified silicone can be a mixture of one or two or more types of a glycerin derivative-modified silicone expressed by general formula (1). More specifically, the glycerin derivative-modified silicone can be a mixture of at least two types of glycerin derivative-modified silicone, with different types of modified groups, modification rate, and degree of polymerization of the siloxane main chain.

As the glycerin derivative-modified silicone, the glycerin derivative-modified silicone represented by the following structural formula (1-1-1) is preferable:

[Formula 32]

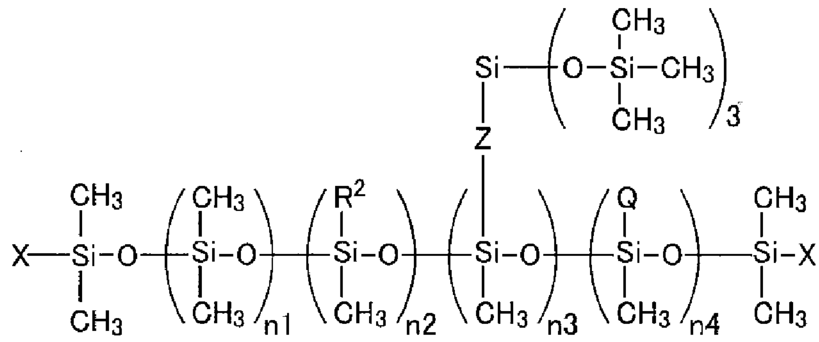

(1-1-1)

(wherein
(In the formula, $R^2$, Q, X, Z, n1, n2, n3, and n4 are synonymous with those described above), or the following structural formula (1-1-2):

[Formula 33]

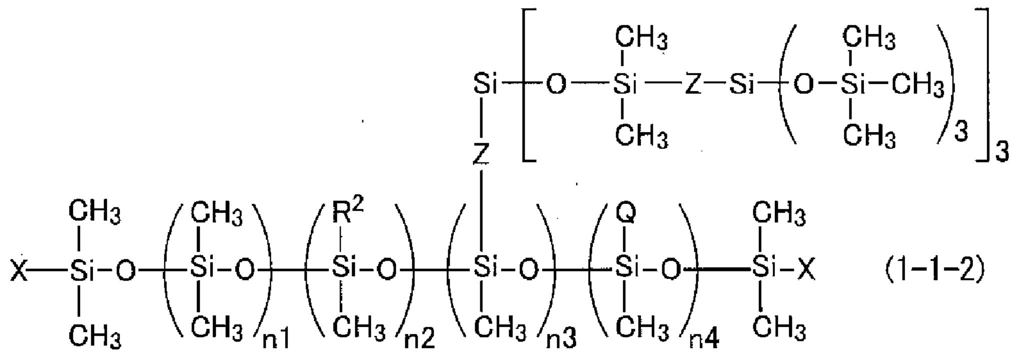

(1-1-2)

(wherein
(In the formula, $R^2$, Q, X, Z, n1, n2, n3, and n4 are synonymous with those described above).

The modification rate of organopolysiloxane due to the glycerin derivative group-containing organic group is preferably in a range from 0.001 to 50 mol%, more preferably within the range from 0.01 to 30 mol%, and yet more preferably within the range from 0.1 to 10 mol% of all functional groups bonded to polysiloxane, which is the main chain. Furthermore, in the glycerin derivative-modified silicone represented by structural formula (1-1), the modification rate (mol%) resulting from the glycerin derivative group-containing organic group is expressed by the following formula:

Modification rate (mol%) = (number of glycerin derivative group-containing organic groups bonded to silicon atoms per molecule) / (6 + 2 × (n1 + n2 + n3 + n4)) × 100

For example, in the case of a glycerin derivative-modified silicone consisting of dodecylsiloxane having ten glycerin derivative group-containing organic groups (GLY groups) (represented by the structural formula $MD^{GLY}{}_{10}M$), 10 of the 26 silicon-bonded functional groups are modified by the glycerin derivative group-containing organic groups, so the modification rate by the glycerin derivative group-containing organic groups is 38.5 mol%.

(Production of glycerin derivative-modified silicone and mixture containing the same as a main component)

The glycerin derivative-modified silicone can be obtained by, for example, reacting (a1) a glycerin derivative having one reactive unsaturated group per molecule, (b1) organopolysiloxane having silicon atom bonded hydrogen

atoms, and (c1) an organic compound having one reactive unsaturated group per molecule, and if necessary, (d1) a siloxane dendron compound having one reactive unsaturated group per molecule, and/or (e1) a long chain hydrocarbon compound or a chain organopolysiloxane compound having one reactive unsaturated group per molecule in the presence of a hydrosilylation reaction catalyst. The reactive unsaturated group preferably is an unsaturated functional group having a carbon-carbon double bond, and is exemplified by an alkenyl group or unsaturated fatty acid ester group. The $-R^1$ is introduced by component (c1), the $-L^1$ is introduced by component (d1), and the id $-R^2$ is introduced by component (e1).

More specifically, a glycerin derivative-modified silicone can be obtained as below, for example.

The glycerin derivative-modified silicone can be obtained by addition reacting with organopolysiloxane having a silicon-hydrogen bond, an unsaturated organic compound having a carbon-carbon double bond at one terminal of the molecular chain, and an unsaturated ether compound of a glycerin derivative having a carbon-carbon double bond in the molecule. Furthermore, a siloxane dendron compound having a carbon-carbon double bond at one terminal of the molecular chain, and/or an unsaturated long chain hydrocarbon compound having a carbon-carbon double bond at one terminal of the molecular chain, or a chain organopolysiloxane having a carbon-carbon double bond at one terminal of the molecular chain can be further addition reacted.

In the above case, the glycerin derivative-modified silicone can be obtained as the product of a hydrosilylation reaction between the unsaturated organic compound and the glycerin derivative unsaturated ether compound, and arbitrarily the siloxane dendron compound and/or an unsaturated long chain hydrocarbon compound, or a chain organopolysiloxane having a carbon-carbon double bond at one terminal of the molecular chain and a SiH group-containing siloxane. This enables the introduction of an organic group and a glycerin derivative group-containing organic group, and optionally a silylalkyl group having a siloxane dendron structure and/or a long chain hydrocarbon group or a chain organopolysiloxane group into the polysiloxane chain of the glycerin derivative-modified silicone. This reaction can be performed as a batch or can take the form of successive reactions. However, successive reactions are preferable from the perspectives of safety and quality control.

For example, the glycerin derivative-modified silicone can be obtained by reacting at least the (b2) organohydrogensiloxane expressed by the following formula (1') and (a2) a glycerin derivative having one reactive unsaturated group per molecule, in the presence of a hydrosilylation reaction catalyst.

[Formula 34] $R^1_aH_{b+c+d}SiO_{(4-a-b-c-d)/2}$ (1')

(wherein
$R^1$, a, b, c, and d are synonymous with those described above)It is preferable to further react (d) a siloxane dendron compound having one reactive unsaturated group per molecule, and/or (e) a hydrocarbon compound having one reactive unsaturated group per molecule, or chain organopolysiloxane having one reactive unsaturated group per molecule.

The glycerin derivative-modified silicone can be preferably produced by reacting together component (a2), component (d) and/or component (e), as well as (b2) the organohydrogensiloxane expressed by general formula (1'), or by successively addition reacting the (b2) organohydrogensiloxane and optionally the component (d), and/or the component (e), and further addition reacting the component (a2), in the state where (a2) a glycerin derivative having one reactive unsaturated group per molecule, and arbitrarily (d) a siloxane dendron compound having one reactive unsaturated group per molecule, and/or (e) a hydrocarbon compound having one reactive unsaturated group per molecule or a chain organopolysiloxane having one reactive unsaturated group per molecule coexist.

As (b2) an organohydrogensiloxane used in the synthesis of the glycerin derivative-modified silicone, the organohydrogensiloxane is preferably represented by, for example, the following structural formula (1-1)':

[Formula 35]

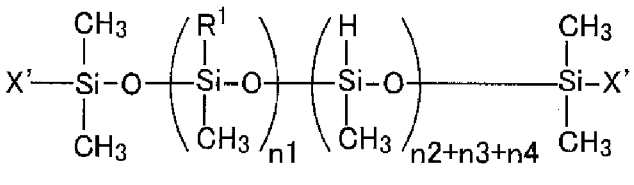

(1-1)'

(wherein

(In the formula, $R^1$ are each independently synonymous with that described above;
X' is a group selected from $R^1$ or hydrogen atom; and
n1, n2, n3, and n4 are synonymous with those described above;however, when n2 + n3 + n4 = 0, at least one X' is

a hydrogen atom)

The glycerin derivative-modified silicone is preferably synthesized by subjecting to a hydrosilylation reaction (a) a glycerin derivative having a carbon-carbon double bond at a terminal of the molecular chain, and (b) an organohydrogenpolysiloxane; and the organohydrogensiloxane (component (b)) is preferably the organohydrogensiloxane obtained by successively addition reacting the component (d1) and/or the component (e1). In this case, the organohydrogensiloxane immediately prior to reaction with component (a) (after successive reactions with other components) is preferably represented by the following structural formula (1-1A).

[Formula 36]

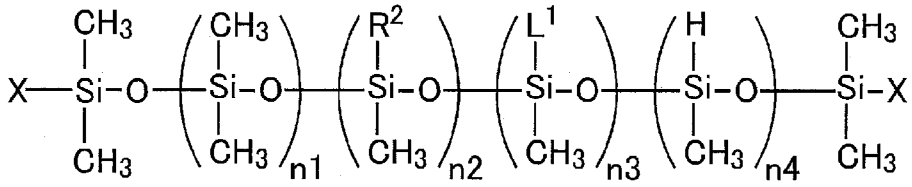

(1-1A)

(wherein

$R^2$ and $L^1$ are each independently synonymous with those described above;
X is selected from the groups consisting of a methyl group, $R^2$, $L^1$, and a hydrogen atom (H);
n1, n2, n3, and n4 are each independently a number in a range from 0 to 2,000, and
n1+n2+n3+n4 is a number in a range from 0 to 2,000;however, when n4=0, at least one X is a hydrogen atom.)

A glycerin derivative having one reactive unsaturated group per molecule, which is used in the synthesis of the glycerin derivative-modified silicone, is preferably (a) a glycerin derivative having a carbon-carbon double bond at the terminal of molecular chain. This is a (poly)glycerin derivative having an allyl(poly)glycerin, allyl polyglycidyl ether, (poly)glycerin monoallyl ether, or similar reactive functional group having an alkenyl group or the like at the molecular terminal, and can be synthesized according to a publicly known method.

In the glycerin derivative-modified silicone of the present invention, from the perspectives of thickening effect and gel formability with respect to an oil agent, use as a surfactant (emulsifier), and various treatment agents (powder dispersing agents or surface treatment agents), component (a) is specifically a (poly)glycerin monoallyl ether or a (poly)glyceryl eugenol, of which examples are (poly)glycerin compounds having a monoglycerin, a diglycerin, a triglycerin, or a tetraglycerin structure.

Such a component (a) can be exemplified by a glycerin derivative having a carbon-carbon double bond at the terminals of the molecular chain shown by the following structural formulae (4-1') through (4-4'). In the formulae, $X^1$, $X^2$, and $R^{10}$ are groups synonymous with the groups described above, and m and q are numbers synonymous with the numbers described above. R' is an unsaturated organic group having a carbon-carbon double bond at the terminal, and is preferably a substituted or unsubstituted, straight or branched unsaturated hydrocarbon group having 3 to 5 carbon atoms. Examples of the unsaturated hydrocarbon group having from 3 to 5 carbon atoms include allyl groups, butenyl groups, methallyl groups, and similar alkenyl groups; andthe unsaturated hydrocarbon group is preferably an allyl group.

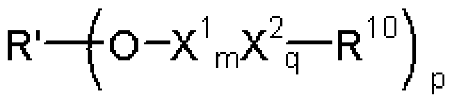

(4-1')

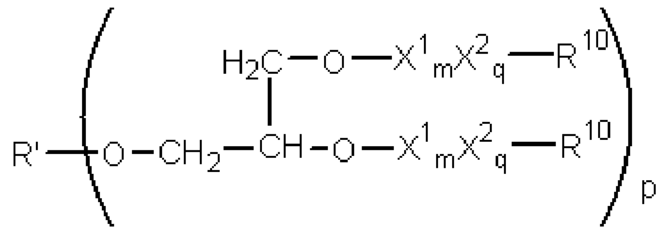

(4-2')

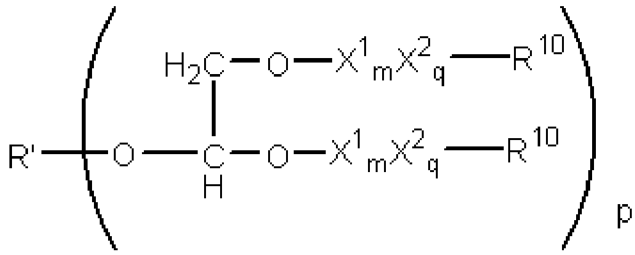

(4-3')

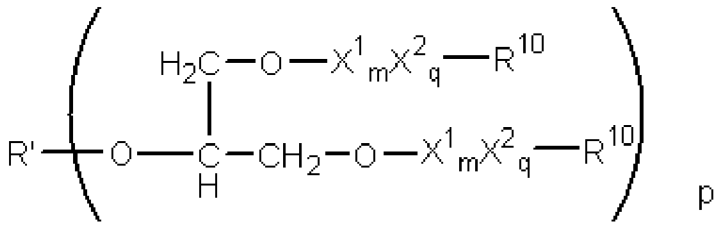

(4-4')

[Formula 37]

(d) The siloxane dendron compound that has one reactive unsaturated group per molecule used in the synthesis of a glycerin derivative-modified silicone of the present invention, is preferably a compound having a siloxane dendron structure with one carbon-carbon double bond at a molecular terminal, and is expressed by the following general formula (3'):

[Formula 38]

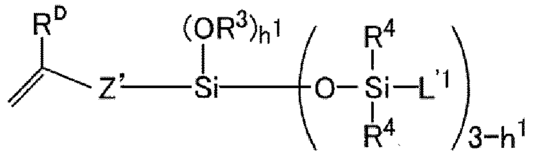

(3')

In this formula:

$R^3$ and $R^4$ are synonymous with those described above, $R^D$ is a hydrogen atom or a methyl group;
Z' is a divalent organic group;
$h^1$ is a number in a range from 0 to 3;
$L'^1$ is the $R^4$ moiety or, when j=1, a silylalkyl group expressed by general formula (3") below:

[Formula 39]

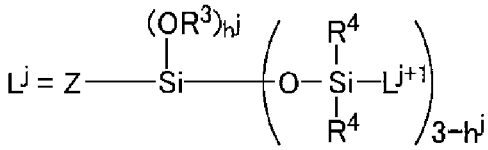

(3")

(wherein $R^3$ and $R^4$ are synonymous with those described above;
Z is a divalent organic group;
j indicates the generations of the silylalkyl group that is represented by $L^j$, when the number of generations (the number of repetitions) of the silylalkyl group is k', j is an integer of 1 to k', and the number of generations k' is an integer from 1 to 9; $L^{j+1}$ is the silylalkyl group when j is less than k' and is the $R^4$ moiety when j = k'; and $h^j$ is a number in a range from 0 to 3).

(e) The hydrocarbon compound having one reactive unsaturated group per molecule or chain organopolysiloxane having one reactive unsaturated group per molecule used in the synthesis of a glycerin derivative-modified silicone of the present invention, is preferably a mono unsaturated organic compound expressed by the following general formula (2'):

[Formula 40] $R'-R^{2'}$ (2')

(wherein R' is synonymous with that described above; and
$R^2$ represents a substituted or unsubstituted, straight or branched monovalent hydrocarbon group having 7 to 58 carbon atoms) or the following general formula (2-1):

[Formula 41]

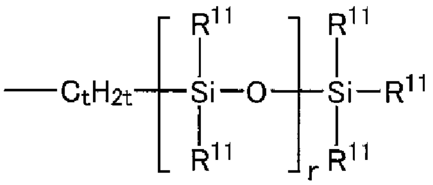

(2-1)

(wherein $R^{11}$, t, and r are synonymous with those described above); or the following general formula (2-2):

[Formula 42]

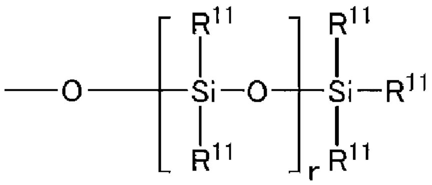

(2-2)

(wherein $R^{11}$ and r are synonymous with those described above).

The hydrocarbon compound having one reactive unsaturated group in the molecule (e) is preferably a monounsaturated hydrocarbons having from 9 to 30 carbon atoms and is more preferably a 1-alkene. Examples of the 1-alkene include 1-nonene, 1-decene, 1-undecene, 1-dodecene, 1-tridecene, 1-tetradecene, 1-hexadecene, 1-octadecene and the like. Examples of the chain organopolysiloxane having one reactive unsaturated group in the molecule include a dimethylpolysiloxane capped at one molecular terminal with a vinyl group, a methylphenylpolysiloxane capped at one molecular terminal with a vinyl group, and the like.

The hydrosilylation reaction used to synthesize the glycerin derivative-modified silicone or the composition thereof can be carried out using a publicly known method in the presence or absence of a solvent. Here, examples of the reaction solvent include alcohol-based solvents such as ethanol and isopropyl alcohol; aromatic hydrocarbon-based

solvents such as toluene and xylene; ether-based solvents such as dioxane and THF; aliphatic hydrocarbon-based solvents such as n-hexane, cyclohexane, n-heptane, cycloheptane, and methylcyclohexane; and chlorinated hydrocarbon-based organic solvents such as carbon tetrachloride.

The hydrosilylation reaction may be performed in the absence of a catalyst, but preferably is performed in the presence of a catalyst because the reaction can be carried out at a low temperature and in a shorter period of time. Examples of the catalyst include platinum, ruthenium, rhodium, palladium, osmium, iridium, and similar compounds, and platinum compounds are particularly effective due to their high catalytic activity. Examples of the platinum compound include chloroplatinic acid; platinum metal; platinum metal supported on a carrier such as platinum supported on alumina, platinum supported on silica, platinum supported on carbon black, or the like; and a platinum complex such as platinum-vinylsiloxane complex, platinum-phosphine complex, platinum-phosphite complex, platinum alcoholate catalyst, or the like. If a platinum catalyst is used, the usage quantity of the solvent is approximately 0.0001 to 0.1 wt.%, and preferably 0.0005 to 0.05 wt.%, relative to the weight of the metal catalyst, but is not particularly limited.

A reaction temperature of the hydrosilylation reaction is typically from 30 to 120°C, and a reaction time is typically from 10 minutes to 24 hours and preferably from 1 to 10 hours.

When the hydrosilylation reaction is performed, the ratio [amount of substance of carbon-carbon double bonds in glycerin derivative group-containing compound / amount of substance of silicon-bonded hydrogen atoms to be added to the carbon-carbon double bonds of the glycerin derivative group-containing compound in the organohydrogenpolysiloxane] is preferably in a range from 0.8 to 1.5, and more preferably in a range from 1.0 to 1.3. That is, when synthesizing a glycerin derivative-modified silicone or a glycerin derivative-modified silicone-containing composition of the present invention, it is more preferable to use a slight excess of glycerin derivative group-containing compound. Although processing with the ratio above 1.5 is also possible, the proportion of residual starting material increases, so it is not economical. In addition, during the hydrosilylation reaction, the terminal carbon-carbon double bonds in the glycerin derivative group-containing compound transition internally so that a deactivating side-reaction occurs simultaneously. Therefore, when the ratio described above is from 0.8 to 1.0, the silicon-bonded hydrogen atoms consumed by the hydrosilylation reaction settle to within a slightly lower range than the range of theoretical values from 0.8 to 1.0, so silicon-bonded hydrogen atoms remain at a slightly greater ratio than 0 to 0.2. However, it is also possible to cause dehydrogenation reactions with hydroxyl groups contained in the glycerin derivative group and alcoholic hydroxyl groups of the reaction solvent, which can consume the remaining silicon-bonded hydrogen atoms, depending on the reaction conditions.

On the other hand, if the ratio is less than 0.8, there is a risk that unreacted organohydrogenpolysiloxane will remain. When such a glycerin derivative-modified silicone or a glycerin derivative-modified silicone-containing composition is used as the starting material for an external use preparation or a cosmetic composition, residual organohydrogenpolysiloxane might react with the other starting materials, and generate hydrogen gas. This might cause such undesirable effects as alteration of the external use preparation or the cosmetic composition at the incorporation destination, fire, container expansion, and the like. In addition, when an attempt is made to consume the remaining silicon-bonded hydrogen atoms by using a dehydrogenation reaction when the ratio is less than 0.8, the proportion of Si-O-C crosslinked bonds increases, which increases the tendency to cause gelation during production. Therefore, to enable the complete and safe consumption of organohydrogenpolysiloxane, it is preferable that the ratio exceeds 0.8, i.e., that 0.8 equivalent or more of the glycerin derivative group-containing compound is reacted.

(Glycerin derivative-modified silicone having Si-C bond in crosslinking portion)

The glycerin derivative-modified silicone described above may be a liquid organo-modified silicone having a silicon-bonded glycerin derivative group-containing organic group and having a crosslinked structure containing a carbon-silicon bond in a crosslinking portion.

The organo-modified silicone can be obtained by reacting:

(A) an organohydrogenpolysiloxane;

(B) a glycerin derivative group-containing organic compound having one or more reactive unsaturated groups in each molecule; and

(C) one or more types of organic compounds selected from the group consisting of (C1) an organic compound having a number of reactive unsaturated groups greater than 1 on average in each molecule and (C2) an organic compound having one or more reactive unsaturated groups and one or more epoxy groups in each molecule (however, the use of the component (B) is optional when the component (C) contains a glycerin derivative group-containing organic group).

The (A) organohydrogenpolysiloxane is not particularly limited as long as it has silicon-bonded hydrogen atoms, but an organohydrogenpolysiloxane having more than one - preferably from 1.01 to 100, more preferably from 1.1 to 50, even more preferably from 1.2 to 25, and particularly preferably from 1.3 to 10 - silicon-bonded hydrogen atoms in

the molecule on average is preferable, and a straight-chain, branched, or reticulated organopolysiloxane may be used. The positions of the silicon-bonded hydrogen atoms in the organohydrogenpolysiloxane is not limited, and can be on the main chain or at the terminals. One type or two or more types of organohydrogenpolysiloxanes may be used as the component (A).

Examples of the component (A) include 1,1,3,3-tetramethyldisiloxane, 1,3,5,7-tetramethylcyclotetrasiloxane, methylhydrogenpolysiloxane capped at both molecular terminals with trimethylsiloxy groups, dimethylsiloxane-methylhydrogensiloxane copolymers capped at both molecular terminals with trimethylsiloxy groups, dimethylsiloxane capped at both molecular terminals with dimethylhydrogensiloxy groups, dimethylpolysiloxane capped at both molecular terminals with dimethylhydrogensiloxy groups, dimethylsiloxane-methylhydrogensiloxane copolymers capped at both molecular terminals with dimethylhydrogensiloxy groups, methylhydrogensiloxane-diphenylsiloxane copolymers capped at both molecular terminals with trimethylsiloxy groups, methylhydrogensiloxane-diphenylsiloxane-dimethylsiloxane copolymers capped at both molecular terminals with trimethylsiloxy groups, copolymers comprising $(CH_3)_2HSiO_{1/2}$ units and $SiO_{4/2}$ units, and copolymers comprising $(CH_3)_2HSiO_{1/2}$ units, $SiO_{4/2}$ units, and $(C_6H_5)SiO_{3/2}$ units.

The component (A) is preferably expressed by the average composition formula (1):

$$R^1{}_eH_fSiO_{(4-e-f)/2} \qquad (1)$$

(wherein the $R^1$ moieties are each independently monovalent organic groups, 1.0≤3≤3.0, and 0.001≤f≤1.5).

Although the molecular structure of the (A) organohydrogenpolysiloxane is not limited, examples include straight-chain, partially branching straight-chain, branched-chain, cyclic, and dendric structures, and straight-chain is preferable. The molecular weight is not particularly limited, and products having a low molecular weight to products having a high molecular weight can be used. Specifically, the number-average molecular weight is preferably in a range from 100 to 1,000,000 and more preferably in a range from 300 to 500,000.

Examples of such organohydrogenpolysiloxanes includes those expressed by the following structural formulas:

(i) $R^1{}_3SiO(R^1{}_2SiO)_v(R^1SiHO)_wSiR^1{}_3$
(ii) $HR^1{}_2SiO(R^1{}_2SiO)_v(R^1SiHO)_zSiR^1{}_3$
(iii) $HR^1{}_2SiO(R^1{}_2SiO)_v(R^1SiHO)_zSiR^1{}_2H$

(wherein $R^1$ is as described above, v is 0 or a positive integer, w is a positive integer, and z is 0 or a positive integer). These organohydrogenpolysiloxanes are straight-chain organohydrogenpolysiloxanes having a silicon-bonded hydrogen atom on (i) only the side chain, (ii) the side chain or one molecular terminal, or (iii) the side chain or both molecular terminals.

The monovalent organic group is not particularly limited but is preferably selected from the following (D1) to (D10):

(D1) a substituted or unsubstituted, straight-chain or branched monovalent hydrocarbon group having from 1 to 60 carbon atoms;
(D2) a polyoxyalkylene group expressed by $-R^8O(AO)_zR^9$ (wherein AO is an oxyalkylene group having from 2 to 4 carbon atoms; $R^8$ is a substituted or unsubstituted, straight-chain or branched divalent hydrocarbon group having from 3 to 5 carbon atoms; $R^9$ is a hydrogen atom, a substituted or unsubstituted, straight-chain or branched monovalent hydrocarbon group having from 1 to 24 carbon atoms, or a substituted or unsubstituted, straight-chain or branched acyl group having from 2 to 24 carbon atoms; and z=1 to 100);
(D3) a substituted or unsubstituted, straight-chain or branched alkoxy group having from 1 to 30 carbon atoms;
(D4) a hydroxyl group;
(D5) an ester group expressed by $-R^{10}-COOR^{11}$ (wherein $R^{10}$ is a substituted or unsubstituted, straight-chain or branched divalent hydrocarbon group having from 2 to 20 carbon atoms, and $R^{11}$ is a substituted or unsubstituted, straight-chain or branched monovalent hydrocarbon group having from 1 to 30 carbon atoms);
(D6) an ester group expressed by $-R^{17}-OCOR^{18}$ (wherein $R^{17}$ substituted or unsubstituted, straight-chain or branched divalent hydrocarbon group having from 2 to 20 carbon atoms, and $R^{18}$ is a substituted or unsubstituted, straight-chain or branched monovalent hydrocarbon group having from 1 to 30 carbon atoms);
(D7) $L^1$
here, $L^1$ is a silylalkyl group having a siloxane dendron structure and, when i=1, is expressed by the following general formula (3):

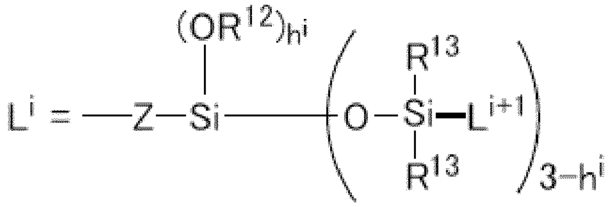

(3)

[Formula 43]

(wherein

$R^{12}$ is a substituted or unsubstituted, straight-chain or branched monovalent hydrocarbon group having from 1 to 30 carbon atoms;
$R^{13}$ moieties each independently represents an alkyl group or a phenyl group having from 1 to 6 carbon atoms;
Z is a divalent organic group;
i represents a generation of the silylalkyl group represented by $L^i$ and is an integer of 1 to k when k is the number of generations, which is the number of repetitions of the silylalkyl group; the number of generations k is an integer from 1 to 10; $L^{i+1}$ is the silylalkyl group when i is less than k, and $R^{13}$ when i=k; and $h^i$ is a number in a range from 0 to 3);

(D8) an alkyl group substituted by a chain polysiloxane structure expressed by the following general formula (4):

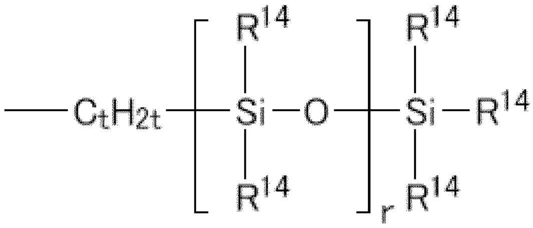

(4)

[Formula 44]

(wherein $R^{14}$ moieties are each independently substituted or unsubstituted, straight-chain or branched monovalent hydrocarbon groups having from 1 to 30 carbon atoms, hydroxyl groups, or hydrogen atoms, and at least one of the $R^{14}$ moieties is the monovalent hydrocarbon group;t is a number in a range from 2 to 10; and r is a number in a range from 1 to 100);
(D9) an epoxy group expressed by the following general formula (5):

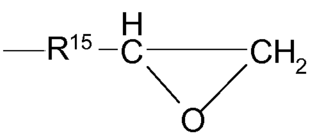

[Formula 45] (5)

(wherein $R^{15}$ is a substituted or unsubstituted, straight-chain or branched divalent hydrocarbon group having from 2 to 20 carbon atoms); and
(D10) a cycloaliphatic epoxy group expressed by the following general formula (6):

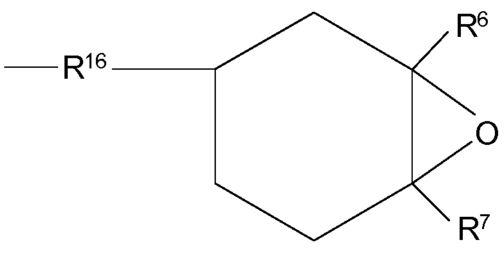

[Formula 46] (6)

(wherein $R^{16}$ is a substituted or unsubstituted, straight-chain or branched divalent hydrocarbon group having from 2 to 20 carbon atoms, and $R^6$ and $R^7$ are each independently a substituted or unsubstituted monovalent hydrocarbon group having from 1 to 30 carbon atoms).

Examples of the substituted or unsubstituted, straight-chain or branched monovalent hydrocarbon group in (D1), (D2), (D5) to (D8), and (D10) include alkyl groups such as methyl groups, ethyl groups, propyl groups, butyl groups, pentyl groups, hexyl groups, heptyl groups, and octyl groups; cycloalkyl groups such as cyclopentyl groups and cyclohexyl groups; alkenyl groups such as vinyl groups, allyl groups, and butenyl groups; aryl groups such as phenyl groups and tolyl groups; aralkyl groups such as benzyl groups; and groups in which the hydrogen atoms bonded to the carbon atoms of these groups are substituted at least partially by halogen atoms such as fluorine atoms or organic groups such as epoxy groups, glycidyl groups, acyl groups, carboxyl groups, amino groups, methacryl groups, and mercapto groups. The monovalent hydrocarbon group is preferably a group other than an alkenyl group, and is particularly preferably a methyl group, an ethyl group, or a phenyl group.

Examples of the substituted or unsubstituted, straight-chain or branched divalent hydrocarbon groups in (D2), (D5), (D6), (D9), and (D10) are as follows. Examples of the substituted or unsubstituted, straight-chain or branched divalent hydrocarbon group having 1 to 30 carbon atoms include: straight-chain or branched alkylene groups having 1 to 30 carbon atoms such as the methylene group, dimethylene group, trimethylene group, tetramethylene group, pentamethylene group, hexamethylene group, heptamethylene group, octamethylene group, or the like; alkenylene groups having 2 to 30 carbon atoms such as the vinylene group, allylene group, butenylene group, hexenylene group, octenylene group, or the like; arylene groups having 6 to 30 carbon atoms such as the phenylene group, diphenylene group, or the like; alkylenearylene groups having 7 to 30 carbon atoms such as the dimethylenephenylene group or the like; and substituted groups thereof in which hydrogen atoms bonded to carbon atoms of the groups are at least partially substituted by a halogen atom such as a fluorine atom or the like, or an organic group containing the carbinol group, epoxy group, glycidyl group, acyl group, carboxyl group, amino group, methacryl group, mercapto group, amide group, oxyalkylene group, or the like. The divalent hydrocarbon groups are preferably alkylene groups having from 1 to 30 carbon atoms, more preferably are alkylene groups having from 1 to 6 carbon atoms, and even more preferably alkylene groups having from 3 to 5 carbon atoms.

Examples of the substituted or unsubstituted, straight or branched alkoxy group in (D3) include lower alkoxy groups such as methoxy groups, ethoxy groups, isopropoxy groups, and butoxy groups and higher alkoxy groups such as lauryl alkoxy groups, myristyl alkoxy groups, palmityl alkoxy groups, oleyl alkoxy groups, stearyl alkoxy groups, and behenyl alkoxy groups.

Among the phenyl group or the alkyl group having from 1 to 6 carbon atoms of (D7), examples of the alkyl group having from 1 to 6 carbon atoms include methyl, ethyl, n-propyl, i-propyl, n-butyl, i-butyl, s-butyl, pentyl, neopentyl, cyclopentyl, hexyl, and similar straight, branched, or cyclic alkyl groups.

In the general formula (3), in the case of i = k, $R^4$ is preferably a methyl group or a phenyl group. In particular, R4 is preferably a methyl group when i = k.

From an industrial standpoint, the number of generations k is preferably an integer from 1 to 3, and more preferably is 1 or 2. In each of the number of generations, the group represented by $L^1$ is expressed as follows. In these formulae, $R^{12}$, $R^{13}$, and Z are groups synonymous with the groups described above.

When the number of generations is k=1, $L^1$ is expressed by the following general formula (3-1).

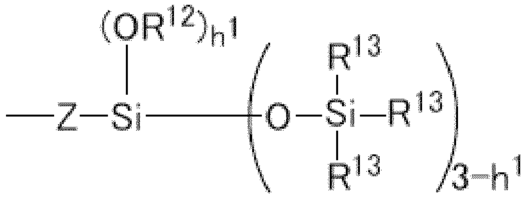

(3-1)

[Formula 47]

When the number of generations is k=2, $L^1$ is expressed by the following general formula (3-2).

[Formula 48]

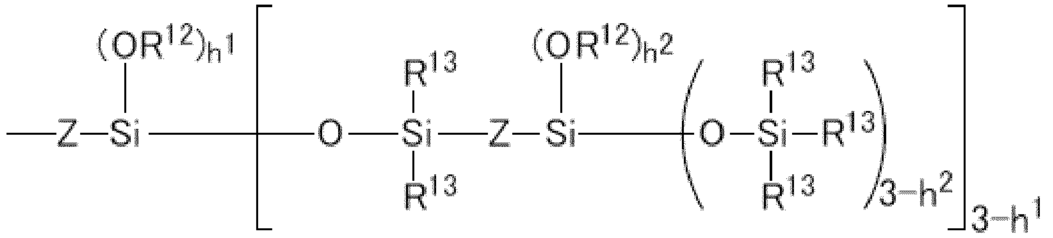

(3-2)

When the number of generations is k=3, $L^1$ is expressed by the following general formula (3-3).

[Formula 49]

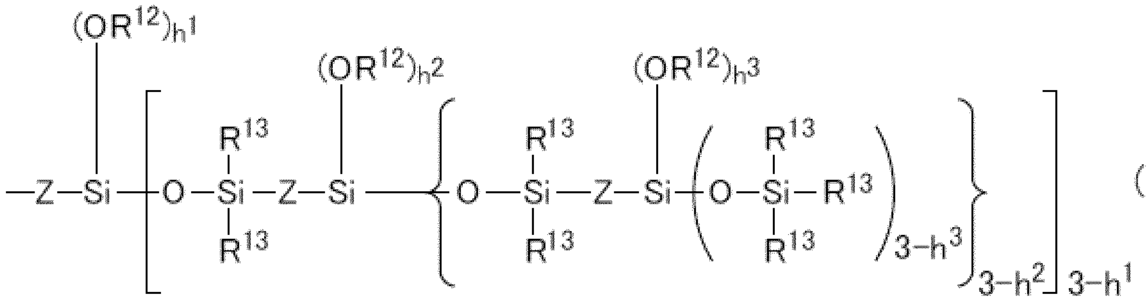

(3-3)

In the structures expressed by the general formulae (3-1) to (3-3) in the case of the number of generations is from 1 to 3, each of $h^1$, $h^2$ and $h^3$ moieties is independently a number in a range from 0 to 3. These $h^i$ moieties are preferably a number in a range from 0 to 1, and $h^i$ is, in particular, preferably 0.

In general formulae (3) and (3-1) to (3-3), Z are each independently a divalent organic group, and specific examples thereof include a divalent organic group formed by addition-reacting a silicon-bonded hydrogen atom and a functional group having an unsaturated hydrocarbon group such as an alkenyl group, an acryloxy group, a methacryloxy group, or the like at the terminal. Depending on the method for introducing the silylalkyl group having a siloxane dendron structure, the functional group can be appropriately selected and is not restricted to the functional groups described above. Preferably, Z are each independently a group selected from divalent organic groups expressed by the following general formula.

[Formula 50]

$$-R^{19}-$$
$$-R^{19}-CO-$$
$$-R^{19}-COO-R^{20}-$$
$$-CO-R^{19}-$$
$$-R^{19}-OCO-R^{20}-$$
$$-R^{19}-CONH-R^{20}-$$
$$-R^{19}-R^{20}-$$

Of these, Z in $L^1$ is preferably a divalent organic group expressed by general formula $-R^{19}-$ that is introduced by a reaction between a silicon-bonded hydrogen atom and an alkenyl group. Likewise, Z is preferably a divalent organic group expressed by general formula $-R^{19}-COO-R^{20}-$ that is introduced by a reaction between a silicon-bonded hydrogen atom and an unsaturated carboxylic ester group. On the other hand, in the silylalkyl group represented by $L^i$, in which the number of generations k is 2 or more, and $L^i$ is $L^2$ to $L^k$, Z is preferably an alkylene group having from 2 to 10 carbon atoms or a divalent organic group represented by $-R^{19}-COO-R^{20}-$ and is particularly preferably a group selected from an ethylene group, a propylene group, a methylethylene group, a hexylene group, and $-CH_2C(CH_3)COO-C_3H_6-$.

In the general formula described above, $R^{19}$ moieties are each independently a substituted or unsubstituted, straight or branched chain alkylene group or alkenylene group having from 2 to 22 carbon atoms or an arylene group having from 6 to 22 carbon atoms. More specifically, examples of $R^{19}$ include an ethylene group, a propylene group, a

butylene group, a hexylene group, and similar straight alkylene groups; a methylmethylene group, a methylethylene group, a 1-methylpentylene group, a 1,4 -dimethylbutylene group, and similar branched alkylene groups. $R^{20}$ is preferably a group selected from an ethylene group, a propylene group, a methylethylene group, and a hexylene group.

In the general formula described above, $R^{20}$ is a group selected from divalent organic groups expressed by the following formula.

[Formula 51]

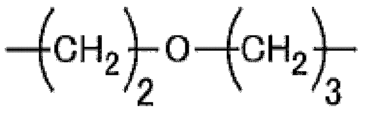

,

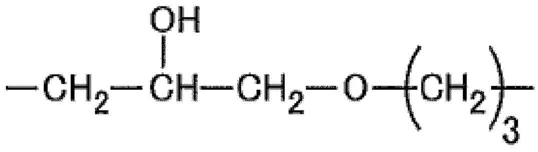

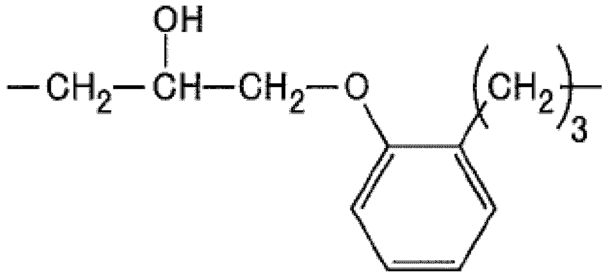

,

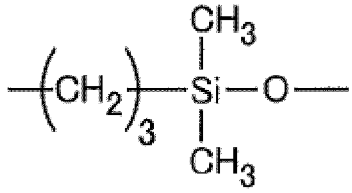

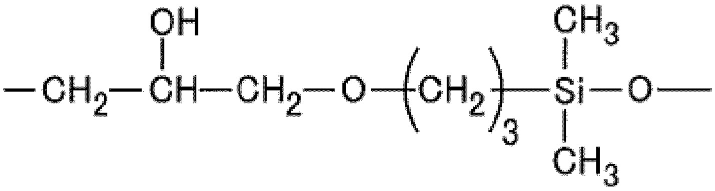

,

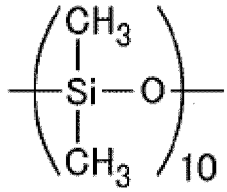

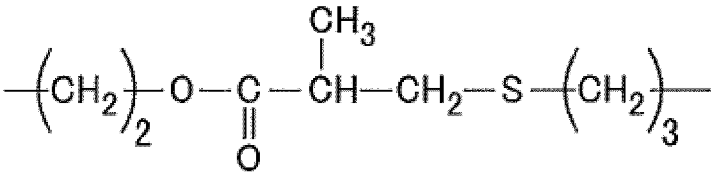

The glycerin derivative group-containing organic compound (B) having a reactive unsaturated group is not particularly limited as long as it has at least one reactive unsaturated group and at least one glycerin derivative group-containing organic group in each molecule, and the compound is preferably a glycerin derivative having carbon-carbon double bonds at the terminals of the molecular chain and more preferably a mono-, di-, tri-, or tetraglycerin derivative. These are glycerin derivatives having reactive functional groups such as an alkenyl group at the molecular chain terminals such as allyl monoglycerol (monoglycerin monoallyl ether), allyl diglycerol (diglycerin monoallyl ether), triglycerin monoallyl ether, triglycerin diallyl ether, or tetraglycerin monoallyl ether, and can be synthesized in accordance with a publicly known method.

There are no particularly restrictions regarding the structure of (C1) the organic compound having an average number of reactive unsaturated groups in each molecule that is greater than 1 serving as the component (C) as long as the compound has more than 1 - preferably from 1.01 to 10, more preferably from 1.2 to 8, even more preferably from 1.5 to 6, and particularly preferably from 2.0 to 4.5 - reactive unsaturated groups and preferably carbon-carbon double bonds on average in each molecule, straight-chain, branched, or reticulated organic compounds may be used. An organopolysiloxane, an unsaturated aliphatic hydrocarbon, or an unsaturated polyether compound is preferable as an organic compound. There are also no restrictions regarding the position of the reactive unsaturated group on the organic compound and preferably the organopolysiloxane, the unsaturated aliphatic hydrocarbon, or the unsaturated polyether compound, and the component may be positioned on the main chain or on a terminal. However, from the perspective of the ease of controlling the crosslinking density, it is preferable to use a compound of high purity having two unsaturated

groups in the molecule, each of which is positioned at either terminal, for example.

A reactive unsaturated group is preferably present in an unsaturated aliphatic hydrocarbon group. The unsaturated aliphatic hydrocarbon group preferably has from 2 to 30 carbon atoms and more preferably has from 2 to 20 carbon atoms. Examples of the monovalent unsaturated aliphatic hydrocarbon group having from 2 to 30 carbon atoms include straight-chain or branched alkenyl groups such as vinyl groups, 1-propenyl groups, allyl groups, isopropenyl groups, 1-butenyl groups, 2-butenyl groups, pentenyl groups, and hexenyl groups; cycloalkenyl groups such as cyclopentenyl groups and cyclohexenyl groups; cycloalkenylalkyl groups such as cyclopentenylethyl groups, cyclohexenylethyl groups, and cyclohexenylpropyl groups; and alkynyl groups such as ethynyl groups and propargyl groups. Alkenyl groups are preferred, and the vinyl group and hexenyl group are particularly preferred.

When the component (C1) is an organopolysiloxane, the unsaturated aliphatic hydrocarbon group containing a reactive unsaturated group is preferably bonded to a silicon atom. In addition, when the component (C1) is an organopolysiloxane, the group bonding to silicon atoms other than the unsaturated aliphatic hydrocarbon may be a substituted or unsubstituted monovalent hydrocarbon group or a monovalent organic group having a reactive functional group.

Substituted or unsubstituted monovalent hydrocarbon groups are typically substituted or unsubstituted, straight or branched monovalent saturated hydrocarbon groups having from 1 to 30 carbon atoms, preferably from 1 to 10 carbon atoms, and more preferably from 1 to 4 carbon atoms, and substituted or unsubstituted monovalent aromatic hydrocarbon groups having from 6 to 30 carbon atoms, and more preferably from 6 to 12 carbon atoms. Moreover, component (C1) may contain, as a monovalent organic group, a hydroxyl group or an alkoxy group having from 1 to 12 carbon atoms, such as a methoxy group, an ethoxy group, a propoxy group or a butoxy group.

Examples of the monovalent saturated hydrocarbon group having from 1 to 30 carbon atoms include straight chain or branched chain alkyl groups such as methyl groups, ethyl groups, n-propyl groups, isopropyl groups, n-butyl groups, isobutyl groups, sec-butyl groups, tert-butyl groups, pentyl groups, hexyl groups, heptyl groups, octyl groups, nonyl groups, decyl groups, and the like; and cycloalkyl groups such as cyclopentyl groups, cyclohexyl groups, cycloheptyl groups, cyclooctyl groups, and the like.

Examples of the monovalent aromatic hydrocarbon group having from 6 to 30 carbon atoms include aryl groups such as phenyl groups, tolyl groups, xylyl groups, mesityl groups, and the like. Of these, a phenyl group is preferable. Note that, in the present specification, "aromatic hydrocarbon group" also includes groups in which an aromatic hydrocarbon and a saturated aliphatic hydrocarbon are conjugated in addition to groups formed only from an aromatic hydrocarbon. Examples of groups in which an aromatic hydrocarbon and a saturated hydrocarbon are conjugated include aralkyl groups such as benzyl groups, phenethyl groups, and the like.

Hydrogen atoms in the above-mentioned monovalent hydrocarbon groups may be substituted by one or more substituted groups, and the substituted groups may be selected from the group consisting of, for example, a halogen atom (a fluorine atom, a chlorine atom, a bromine atom, or an iodine atom), a hydroxyl group, an amide group, an ester group, a carboxyl group and an isocyanate group. A monovalent saturated or aromatic hydrocarbon group having at least one of the above-mentioned substituted groups is preferred. Specifically, it is possible to use a 3,3,3-trifluoropropyl group, a 3-chloropropyl group, a 3-hydroxypropyl group, a 3-(2-hydroxyethoxy)propyl group, a 3-carboxypropyl group, a 10-carboxydecyl group, a 3-isocyanatopropyl group and the like.

Examples of monovalent organic groups having reactive functional groups include monovalent saturated or aromatic hydrocarbon groups having reactive functional groups selected from the group consisting of, for example, hydroxyl groups, mercapto groups, epoxy groups, amino groups, amide groups, ester groups, carboxyl groups and isocyanate groups. One or a plurality of reactive functional groups may exist in the monovalent organic group. $R^1$ is preferably a monosaturated or aromatic hydrocarbon group having at least one of the reactive functional groups described above. Specific examples of the reactive functional group include 3-hydroxypropyl groups, 3-(2-hydroxyethoxy)propyl groups, 3-mercaptopropyl groups, 2,3-epoxypropyl groups, 3,4-epoxybutyl groups, 4,5-epoxypentyl groups, 2-glycidoxyethyl groups, 3-glycidoxypropyl groups, 4-glycidoxybutyl groups, 2-(3,4-epoxycyclohexyl) ethyl groups, 3-(3,4-epoxycyclohexyl)propyl groups, aminopropyl groups, N-methylaminopropyl groups, N-butylaminopropyl groups, N,N-dibutylaminopropyl groups, 3-(2-aminoethoxy)propyl groups, 3-(2-aminoethylamino)propyl groups, 3-carboxypropyl groups, 10-carboxydecyl groups, 3-isocyanate propyl groups, and the like.

A straight-chain or branched polysiloxane is preferable as the component (C1). A straight-chain component (C1) is preferably a polymer having a diorganosiloxane unit and a triorganosiloxane unit, examples of which include dimethylpolysiloxanes capped at both molecular terminals with dimethylvinylsiloxy groups, copolymers of dimethylsiloxane and methylphenylsiloxane capped at both molecular terminals with dimethylvinylsiloxy groups, copolymers of dimethylsiloxane and methylvinylsiloxane capped at both molecular terminals with dimethylvinylsiloxy groups, copolymers of dimethylsiloxane and methylvinylsiloxane capped at both molecular terminals with trimethylsiloxy groups, copolymers of dimethylsiloxane, methylvinylsiloxane and methylphenylsiloxane capped at both molecular terminals with trimethylsiloxy groups, copolymers of dimethylsiloxane and methylvinylsiloxane capped at both molecular terminals with silanol groups, polymers in which some of the methyl groups in these polymers are substituted by alkyl groups other than methyl groups, such as ethyl groups or propyl groups, or halogenated alkyl groups such as 3,3,3-trifluoropropyl

groups, and mixtures of two or more of these polymers, with straight-chain diorganopolysiloxanes having unsaturated aliphatic hydrocarbon groups, and especially alkenyl groups, at both molecular terminals only being particularly preferred.

It is particularly preferable for a branched chain polysiloxane of component (C1) to be a polymer that contains a diorganosiloxane unit, an organosilsesquioxane unit and a triorganosiloxy unit. Silicon-bonded organic groups in these units are preferably monovalent hydrocarbon groups including alkyl groups such as methyl groups, ethyl groups and propyl groups; alkenyl groups such as vinyl groups, allyl groups, butenyl groups and hexenyl groups; aryl groups such as phenyl groups and tolyl groups; and halogenated alkyl groups such as 3,3,3-trifluoropropyl groups, and the like, and may contain extremely small quantities of hydroxyl groups and alkoxy groups such as methoxy groups, but at least two silicon-bonded organic groups in this polymer needs to be unsaturated aliphatic hydrocarbon groups, and especially alkenyl groups. In addition, the proportions of these units are not limited, but in this polymer, it is preferable for diorganosiloxane units to account for in the range of 80.00 to 99.65 mol% and organosilsesquioxane units to account for in the range of 0.10 to 10.00 mol%, with the balance comprising triorganosiloxy units.

Examples of the component (C1) include unsaturated group-containing silicone compounds expressed by the average composition formula (2-5):

$$R^5_pR^6_qSiO_{(4-p-q)/2} \qquad (2-5)$$

(wherein $R^5$ moieties may each be independent from one another but are monovalent organic groups that are different from $R^6$;
$R^6$ moieties are each independently monovalent unsaturated aliphatic hydrocarbon groups having from 2 to 30 carbon atoms, $1 \leq p \leq 2.5$, and $0.001 \leq q \leq 1.5$). The monovalent unsaturated aliphatic hydrocarbon group having from 2 to 30 carbon atoms is as described above.

In the average composition formula (2-5), the monovalent organic group represented by $R^5$ is not particularly limited, but is preferably selected from the following (E1) to (E6):

(E1) a substituted or unsubstituted, straight-chain or branched monovalent hydrocarbon group having from 1 to 60 carbon atoms (excluding monovalent hydrocarbon groups having from 2 to 20 carbon atoms and an aliphatic unsaturated group);
(E2) a hydroxyl group;
(E3) an ester group expressed by $-R^{10}-COOR^{11}$ (wherein $R^{10}$ and $R^{11}$ are as defined above);
(E4) an ester group expressed by $-R^{17}-OCOR^{18}$ (wherein $R^{17}$ and $R^{18}$ are as defined above);
(E5) an amide group expressed by $-R^{21}-NR^{22}COR^{23}$ (wherein $R^{21}$ is a substituted or unsubstituted, straight-chain or branched divalent hydrocarbon group having from 2 to 20 carbon atoms, $R^{22}$ is a hydrogen atom, or a substituted or unsubstituted, straight-chain or branched monovalent hydrocarbon group having from 1 to 20 carbon atoms, and $R^{23}$ is a substituted or unsubstituted, straight-chain or branched monovalent hydrocarbon group having from 1 to 30 carbon atoms); and
(E6) an amide group expressed by $-R^{24}-CONR^{25}R^{26}$ (wherein $R^{24}$ is a substituted or unsubstituted, straight-chain or branched divalent hydrocarbon group having from 2 to 20 carbon atoms, and $R^{25}$ and $R^{26}$ are each independently a hydrogen atom or a substituted or unsubstituted, straight-chain or branched monovalent hydrocarbon group having from 1 to 20 carbon atoms).

The definitions, types, and the like of the substituted or unsubstituted, straight-chain or branched monovalent hydrocarbon groups or divalent hydrocarbon groups are as described above.

On the other hand, the component (C1) may be an unsaturated aliphatic hydrocarbon. Examples of unsaturated aliphatic hydrocarbons include various dienes, diynes, enynes, and similar products having two or more reactive unsaturated groups. In view of crosslinking, dienes, diynes, and enynes are preferable. Dienes, diynes, and enynes are compounds having a structure in which at least two reactive unsaturated groups are separated by one or more, and preferably two or more single bonds in a molecule. The unsaturated aliphatic hydrocarbon group may be present at the terminal of the molecular chain, or as a pendant group in the molecular chain.

Examples of unsaturated aliphatic hydrocarbons serving as the component (C1) include α,ω-unsaturated alkenes and alkynes having from 2 to 30 carbon atoms. Examples of the component (C1) include an α,ω-diene expressed by the general formula (2-1):

$$CH_2=CH(CH_2)_xCH=CH_2 \qquad (2-1)$$

(wherein $1 \leq x \leq 20$); an α,ω-diyne expressed by the general formula (2-2):

$CH \equiv C(CH_2)_xC \equiv CH$ (2-2)

(wherein $1 \leq x \leq 20$); and an $\alpha,\omega$-ene-yne expressed by the general formula (2-3):

$CH_2=CH(CH_2)_xC \equiv CH$ (2-3)

(wherein $1 \leq x \leq 20$).

An example of the unsaturated polyether compound serving as the component (C1) is an $\alpha,\omega$-unsaturated polyether. Examples of the component (C1) include a bisalkenyl polyether compound expressed by the general formula (2-4):

$C_mH_{2m-10}(C_nH_{2n}O)_yC_mH_{2m-1}$ (2-4)

(wherein $2 \leq m \leq 20$, $2 \leq n \leq 4$, y is the total value of the number of repetitions of the oxyethylene unit, the oxypropylene unit, and the oxybutylene unit, and $1 \leq y \leq 180$).

Specific examples of unsaturated aliphatic hydrocarbons serving as the component (C1) include 1,4-pentadiene, 1,5-hexadiene, 1,6-heptadiene, 1,7-octadiene, 1,8-nonadiene, 1,9-decadiene, 1,11-dodecadiene, 1,13-tetradecadiene, 1,19-eicosadiene, 1,3-butadiene, 1,5-hexadiyne, and 1-hexene-5-yne.

The component (C1) may be a single component, but may also be a mixture of two or more components having different structures. That is, the component (C1) may be a mixture of one or more types of organopolysiloxanes and one or more types of unsaturated aliphatic hydrocarbons. Therefore, "having a number of reactive unsaturated groups greater than 1 on average" means having more than one reactive unsaturated group per molecule when two or more types of at least one of organopolysiloxanes and unsaturated aliphatic hydrocarbons are used.

The (C2) organic compound having at least one reactive unsaturated group and at least one epoxy group in the molecule serving as the component (C) is not structurally limited as long as the compound has a total of two or more - preferably from 2 to 10, more preferably from 2 to 7, even more preferably from 2 to 5, and particularly preferably from 2 to 4 - reactive unsaturated groups and epoxy groups in each molecule, and straight-chain, branched, or reticulated organic compounds can be used. An organopolysiloxane or an unsaturated aliphatic hydrocarbon is preferable as an organic compound. There are also no restrictions regarding the position of the reactive unsaturated group on the organic compound and preferably the organopolysiloxane or the unsaturated aliphatic hydrocarbon, and the component may be positioned on the main chain or on a terminal. However, from the perspective of the ease of controlling the crosslinking density, it is preferable to use a compound of high purity in which the total of unsaturated groups and epoxy groups in the molecule is two.

A reactive unsaturated group is preferably present in an unsaturated aliphatic hydrocarbon group. Examples of unsaturated aliphatic hydrocarbon groups are as described above.

When the component (C2) is an organopolysiloxane, at least one of the unsaturated aliphatic hydrocarbon group containing a reactive unsaturated group and the epoxy group-containing organic group is preferably bonded to a silicon atom. In addition, when the component (C2) is an organopolysiloxane, the group bonding to silicon atoms other than the unsaturated aliphatic hydrocarbon or the epoxy group-containing organic group may be a substituted or unsubstituted monovalent hydrocarbon group or a monovalent organic group having a reactive functional group as described above.

The component (C2) is preferably an epoxy group-containing unsaturated aliphatic hydrocarbon having at least one epoxy group. Examples of the unsaturated aliphatic hydrocarbon include compounds having the unsaturated aliphatic hydrocarbon groups described above. A compound having a monovalent unsaturated aliphatic hydrocarbon group is preferable.

Examples of the component (C2) include an unsaturated epoxy compound expressed by the general formula (2-6):

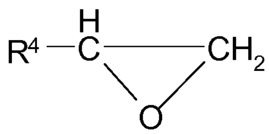

[Formula 52] (2-6)

(wherein $R^4$ has one reactive unsaturated group and is a substituted or unsubstituted, straight-chain or branched monovalent hydrocarbon group having from 2 to 20 carbon atoms); and an unsaturated group-containing alicyclic epoxy compound represented by the general formula (2-7):

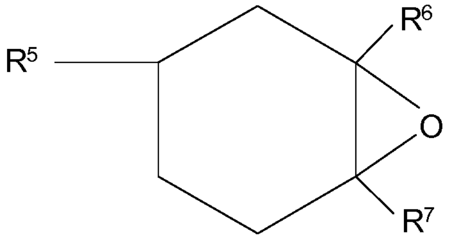

[Formula 53] (2-7)

(wherein $R^5$ has one reactive unsaturated group and is a substituted or unsubstituted, straight-chain or branched monovalent hydrocarbon group having from 2 to 20 carbon atoms; and
$R^6$ and $R^7$ area each independently a hydrogen atom or a substituted or unsubstituted monovalent hydrocarbon group having from 1 to 30 carbon atoms). The definitions, types, and the like of the reactive unsaturated groups in the general formulas above and the substituted or unsubstituted, straight-chain or branched monovalent hydrocarbon groups are as described above.

Specific epoxy group-containing unsaturated aliphatic hydrocarbons serving as the component (C2) include an allylglycidylether, methallylglycidylether, 1-methyl-4-isopropenylcyclohexene oxide, 1,4-dimethylcyclohexene oxide, 4-vinylcyclohexene oxide, vinylnorbornene monooxide, dicyclopentadiene monooxide, butadiene monooxide, 1,2-epoxy-5-hexene, 1,2-epoxy-9-decene, and 2,6-dimethyl-2,3-epoxy-7-octene. Among these, 4-vinyl cyclohexane oxide is preferable.

The component (C2) may be a single component, but may also be a mixture of two or more components having different structures.

The reaction for producing the glycerin derivative-modified silicone described above can be performed in accordance with a publicly known method in the presence or absence of a reaction solvent. The reaction between the unsaturated group and the Si-H group in the present invention is a hydrosilylation reaction. In addition, when crosslinking is performed using an epoxide of (C2) the organic compound having one or more reactive unsaturated groups and one or more epoxy groups in each molecule, bonding caused by the reaction of the unsaturated group and the Si-H group and ether bond generation caused by the self ring-opening polymerization of the epoxy groups (cationic polymerization reaction that occurs in the presence of a SiH group and a platinum catalyst) both occur, resulting in crosslinking. In order to accelerate this reaction, irradiation using high energy beams such as ultraviolet light can be applied, or a common cation polymerization catalyst can be further added.

The reaction solvent is not particularly limited as long as the solvent is non-reactive, and examples thereof include alcohol-based solvents such as ethanol and isopropyl alcohol; aromatic hydrocarbon-based solvents such as toluene and xylene; ether-based solvents such as dioxane and THF; aliphatic hydrocarbon-based solvents such as n-hexane, cyclohexane, n-heptane, cycloheptane, and methylcyclohexane; and chlorinated hydrocarbon-based organic solvents such as carbon tetrachloride. An oil agent described below may also be used as a reaction solvent. When an oil agent is used as a reaction solvent, it is possible to directly obtain a composition consisting of an oil agent and a liquid organo-modified silicone having a silicon-bonded glycerin derivative group-containing group and having a crosslinked structure containing a carbon-silicon bond in a crosslinking portion.

The hydrosilylation reaction may be performed in the presence or absence of a catalyst, but preferably is performed in the presence of a catalyst because the reaction can be carried out at a low temperature and in a shorter period of time. Examples of the hydrosilylation reaction catalyst include platinum, ruthenium, rhodium, palladium, osmium, iridium, and similar compounds, and platinum compounds are particularly effective due to their high catalytic activity. Examples of the platinum compound include chloroplatinic acid; platinum metal; platinum metal supported on a carrier such as platinum supported on alumina, platinum supported on silica, platinum supported on carbon black, or the like; and a platinum complex such as platinum-vinylsiloxane complex, platinum-phosphine complex, platinum-phosphite complex, platinum alcoholate catalyst, or the like. A usage amount of the catalyst is about 0.5 to 1000 ppm in terms of platinum metal, when using a platinum catalyst.

A reaction temperature of the hydrosilylation reaction is typically from 30 to 150°C, and a reaction time is typically from 10 minutes to 24 hours and preferably from 1 to 10 hours.

The component (A) is crosslinked by the component (C) as a result of the hydrosilylation reaction or the cationic polymerization reaction of the epoxy groups, and the polysiloxane chains originating from the component (A) are linked by the crosslinking portion having a carbon-silicon bond originating from the component (C). In addition, the component (A) is provided with a glycerin derivative group-containing organic group originating from the component (B). In this way, it is possible to obtain the liquid glycerin derivative-modified silicone of the present invention having a silicon-bonded glycerin derivative group-containing organic group and having a crosslinked structure containing a carbon-silicon bond

in a crosslinking portion.

Furthermore, the liquid glycerin derivative-modified silicone of the present invention having a silicon-bonded glycerin derivative group-containing organic group and having a crosslinked structure containing a carbon-silicon bond in a crosslinking portion essentially has a linked structure formed by the crosslinking portion containing a carbon-silicon bond originating from the component (C), but it may also have a portion crosslinked by the Si-O-C bond. This is because when the structure has a condensation-reactable functional group such as a silanol group or an alkoxy group in the components (A) to (C), links can not only be formed between polysiloxane chains but can also be formed intermittently as a result of a partial reaction between the hydroxyl groups in the glycerin derivative group-containing organic group originating from the component (B) and the Si-H groups of (A) when the crosslinking conditions are severe.

In the production of the liquid glycerin derivative-modified silicone of the present invention having a silicon-bonded glycerin derivative group-containing organic group and having a crosslinked structure containing a carbon-silicon bond in a crosslinking portion, the component (C) may be further reacted with the component (A) after a reaction between the component (A) and the component (B), or the component (B) may be further reacted with the component (A) after a reaction between the component (A) and the component (C).

When the component (C) is further reacted with the component (A) after the reaction between the component (A) and the component (B), the average value of the number of silicon-bonded hydrogen atoms per molecule of the component (A) reacting with the reactive unsaturated groups of the component (C) is preferably at least 1.0. That is, the number of silicon-bonded hydrogen atoms per molecule of the component (A) which constitute the crosslinking portion and react with the reactive unsaturated groups in the component (C) is, on average, at least 1.0, preferably within a range of from 0.2 to 1.5, and particularly preferably within a range of from 0.6 to 1.3.

(Glycerin derivative group-containing alternating copolymer)

The glycerin derivative-modified silicone may be a glycerin derivative-modified silicone in the form of a straight-chain glycerin derivative group-containing alternating copolymer obtained by reacting at least:

(D) an organopolysiloxane having reactive functional groups at both terminals of a molecular chain; and
(E) an organic compound having two reactive functional groups capable of reacting with the reactive functional groups positioned at both of the molecular chain terminals of the organopolysiloxane (D) in the molecule.

The combination of reactive functional groups is not particularly limited, but examples include combinations of Si-H groups and C=C groups. In this case, it is preferable for the glycerin derivative portion to be contained in the molecule of (E). An example of such a glycerin derivative group-containing alternating copolymer is the composition described in Japanese Unexamined Patent Application Publication No. 2005-42097A, and the description of this publication is incorporated herein by reference.

In order to prevent oxidative degradation, the oxidative stability can be increased by blending antioxidants such as phenols, hydroquinones, benzoquinones, aromatic amines, or vitamins into the high-purity glycerin derivative-modified silicone obtained with the manufacturing method of the present invention. In the case of applications such as cosmetics and external use preparations, adding BHT(2,6-di-t-butyl-p-cresol), vitamin E, or the like, for example, will result in a further increase in stability. In this case, an added amount of the antioxidant that is used is in a range (by weight (mass)) from 10 to 1,000 ppm, and preferably from 50 to 500 ppm, of the high-purity glycerin derivative-modified silicone.

(Manufacturing method of solution containing high-purity glycerin derivative-modified silicone)

In the manufacturing method of the present invention, when the mixture of the glycerin derivative-modified silicone and impurities - in particular, impurities originating from the organic modifier - contains the solvent of the glycerin derivative-modified silicone, a solution containing a high-purity glycerin derivative-modified silicone can be produced.

Any solvent can be used as long as it satisfies the condition of being a fluid that is a good solvent for the glycerin derivative-modified silicone, but it preferably further satisfies the condition of being a fluid that is a good solvent for the glycerin derivative-modified silicone and a poor solvent for the impurities. For example, the solvent is one or more oil agents selected from various silicone oils and organo-modified silicones in a liquid state at normal temperature to 100°C, organomodified silane compounds such as silane coupling agents, and non-polar organic compounds or lowly polar to highly polar organic compounds, and it may be volatile or nonvolatile. A silicone oil agent or a silane coupling agent is optimal as the solvent, but of non-polar organic compounds and lowly polar to highly polar organic compounds, hydrocarbon oils, fatty acid ester oils, and liquid fatty acid triglycerides are preferable. In addition, the solvent may be a mixed fluid of a silicone oil agent and an organic compound.

The timing of adding the solvent to the mixture containing the glycerin derivative-modified silicone as a main component and containing impurities - in particular, impurities originating from the organic modifier serving as a starting

material of the glycerin derivative-modified silicone - may be before, after, or during the treatment with the organic wax. Furthermore, the mixture may already contain the solvent at the stage when the mixture undergoes treatment with an acidic aqueous solution as described below (including before, after, and during treatment). A solution containing the high-purity glycerin derivative-modified silicone of the present invention can be produced in the same manner as in the manufacturing method for a high-purity glycerin derivative-modified silicone described above in all other respects.

In order to prevent oxidative degradation, the antioxidants described above may be blended into the high-purity glycerin derivative-modified silicone obtained with the manufacturing method of the present invention. The compounding ratios of the antioxidants are also as described above.

(Acid treatment and odor reduction of mixture containing glycerin derivative-modified silicone and impurities)

In the manufacturing method of the present invention, when the mixture containing the glycerin derivative-modified silicone and impurities - in particular, impurities originating from the organic modifier - is treated with an acidic aqueous solution and water and odor-causing substances produced by treatment with the acidic aqueous solution are removed by heating or depressurization, it is possible to obtain a glycerin derivative-modified silicone of even higher purity.

The acidic substance contained in the acidic aqueous solution can be selected optionally, but it is optimal to use one or more types of acidic inorganic salts which are solids at 25°C, are water-soluble, and have an aqueous solution pH of at most 4 at 25°C when 50 g is dissolved in 1 L of ion exchanged water. In addition, when treatment is performed using this acidic aqueous solution, it is preferably performed prior to the treatment for increasing purity using the organic wax, but it may also be performed after or at the same time as the treatment for increasing purity using the organic wax.

Furthermore, treatment using the acidic aqueous solution can be most preferably performed when the glycerin derivative-modified silicone is synthesized by a hydrosilylation reaction. Therefore, the case of a glycerin derivative-modified silicone synthesized by a hydrosilylation reaction will be described hereinafter as an example of an acid treatment and odor reducing method for a glycerin derivative-modified silicone and a mixture containing the same.

Acid treatment preferably includes:

a process (V) of synthesizing a glycerin derivative-modified silicone or a reaction mixture containing the same as a main component by performing a hydrosilylation reaction on:

(ax) a glycerin derivative having carbon-carbon double bonds at the terminals of the molecular chain; and
(bx) an organohydrogenpolysiloxane; and

together with the synthesis process (V) or after the synthesis process (V),
a process (W) of treating the glycerin derivative-modified silicone or a reaction mixture containing the same as a main component
in the presence of at least one type of acidic inorganic salts which are solids at 25°C, are water-soluble, and have an aqueous solution pH of at most 4 at 25°C when 50 g is dissolved in 1 L of ion exchanged water.

In addition, because a treatment process that uses the acidic inorganic salt involves the generation of odor-causing substances it is more preferable to include a process of removing odor-causing substances by heating or depressurizing after process (W), from the perspective of odor reduction effectiveness.

For example, in process (V), when the hydrosilylation reaction is performed using (ax) a glycerin derivative such as (poly)glycerin monoallyl ether and (bx) a straight-chain organohydrogenpolysiloxane represented by the structural formula (1-1A) in amounts so that there is an excessive amount of the substance of the component (ax) with respect to the silicon-bonded hydrogen atoms in the component (bx), the glycerin derivative-modified silicone represented by the structural formula (1-1) is synthesized, and a crude product of a reaction mixture containing the glycerin derivative-modified silicone and the unreacted component (ax) and containing the glycerin derivative-modified silicone as a main component is obtained.

Process (W) is a process for efficiently reducing the odors of the composition highly effectively and effectively suppressing the generation of odors over time by hydrolyzing the crude product using specific acidic inorganic salts, with practically no breakage of the silicon-oxygen bonds forming the main chain of polysiloxane or the carbon-oxygen bonds of side chain portions.

Process (W) specifically removes odor-causing substances from the crude product of the reaction mixture containing the glycerin derivative-modified silicone as a main component by using hydrolysis, and it is characterized by performing treatment in the presence of one or more types of acidic inorganic salts which are solids at 25°C, are water-soluble, and have an aqueous solution pH of at most 4 at 25°C when 50 g is dissolved in 1 L of ion exchanged water. Note that pH values in the present invention are values that are measured using a pH meter having a glass electrode in a sample aqueous solution at room temperature (25°). In the present application, HM-10P produced by DKK-TOA

Corporation was used for the pH measurement.

The acidic inorganic salt serving as a component (cx) needs to be a solid at 25°, needs to be water-soluble, and the aqueous solution needs to have a pH of at most 4 when 50 g of the acidic inorganic salt is dissolved in 1 L of ion exchanged water. The pH is preferably at most 3.5 and particularly preferably at most 2.0. By using such a water-soluble acidic inorganic salt for hydrolysis treatment of the composition, it is possible to reduce odors in the composition highly effectively and suppress odorization over time effectively, with almost no breakage of C-O bonds or Si-O bonds.

Examples that can be used as the acidic inorganic salt include acidic inorganic salts in which at least a monovalent hydrogen atom of the inorganic acid that is at least divalent is neutralized by a base. Examples of the inorganic acid that is at least divalent include sulfuric acid, sulfurous acid, and the like. Examples of the base include an alkali metal, ammonia, and the like.

More specifically, the component (cx) is preferably at least one type of acidic inorganic salt comprising a hydrogensulfate ion ($HSO_4^-$) or a hydrogensulfite ion ($HSO_3^-$) and a monovalent cation ($M^+$). Examples of the monovalent cation ($M^+$) include alkali metal ions or an ammonium ion. Particularly, the monovalent cation is preferably at least one type selected from the group consisting of a sodium ion, a potassium ion, and an ammonium ion. Additionally, one type of the acidic inorganic salt may be used alone or two or more types of acidic inorganic salt may be used. Furthermore, the acidic inorganic salt can be easily removed via filtration because the acidic inorganic salt is solid at room temperature (25°C). Additionally, because it is water soluble, the acidic inorganic salt can be easily rinsed off using water, even in the cleaning process after production.

On the other hand in hydrolysis treatment based on an acetic acid salt, phosphoric acid salt, and the like that does not satisfy the conditions of the component (cx), it is impossible to sufficiently reduce the odor of the composition after hydrolysis. On the other hand, in hydrolysis treatment based on a strong acid such as hydrochloric acid and the like, and in hydrolysis treatment based on a publicly known solid acid of zirconium sulfate and the like, the odor can be reduced by a certain amount, but C-O bonds and Si-O bonds of the composition break easily at the time of hydrolysis.

Specific examples of the acidic inorganic salt serving as the component (cx) are lithium hydrogensulfate, sodium hydrogensulfate, potassium hydrogensulfate, rubidium hydrogensulfate, cesium hydrogensulfate, ammonium hydrogensulfate, sodium hydrogensulfite, or hydrates thereof. The pH of aqueous solutions in which 50 g of the acidic inorganic salt is dissolved in 1 L of ion exchanged water is as shown in Table below. From the perspective of the technical benefit of reducing odor, the water soluble acidic inorganic salt having a pH of not higher than 2.0 is most preferably at least one type of acidic inorganic salt selected from the group consisting of sodium hydrogensulfate, potassium hydrogensulfate, and ammonium hydrogensulfate.

[Table 1]

| Acidic inorganic salt | pH (50 g/L) |
|---|---|
| Sodium hydrogensulfate | 1.5 or lower |
| Potassium hydrogensulfate | 2.0 or lower |
| Ammonium hydrogensulfate | 1.5 or lower |
| Sodium hydrogensulfite | 3.5 |

For example, treatment in the presence of an acidic inorganic salt refers to (1) decomposition treatment involving adding and stirring the acidic inorganic salt into the reaction system (for example, a reaction vessel such as a flask) of the reaction mixture containing the glycerin derivative-modified silicone synthesized by a hydrosilylation reaction as a main component, and (2) hydrolysis treatment or the like involving adding and stirring an acidic inorganic salt and water or an acidic inorganic salt, water, and a hydrophilic solvent. The treatment process that uses the acidic inorganic salt is preferably carried out in the presence of at least one of water and a hydrophilic solvent.

A particularly preferable hydrolysis treatment is a hydrolysis treatment whereby, after the process (V), at least an acidic inorganic salt and water are added to a reaction system containing a crude product of the reaction mixture containing the glycerin derivative-modified silicone as a main component, and depending on the case, another hydrophilic solvent is further added with the objective of increasing the treatment efficiency by improving computability, and the solution is further stirred using a mechanical force. The hydrolysis treatment can be carried out at any temperature and treatment time, and can be carried out at a temperature from 0 to 200°C and more preferably from 50 to 100°C for a reaction time of from 0.1 to 24 hours and more preferably from about 0.5 to 10 hours. The amount of the acidic inorganic salt that is used can be selected appropriately in accordance with the treatment apparatus and the treatment time. However, the amount is preferably within a range of from 50 to 10,000 ppm and more preferably within a range of from 100 to 5,000 ppm with respect to the reaction mixture containing the glycerin derivative-modified silicone as a main component.

After the acid treatment described above, it is preferable to include a stripping process in which low-boiling-point components (propionaldehyde and the like), which are odor-causing substances, are removed. In addition, after stripping, it is possible to hydrolyze more of the propenyl ether group-containing glycerin derivative or the like by treating again in the presence of an acidic inorganic salt, and propionaldehyde and the like, which are odor-causing substances, can be removed. At this time, there is an advantage that, because acidic inorganic salt remains, an acidic inorganic salt need not be newly added. Therefore, it is only necessary to add a hydrophilic solvent, typified by water. That is, the aforementioned process [W] and the stripping process can be repeated two times or more, to increase the degree of odor reduction, or the like.

Furthermore, the "materials with a low boiling point" which are distilled off by the stripping process, include not only propionaldehyde which is an odor-causing substance, but also the reaction solvents used in the hydrosilylation reaction (process [V]), the water used in the odor reduction treatment process, hydrophilic solvents, and the like.

The stripping process (removal of low-boiling-point substances) may be performed on the crude product of the reaction mixture containing the glycerin derivative-modified silicone as a main component as the process preceding process (W), or may be performed on the reaction mixture containing the glycerin derivative-modified silicone as a main component as the process following process (W). In addition, the stripping process can be performed as the pre processing and post processing of process [W]. The stripping process is preferably performed after the process [W], to remove propionaldehyde, which is an odor-causing substance generated by the hydrolysis reaction.

As the removal method, stripping under normal pressure or under reduced pressure is preferable, and stripping at a temperature of 120°C or lower is preferable. In order to effectively perform the stripping, the stripping is preferably performed under reduced pressure or, for example, performed under a nitrogen gas or similar inert gas stream. A specific example of the operation for removing low-boiling-point matter is one in which a crude product of the reaction mixture containing the glycerin derivative-modified silicone containing the low-boiling-point matter as a main component is placed in a flask having a refluxing cooler, a nitrogen injection port, or the like; and, while supplying nitrogen gas, the internal pressure is reduced, and the internal temperature is increased and the pressure and temperature are maintained so as to be constant. Thus, the light matter is removed. Here, typically, a pressure reduction parameter is from 0.1 to 10.0 kPa, a heating temperature is from 40 to 120°C, and a treatment time is from 10 minutes to 24 hours.

Furthermore, after the acid treatment process, a basic substance may be used to neutralize the reaction mixture containing the glycerin derivative-modified silicone as a main component. Examples of the basic substance include sodium hydroxide, potassium hydroxide, calcium hydroxide, barium hydroxide, ammonia water, sodium hydrogen carbonate, and similar inorganic salt groups; various amines, basic amino acids, and similar organic bases; and the like. The amount of the basic substance is preferably an amount needed to neutralize a reaction system comprising the reaction mixture containing the glycerin derivative-modified silicone as a main component but, as necessary, the amount of the basic substance may be adjusted to an amount by which weak acidity or weak alkalinity is obtained.

In addition, an alkaline buffer may be further added in an amount corresponding to 100 ppm to 50,000 ppm to the reaction mixture containing the glycerin derivative-modified silicone obtained after the acid treatment process as a main component. A minute amount of acid may be locally dissolved in the reaction mixture containing the glycerin derivative-modified silicone as a main component even after a neutralization or filtration process. By adding an alkaline buffer, the liquidity of the cosmetic or the like into which the glycerin derivative-modified silicone is blended is maintained on the alkali side, which makes it possible to reduce the risk of odorization caused by the impurities of the glycerin derivative-modified silicone. A useful alkaline buffer is not particularly limited as long as the alkaline buffer comprises a combination of a strong base and a weak acid. Examples of the alkaline buffer include trisodium phosphate, tripotassium phosphate, trisodium citrate, sodium acetate, and other alkaline buffers. Furthermore, these alkaline buffers may be added to a cosmetic composition starting material or the like comprising a glycerin derivative-modified silicone or a mixture containing the same as a main component, or they may be added to a composition at the preparation stage or after the blending of a glycerin derivative-modified silicone or cosmetic composition that contains another cosmetic composition starting material or water. However, in the present invention, treatment for increasing purity using an organic wax, which is a feature of the present invention, is performed after treatment is performed in the presence of an acidic solution containing water as necessary on the glycerin derivative-modified silicone or the mixture containing the same as a main component, so sufficient deodorization is achieved together with high purity. Therefore, as long as the manufacturing method of the present invention is used, the need to further add an alkaline buffer to suppress odorization over time is low.

The glycerin derivative-modified silicone or the mixture containing the same as a main component can also be subjected to hydrogenation treatment as a process before or after treatment in the presence of an acidic inorganic salt in process (W). A deodorizing treatment using a hydrogenation reaction may be performed after treatment in the presence of the acidic inorganic salt of the process (W). On the other hand, the treatment in the presence of the acidic inorganic salt of the process (W) may be performed after deodorizing treatment using a hydrogenation reaction. However, hydrogenation treatment typically leads to an increase in the cost of the product over time. In the present invention, treatment for increasing purity using an organic wax, which is a feature of the present invention, is performed after treatment is performed in the presence of an acidic solution containing water as necessary on the glycerin derivative-modified silicone

or the mixture containing the same as a main component, so deodorization surpassing that of hydrogenation treatment is achieved together with high purity. Therefore, as long as the manufacturing method of the present invention is used, it is meaningless to further perform hydrogenation treatment for the purpose of deodorization.

A second aspect of the present invention is an external use preparation, a cosmetic, or an industrial material containing the high-purity glycerin derivative-modified silicone obtained by the manufacturing method of the present invention.

<External use preparation/cosmetic>

The high-purity glycerin derivative-modified silicone obtained by the manufacturing method of the present invention can be suitably blended into an external use preparation or a cosmetic and can form the external use preparation or cosmetic of the present invention. In addition, it is also possible to produce a starting material for external use preparations and cosmetics containing the high-purity glycerin derivative-modified silicone obtained by the manufacturing method of the present invention and to blend the starting material into an external use preparation or a cosmetic.

In particular, the high-purity glycerin derivative-modified silicone obtained by the manufacturing method of the present invention has no specific odor and demonstrates practically no odorization during formulation or over time. Moreover, there is the advantage of breaking almost no silicon-oxygen bonds which may form the main chain of the glycerin derivative-modified silicone and the carbon-oxygen bonds which may form the side chains. Therefore, the high-purity glycerin derivative-modified silicone obtained by the manufacturing method of the present invention can be suitably used as a starting material for external use preparations and cosmetics used on the human body.

The high-purity glycerin derivative-modified silicone may also be diluted with an appropriate medium such as a silicone oil, an organic oil, or an alcohol and used as a starting material of an external use preparation or a cosmetic. The proportion of the high-purity glycerin derivative-modified silicone in the starting material for an external use preparation or a cosmetic is preferably from 10 to 100 wt.% (mass%), more preferably from 20 to 100 wt.% (mass%), and even more preferably from 30 to 100 wt.% (mass%) relative to the total weight (mass) of the starting material. The proportion of the starting material compounded in the external use preparation or the cosmetic composition is not particularly limited but, for example, can be from 0.1 to 40 wt.% (mass%), and is preferably from 1 to 30 wt.% (mass%), more preferably from 2 to 20 wt.% (mass%), and even more preferably from 3 to 10 wt.% (mass%) based on the total weight (mass) of the external use preparation or the cosmetic composition.

The high-purity glycerin derivative-modified silicone obtained by the manufacturing method of the present invention can be applied, depending on the structure thereof and kinds of possessed functional group, to applications common to the co-modified organopolysiloxanes described in Patent Document 14 (WO2011/049248), Patent Document 15 (WO2011/049247), and Patent Document 17 (Japanese Unexamined Patent Application Publication No. 2012-046507A) or the novel organopolysiloxane copolymer described in Patent Document 16 (WO2011/049246). In addition, the high-purity glycerin derivative-modified silicone obtained by the manufacturing method of the present invention can be used in the same manner as the co-modified organopolysiloxanes described in Patent Documents 14, 15, and 17 and the novel organopolysiloxane copolymer described in Patent Document 16 with regard to combinations with any cosmetic starting material components, external use preparations, and, in particular, formulations, types, and formulation examples of cosmetics, and can be blended into various cosmetics or the like.

The external use preparation of the present invention is not particularly limited, provided that it is a composition applied to the human body as a cosmetic or a medicament. Specific examples of cosmetic composition products of the present invention include skin cleansing agent products, skin care products, makeup products, anti-perspirant products, ultraviolet light blocking products, and similar skin use cosmetic products; hair use cleansing agent products, hair dressing products, hair use coloration products, hair growth products, hair rinsing products, hair conditioning products, hair treatment products, and similar hair use cosmetic products; and bath use cosmetic products. Examples of the medicament of the present invention include hair regrowth agents, hair growth promoters, analgesics, germicides, anti-inflammatory agents, refreshing agents, and skin anti-aging agents, but are not limited thereto.

The external use preparation is a product to be applied to human skin, nails, hair, and the like and, for example, medicament active components can be compounded therein and used in the treatment of various disorders. The cosmetic composition is also a product to be applied to human skin, nails, hair, and the like, and is used for beauty purposes. The external use preparation or cosmetic composition is preferably an anti-perspirant, a skin cleansing agent, a skin conditioner, a skin cosmetic composition product, a hair cleansing agent, an external use preparation for hair or a hair cosmetic composition.

The anti-perspirant, skin cleansing agent, skin external use preparation, or skin cosmetic composition of the present invention contains the high-purity glycerin derivative-modified silicone obtained by the manufacturing method of the present invention, and the form thereof is not particularly limited, but may be in the form of a solution, milk-like, cream-like, solid, semi-solid, paste-like, gel-like, powder-like, multi-layer, mousse-like, or a water-in-oil or oil-in-water emulsion composition. Specific examples of the skin external use preparation or the skin cosmetic composition product

according to the present invention include toilet water, emulsions, creams, sunscreen emulsions, sunscreen creams, hand creams, cleansing compositions, massage lotions, cleansing agents, anti-perspirants, deodorants, and similar basic cosmetic products; foundations, make-up bases, blushers, rouges, eye shadows, eye liners, mascaras, nail enamels, and similar make-up cosmetic products; and the like.

Similarly, the hair cleansing agent, hair external use preparation or the hair cosmetic composition product according to the present invention contains the high-purity glycerin derivative-modified silicone obtained by the manufacturing method of the present invention and can be used in various forms. For example, the hair cleansing agent, the hair external use preparation or the hair cosmetic composition product according to the present invention may be dissolved or dispersed in an alcohol, a hydrocarbon, a volatile cyclic silicone, or the like and used; furthermore, these may be used in the form of an emulsion by dispersing a desired emulsifier in water. Additionally, the hair cleansing agent, the hair external use preparation or the hair cosmetic composition product according to the present invention can be used as a spray by using propane, butane, trichloromonofluoromethane, dichlorodifluoromethane, dichlorotetrafluoroethane, carbonic acid gas, nitrogen gas, or a similar propellant. Examples of other forms include milk-like, cream-like, solid, semi-solid, paste-like, gel-like, powder-like, multi-layer, mousse-like, and similar forms. There various forms can be used as shampooing agents, rinsing agents, conditioning agents, setting lotions, hair sprays, permanent wave agents, mousses, hair colorants, and the like.

In addition, the type, form, and container of the cosmetic or external use preparation composition according to the present invention are the same as those disclosed in paragraphs 0230 to 0233 and the like of Patent Document 14.

The following other components generally used in external use preparations or cosmetic compositions may be added to the external use preparation or the cosmetic composition of the present invention, provided that such components do not inhibit the effectiveness of the present invention: water, powders or coloring agents, alcohols, water-soluble polymers, film-forming agents, oil agents, oil-soluble gelling agents, organo-modified clay minerals, surfactants, resins, UV absorbers, salts, moisturizing agents, preservatives, antimicrobial agents, perfumes, salts, antioxidants, pH adjusting agents, chelating agents, refreshing agents, anti-inflammatory agents, skin beautifying components (skin-lightening agents, cell activating agents, agents for ameliorating skin roughness, circulation promoters, astringents, antiseborrheic agents, and the like), vitamins, amino acids, nucleic acids, hormones, clathrates, and the like; bioactive substances, medicament active ingredients, and perfumes. However, the additives are not particularly limited to thereto.

The water that can be used in the cosmetic or external use preparation of the present invention needs to be clean and free of components that are harmful to the human body, and examples thereof include tap water, purified water, mineral water, and deep sea water.

(Oil agent)

The oil agent that can be used in the cosmetic or external use preparation according to the present invention is preferably one or more oil agents selected from silicone oils, non-polar organic compounds, and lowly polar to highly polar organic compounds that are liquid at 5 to 100°C, and the non-polar organic compounds and lowly polar to highly polar organic compounds are preferably hydrocarbon oils, fatty acid ester oils, and liquid fatty acid triglycerides. These are components that are particularly widely used as base materials for cosmetic compositions, but it is possible to additionally use one or more types of compound selected from among publicly known vegetable oils and fats, animal oils and fats, higher alcohols, fatty acid triglycerides, artificial sebum and fluorine-based oils as well as these oil agents.

By combining the hydrocarbon oil and/or the fatty acid ester oil with the silicone oil, in addition to the dry tactile sensation unique to silicone oils, moisture will be retained on the skin and a moisturizing feel whereby the skin or hair feels moisturized (also referred to as a luxurious tactile sensation) and smooth tactile sensation can be imparted to the cosmetic composition of the present invention. Moreover, there is a benefit in that stability over time of the cosmetic composition will not be negatively affected. Furthermore, with a cosmetic composition comprising the hydrocarbon oil and/or the fatty acid ester oil and the silicone oil, these moisturizing components (the hydrocarbon oil and/or the fatty acid ester oil) can be applied on the skin or hair in a more stable and uniform manner. Therefore, the moisturizing effects of the moisturizing components on the skin are improved. Thus, compared to a cosmetic composition comprising only a non silicone-based oil agent (e.g. a hydrocarbon oil, a fatty acid ester oil, or the like), the cosmetic composition comprising a non silicone-based oil agent along with a silicone oil is advantageous in that a smoother, more luxurious tactile sensation is imparted.

These oil agents are the same as those disclosed in paragraphs 0130 to 0135, paragraph 0206, and the like of Patent Document 14. Examples of the fluorine-based oil include perfluoropolyether, perfluorodecalin, perfluorooctane, and the like.

(Powder or coloring agent)

A powder or coloring agent which can be used in the cosmetic or external use preparation according to the

present invention is one that is commonly used as a component of a cosmetic composition, and includes white or colored pigments and extender pigments. The white and colored pigments are used to impart color and the like to the cosmetic composition, and the extender pigments are used to improve the tactile sensation and the like of the cosmetic composition. In the present invention, white and colored pigments as well as extender pigments commonly used in cosmetic compositions can be used as the powder without any particular restriction. In the present invention, preferably, one or two or more of the powders are compounded. The form (sphere, bar, needle, plate, amorphous, spindle, cocoon, or the like), particle size (aerosol, micro-particle, pigment-grade particle, or the like), and particle structure (porous, nonporous, or the like) of the powder are not limited in any way, but an average primary particle size is preferably in a range of 1 nm to 100 μm. Particularly, when compounding the powder or coloring agent as a pigment, preferably one or two or more selected from an inorganic pigment powder, an organic pigment powder, and a resin powder having an average diameter in a range from 1 nm to 20 μm is compounded.

Examples of the powder include inorganic powders, organic powders, surfactant metal salt powders (metallic soaps), colored pigments, pearl pigments, metal powder pigments, and the like. Compounded products of these pigments can be used. Furthermore, the surfaces of these pigments may be water-repellent treated.

Specific examples include the same powders or colorants disclosed in paragraphs 0150 to 0152 or the like of Patent Document 14.

Of the powders recited, description of a silicone elastomer powder shall be given. The silicone elastomer powder is a crosslinked product of a straight diorganopolysiloxane formed principally from diorganosiloxy units (D units), and can be preferably obtained by crosslinking an organohydrogenpolysiloxane having a silicon-bonded hydrogen atom on the sidechain or the molecular terminal and a diorganopolysiloxane having an unsaturated hydrocarbon group such as an alkenyl group or the like on the sidechain or the molecular terminal, in the presence of a hydrosilylation reaction catalyst. Compared to a silicone resin powder formed from T units and Q units, the silicone elastomer powder is soft, has elasticity, and has superior oil absorbency. Therefore, oils and fats on the skin can be absorbed and makeup smearing can be prevented. When surface treatment is performed on the high-purity glycerin derivative-modified silicone obtained by the manufacturing method of the present invention, uniform treatment can be performed with good treatment efficiency, so it is possible to provide a unique effect or feel corresponding to the type of the high-purity glycerin derivative-modified silicone without diminishing the suede-like feel of the silicone elastomer powder. Furthermore, when the high-purity glycerin derivative-modified silicone is blended into a cosmetic together with a silicone elastomer powder, the dispersion stability of the powder in the overall cosmetic composition is improved, and it is possible to obtain a cosmetic that is stable over time.

The silicone elastomer powder can be in various forms such as spherical, flat, amorphous, or the like. The silicone elastomer powder may also be in the form of an oil dispersion. With the cosmetic composition of the present invention, the silicone elastomer powder is particulate in form, and the primary particle size observed using an electron microscope and/or the average primary particle size measured by laser analysis or scattering is in a range from 0.1 to 50 μm. Additionally, a silicone elastomer powder having spherical primary particles can be preferably compounded. The silicone elastomer that constitutes the silicone elastomer powder is preferably one having a hardness, as measured using a type A durometer in the "Rubber, Vulcanized or Thermoplastic - Determination of Hardness" specified in JIS K 6253, of 80 or lower, and more preferably 65 or lower.

Of these silicone elastomer powders, specific examples of silicone elastomer spherical powders, in particular, are the same as those disclosed in paragraph 0168 of Patent Document 14 and may be silicone elastomer powders that have been subjected to various surface treatments such as water-repellent treatment, as disclosed in paragraphs 0150 to 0152.

It is possible to further blend another surfactant in the cosmetic or external use preparation of the present invention. These other surfactants are components that function as cleansing components of the skin or the hair or, alternatively, as the oil agent or an emulsifier, and can be selected as desired depending on the type and function of the cosmetic composition. More specifically, the other surfactants can be selected from the group consisting of an anionic surfactant, a cationic surfactant, a nonionic surfactant, an amphoteric surfactant, and a semipolar surfactant. Preferably a silicone-based nonionic surfactant is used in combination.

These surfactants are the same as those disclosed in paragraphs 0162, 0163, 0195 to 0201, and the like of Patent Document 14. The high-purity glycerin derivative-modified silicone obtained by the manufacturing method of the present invention functions as a dispersant since it has polar groups and non-polar groups in the molecule. Therefore, when used in combination with a non-ionic surfactant, the diglycerin derivative-modified silicone functions as an aid to enhance the performance of the non-ionic surfactant, and may improve the overall stability of the formulation. In particular, the high-purity glycerin derivative-modified silicone obtained by the manufacturing method of the present invention or a solution containing the high-purity glycerin derivative-modified silicone can be used in combination with a polyoxyalkylene-modified silicone, a polyglyceryl-modified silicone, a glyceryl-modified silicone, a sugar-modified silicone, and a sugar alcohol-modified silicone due to its enhanced compatibility and affinity with various modified silicones. Moreover, nonionic surfactants of these silicones in which an alkyl branch, a straight-chain silicone branch, a siloxane dendrimer branch,

or the like is provided as necessary along with the hydrophilic group can also be advantageously used.

Depending on the intended use thereof, the cosmetic or external use preparation of the present invention can contain one or two or more polyhydric alcohols and/or lower monohydric alcohols. These alcohols are the same as those disclosed in paragraphs 0159, 0160, and the like of Patent Document 14.

Depending on the purpose thereof, the cosmetic or the external use preparation of the present invention can contain one or two or more inorganic salts and/or organic salts. These salts are the same as those disclosed in paragraph 0161 and the like of Patent Document 14.

Depending on the purpose thereof, the cosmetic or the external use preparation of the present invention can contain at least one selected from the group consisting of a crosslinking organopolysiloxane, an organopolysiloxane elastomer spherical powder, a silicone resin, an acryl silicone dendrimer copolymer, a silicone raw rubber, a polyamide-modified silicone, an alkyl-modified silicone wax, and an alkyl-modified silicone resin wax. These silicone-based components are the same as those disclosed in paragraphs 0162 to 0194 and the like of Patent Document 14.

Depending on the intended use thereof, the cosmetic or external use preparation of the present invention can contain one or two or more water-soluble polymers. These water-soluble polymers are the same as those disclosed in paragraph 0201 and the like of Patent Document 14.

Depending on the intended use thereof, the cosmetic or external use preparation of the present invention can contain one or two or more ultraviolet light blocking components. These ultraviolet light blocking components are the same as the organic and inorganic ultraviolet light blocking components disclosed in paragraphs 0202 to 0204 and the like of Patent Document 14, but specifically, an ultraviolet light blocking component that can be suitably used is at least one selected from the group consisting of microparticulate titanium oxide, microparticulate zinc oxide, 2-ethylhexyl p-methoxycinnamate, 4-tert-butyl-4'-methoxydibenzoylmethane, diethylamino hydroxybenzoyl hexyl benzoate, benzotriazole-based UV absorbers, and triazine-based UV absorbers such as 2,4,6-tris[4-(2-ethylhexyloxycarbonyl)anilino]1,3,5-triazine (INCI: octyl triazone) and 2,4-bis([4-(2-ethyl-hexyloxy)-2-hydroxy]phenyl)-6-(4-methoxyphenyl)-1,3,5-triazine (INCI: bis-ethylhexyloxyphenol methoxyphenyl triazine, trade designation: Tinosorb®S). These ultraviolet light blocking components are generally used, are easy to acquire, and have high ultraviolet light blocking effects and, thus can be beneficially used. In particular, using both inorganic and organic ultraviolet light blocking components is preferable, and using a UV-A blocking component in combination with a UV-B blocking component is more preferable.

By using an ultraviolet light blocking component in combination with the high-purity glycerin derivative-modified silicone in the cosmetic or the external use preparation of the present invention, it is possible to stably and finely disperse the ultraviolet light blocking component in the cosmetic composition while improving the feeling to touch and storage stability of the overall cosmetic composition, and it is therefore possible to impart the cosmetic composition with excellent ultraviolet light blocking properties.

Various components other than the components described above can be used in the cosmetic composition or external use preparation of the present invention, provided that such use does not impair the effects of the present invention. Examples thereof include oil-soluble gelling agents, organo-modified clay minerals, preservatives, bioactive components, skin beautifying components, pH adjusting agents, antioxidants, solvents, chelating agents, moisturizing components, perfumes and the like. These optional components for a cosmetic product are the same as those disclosed in paragraphs 0207, 0208, 0220 to 0228, and the like of Patent Document 14.

Additionally, in cases where the external use preparation or the cosmetic composition according to the present invention is an anti-perspirant, or depending on the purpose thereof, the external use preparation or the cosmetic composition can contain an anti-perspiration active component and/or a deodorant agent. These anti-perspiration components and deodorant components are the same as those disclosed in paragraphs 0209 to 0219 and the like of Patent Document 14. Similarly, in cases in which the cosmetic or external use preparation according to the present invention is an anti-perspirant composition, the preparation, method of use, and the like of the various anti-perspirant compositions are the same as those disclosed in paragraphs 0234 to 0275 and the like of Patent Document 14.

## Industrial Applicability

The manufacturing method for a high-purity glycerin derivative-modified silicone according to the present invention can be applied regardless of the type of the organic modifier, is inexpensive and simple, has excellent yield or productivity, and can reasonably accommodate production on a commercial scale. In addition, the high-purity glycerin derivative-modified silicone obtained by the manufacturing method of the present invention substantially consists of a single component from which impurities originating from the organic modifier have been removed, so phase separation, precipitation of the unreacted starting material, or the like does not occur after production. In particular, the high-purity glycerin derivative-modified silicone obtained by the manufacturing method of the present invention maintains an appearance with high transparency, regardless of the temperature environment in which it is used, so even when the compound is used or various industrial materials such as oil agents into which the compound is blended are used in cold regions, problems such as decreases in performance or fluctuations in quality due to poor compatibility between

the main component and the impurities do not occur, and the production process can thus be stabilized. Conversely, even when the compound is used or various industrial materials such as oil agents into which the compound is blended are used in hot seasons or regions, problems such as decreases in performance or fluctuations in quality due to poor compatibility between the main component and the impurities do not occur, and the compound is unlikely to be affected by degradation due to oxidation or the like, so it is possible to stabilize the production process as well as to improve the quality level of the final product. Therefore, the present invention solves the basic problems of organo-modified silicones and organomodified silanes which are difficult to prepare with high purity using conventional methods.

Specifically, the high-purity glycerin derivative-modified silicone obtained by the manufacturing method of the present invention can be suitably used not only as a starting material for external use preparations, medicaments, or cosmetics, but also, for example, as a fiber treating agent, a varnish or paint additive with excellent heat resistance, weather resistance, and electrical characteristics, a coating agent, a primer, a tackifier, a polyol main agent, a foam stabilizer, or a modifier for various urethanes or foaming materials, a mold-releasing agent or peeling agent, an antifoam agent, greases or oil compounds, oils for insulation, burnishing, water repellency, heating/cooling mediums, lubrication, or the like, a modifier, additive, or surface treating agent for a rubber or resin, a starting material for a silicone-modified organic resin, a compounding agent, modifier, or precursor for a silane coupling agent, a coating material or sealing material for a building/lining, a protecting agent or lubricant for optical fibers/electrical lines, and starting materials for general industrial materials such as electronic/electrical parts. Examples

The present invention will be described in detail hereinafter using working examples and comparative examples, but the present invention is not limited to the working examples described below. In addition, the light transmittance of each sample that was obtained was measured at room temperature (25°C) with the method described below.

[Light transmittance] The light transmittance (%) at a wavelength of 750 nm and a cell thickness of 10 mm was measured using a light transmittance meter [manufactured by the Shimadzu Corporation, UV-265FW]. Purified water was used as a control.

Note that in the production examples and comparative examples below, the language "production of glycerin derivative-modified silicone No. X" is used for the sake of convenience, but the obtained products are in the form of mixtures containing a small amount of unreacted starting material and the like in addition to the main components.

In the following compositional formulae, "Me" represents a methyl ($-CH_3$) group, "M" represents a $Me_3SiO$ group (or an $Me_3Si$ group), "D" represents an $Me_2SiO$ group, "D"" represents an MeHSiO group, and "$M^R$" and "$D^R$" respectively represent units in which a methyl group in "M" or "D" is modified by any substituent. Additionally, in the production examples, "IPA" represents isopropyl alcohol.

[Production Example 1] <Production of glycerin derivative-modified silicone No. 1>

Step 1: First, 215 g of a methylhydrogenpolysiloxane expressed by the average composition formula $MD_{47.5}D^H_{10.5}M$, 16.9 g of a vinyl tris (trimethylsiloxy)silane expressed by the average composition formula $CH_2$=CH-Si$(OSiMe_3)_3$, and 0.39 g of an IPA solution of a platinum-1,3-divinyl-1,1,3,3-tetramethyldisiloxne complex (Pt concentration: 0.45 wt.%) were charged into a reaction vessel, and heating was started while stirring under a nitrogen stream. After a reaction was performed for 2 hours at 30 to 50°C, the reaction liquid was collected, and when confirmed by an alkali decomposition gas generation method (the remaining Si-H groups are decomposed using a KOH ethanol/water solution, and the reaction rate is calculated from the volume of the produced hydrogen gas), the reaction rate was as planned.

Step 2: The reaction liquid was set to 39°C, and when 47.2 g of hexadecene ($\alpha$-olefin purity = 91.7%) (first time) was added, an increase in temperature to 68°C was observed. When the reaction liquid was collected and confirmed with the same method as in step 1 at the point when the liquid temperature reached 64°C after a reaction for one hour, the reaction was as planned.

Step 3: First, 23.7 g of a diglycerin monoallyl ether expressed by the composition formula $CH_2$=CH-$CH_2$-O$(CH_2CH(OH)CH_2O)_2$-H, 0.035 g of natural vitamin E, and 245 g of IPA were added to the reaction liquid, and 0.38 g of the same platinum catalyst solution as described above was additionally added. A reaction was performed for one hour at 45 to 65°C, and when confirmed with the same method as in step 1, the reaction rate was as planned. Here, the charged amount of the diglycerin monoallyl ether was over 1.10 times the molar amount of the Si-H groups to be reacted (for $D^H_2$ units). Therefore, the excess glycerin derivative remains in the reaction liquid.

Step 4: First, 47.2 g of hexadecene ($\alpha$-olefin purity = 91.7%) (second time) was added to the reaction liquid, and 0.2 g of the same platinum catalyst solution as described above was additionally added. A reaction was performed for three hours at 60 to 70°C, and when confirmed with the same method as in step 1, the reaction was complete.

Step 5: The reaction liquid was heated under reduced pressure and maintained for one hour under conditions at 95 to 105°C and 10 mmHg while hovering in nitrogen gas so as to remove low-boiling-point matter such as IPA. When pressure was then restored after cooling to 75°C or lower, the content was a yellowish brown, uniform liquid with a transparent feel.

Step 6: An aqueous solution prepared by dissolving 0.055 g of a sodium hydrogen sulfate monohydrate in 5.3 g of ion exchanged water was charged into the content of the reaction vessel, and acid treatment was performed for one hour at 60 to 70°C while stirring under a nitrogen stream. The pressure was then reduced at 66°C, and the pressure was restored when the distillation of water and other low-boiling-point matter stopped (first cycle of acid treatment). Next, 5.3 g of water was added, and after treatment was performed for 10 minutes, the pressure was similarly reduced. The pressure was then restored when the distillation of water and other low-boiling-point matter stopped (second cycle of acid treatment). Next, 5.3 g of water was once again added, and after treatment was performed for 15 minutes, the pressure was reduced and the heated-depressurized state was maintained for 2 hours at 55 to 70°C. The pressure was restored after the water droplets in the system disappeared (third cycle of acid treatment). As a result, 347 g of a composition containing a diglycerin derivative-modified silicone expressed by the average composition formula $MD_{47.55}D^{R*11}{}_{7.5}D^{R*31}{}_{1}D^{R*21}{}_{2}M$ was obtained as a grayish brown, opaque, uniform liquid. Viscosity (25°C): 8,400 mPa. s

Here, $R^{*11}$, $R^{*21}$, and $R^{*31}$ are as described below.

$R^{*11} = -C_{16}H_{33}$
$R^{*21} = -C_3H_6O\{CH_2CH(OH)CH_2O\}_2-H$
$R^{*31} = -C_2H_4Si(OSiMe_3)_3$

The turbidity of the appearance of the content increased dramatically due to acid treatment, but this is considered to be due to the result of a greater increase in polarity and a decrease in the compatibility with the modified silicone serving as the main component as the unreacted unsaturated diglycerin (triol) was hydrolyzed and transformed into a corresponding diglycerin (tetraol).

[Comparative Example 1]

<Preparation of comparative composition RE-1 containing glycerin derivative-modified silicone No. 1>

First, 340 g of the grayish brown, opaque, uniform liquid obtained in Production Example 1 was filtered with a pressure filter at room temperature and an $N_2$ pressure of 400 kPa using 10 g of Hiflo Super Cell (Celite Corporation, flux calcined diatomaceous earth) as a filter aid and using ADVANTEC No. 424 (diameter: 110 mm, Toyo Roshi Co., Ltd.) as filter paper. However, the turbidity did not improve whatsoever from the start to the end of filtration, and 322 g of a grayish brown, opaque, cloudy liquid was obtained over the course of one hour. (Viscosity (25°C): 8,400 mPa. s) The transparency of the appearance of this composition was not improved whatsoever in comparison to the composition obtained in Production Example 1.

[Comparative Example 2]

<Preparation of comparative composition RE-2 containing glycerin derivative-modified silicone No. 2>

Next, 315 g of the grayish brown, opaque, uniform liquid obtained in Comparative Example 1 was extracted and pressure-filtered at an $N_2$ pressure of 150 kPa using a specialized filter with a Zeta Plus Filter 30C (diameter: 90 mm, 3M Corporation, zeta-potential adsorption filter). The filter has a low pressure resistance by nature and cannot withstand a pressure exceeding 200 kPa, so it was necessary to perform filtration in a lower pressurized state than in Comparative Example 1. At this time, filtration was very slow at room temperature, so filtration was performed while maintaining a temperature of from 40 to 50°C. The first approximately 40 g of the filtrate had an improved appearance with a transparent feel, but turbidity appeared thereafter. Therefore, the composition was mixed so that the entire amount of the filtrate that was ultimately obtained was uniform, and as a result, 283 g of a grayish brown, opaque, uniform liquid was obtained over the course of seven hours. (Viscosity (25°C): 8,400 mPa. s) Practically no improvement in the transparency of the appearance of this composition was observed in comparison to the composition obtained in Comparative Example 1.

[Working Example 1]

<Preparation of high-purity glycerin derivative-modified silicone No. 1>

First, 120 g of the grayish brown, opaque, uniform liquid obtained in Comparative Example 2 and 3.6 g of a flaked product (organic wax) of PEG#20000 (polyethylene oxide with a molecular weight of 20,000, melting point: approximately 65°C) were charged into a flask, and heating was started while stirring under a nitrogen stream. When mixing and stirring were performed aggressively for 40 minutes at 80°C, the appearance was a cloudy white, uniform dispersion.

The composition was then left to cool (two hours) while stirring until the temperature reached 40°C or lower, and treatment was ended. The appearance of the flask content was the same as before being left to cool. Next, the flask content was filtered with a pressure filter over the course of one hour at room temperature and an $N_2$ pressure of 400 kPa using 10 g of Hiflo Super Cell (Celite Corporation, flux calcined diatomaceous earth) as a filter aid and using ADVANTEC No. 424 (diameter: 110 mm, Toyo Roshi Co., Ltd.) as filter paper. As a result, a translucent, uniform, light yellowish brown filtrate was surprisingly obtained from start to finish, and the total amount was 92 g. That is, with the technique of the present invention described in Working Example 1, it was possible to remove most of the turbidity from the opaque reaction mixture of Comparative Example 2 and to achieve translucence of the entire amount. Viscosity (25°C): 8,500 mPa. s

[Production Example 2]

<Production of glycerin derivative-modified silicone No. 2>

Step 1: First, 147.5 g of a methylhydrogenpolysiloxane expressed by the average composition formula $MD_{43.4}D^{H}_{7.4}M$, 28.8 g of hexadecene (α-olefin purity = 91.7%), and 5.2 g of a 3-methacryloxypropyl(tris(trimethylsiloxy)silylethyldimethylsiloxy)silane expressed by the following average composition formula:

[Formula 54]

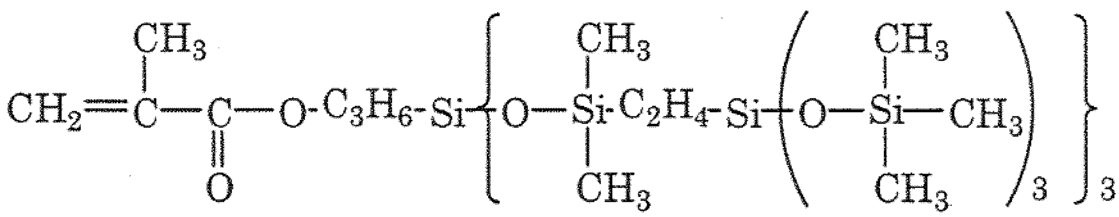

were charged into a reaction vessel, and heating was started by charging 0.10 g of a hexamethyldisiloxane solution of a platinum-1,3-divinyl-1,1,3,3-tetramethyldisiloxane complex (Pt concentration: 0.45 wt.%) while stirring under a nitrogen stream. After a reaction was performed for two hours at 50 to 60°C, the reaction liquid was recovered, and when confirmed by an alkali decomposition gas generation method, the reaction rate was as planned.

Step 2: First, 14.8 g of a diglycerin monoallyl ether expressed by the average composition formula $CH_2=CH-CH_2-O(C_3H_6O_2)_2-H$, 0.03 g of natural vitamin E, and 120 g of IPA were added to the reaction liquid, and 0.15 g of the same platinum catalyst solution as described above was additionally added. A reaction was performed for 5.5 hours at 50 to 60°C, and when confirmed with the same method as in step 1, the reaction rate was as planned, and it was clear that a modified silicone intermediate expressed by the average composition formula $MD_{43.4}D^{R*32}_{0.1}DR^{R*22}_{1.56}D^{R*11}_{3.1}D^{H}_{2.64}M$ was produced. Here, the charged amount of the diglycerin monoallyl ether was over 1.13 times the molar amount of the Si-H groups to be reacted (for $D^{H}_{1.6}$ units). Therefore, the excess glycerin derivative remains in the reaction liquid.

Here, $R^{*11}$, $R^{*22}$, and $R^{*32}$ are as described below.

$R^{*11} = -C_{16}H_{33}$

$R^{*32} =$

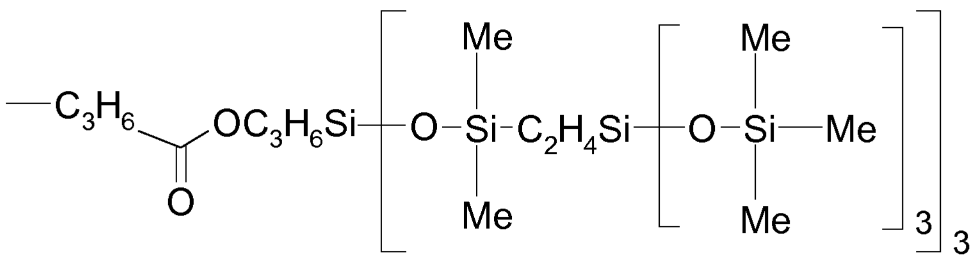

[Formula 55]

Here, the diglycerin monoallyl ether used in Production Example 2 was synthesized by performing a ring-opening addition reaction with one molar equivalent of glycidol with respect to one mol of the glycerin monoallyl ether. The glycerin monoallyl ether has two hydroxyl groups, and glycidol can react with both, so the diglycerin portion here is

not a simple composition with only a chain structure.

$R^{*22}$= $-C_3H_6O-X$ {$X=(C_3H_6O_2)_2-H$ (diglycerin portion)

Step 3: First, 200 g of FZ-3196 (manufactured by the Toray-Dow-Corning Corporation, caprylyl methicone), which is a reaction solvent and diluent at the time of a crosslinking reaction, was charged into the reaction liquid, and the IPA was removed under reduced pressure at 45 to 50°C. The pressure was then restored, and after 5.51 g of 1,5-hexadiene was added at 47°C, 0.25 g of the same platinum catalyst solution as described above was additionally added. The C = C/Si-H molar ratio at the time of this crosslinking reaction was 1.20. When confirmed with the same method as in step 1 after a reaction was performed for eight hours at 50°C, the reaction was complete, and a thick, cloudy white liquid was obtained.

Step 4: First, 6.0 g of a 0.16% phosphoric acid aqueous solution, 80 g of IPA, and 3.0 g of ion exchanged water were charged into the content of the reaction vessel, and acid treatment was performed for three hours at 70 to 80°C while stirring under a nitrogen stream. The pressure was then reduced, and after the composition was held for 1.5 hours under conditions at 70 to 90°C and 10 mmHg or lower from the point when the distillation of the low-boiling-point matter stopped, the pressure was restored. As a result, 395 g of a composition (reaction mixture) containing a liquid organo-modified silicone as a main component and containing the same amount of caprylyl methicone (oil agent for dilution) as a second main component, the composition having a glycerin derivative group and a crosslinking portion, wherein the crosslinking portion links the organopolysiloxane part and the organic part by means of an Si-C bond. This product was a grayish brown liquid with substantial turbidity at 25°C. Viscosity (25°C): 1,400 mPa. s

The average structural formula (schematic illustration) of the liquid organo-modified silicone obtained in Production Example 2 is illustrated below.

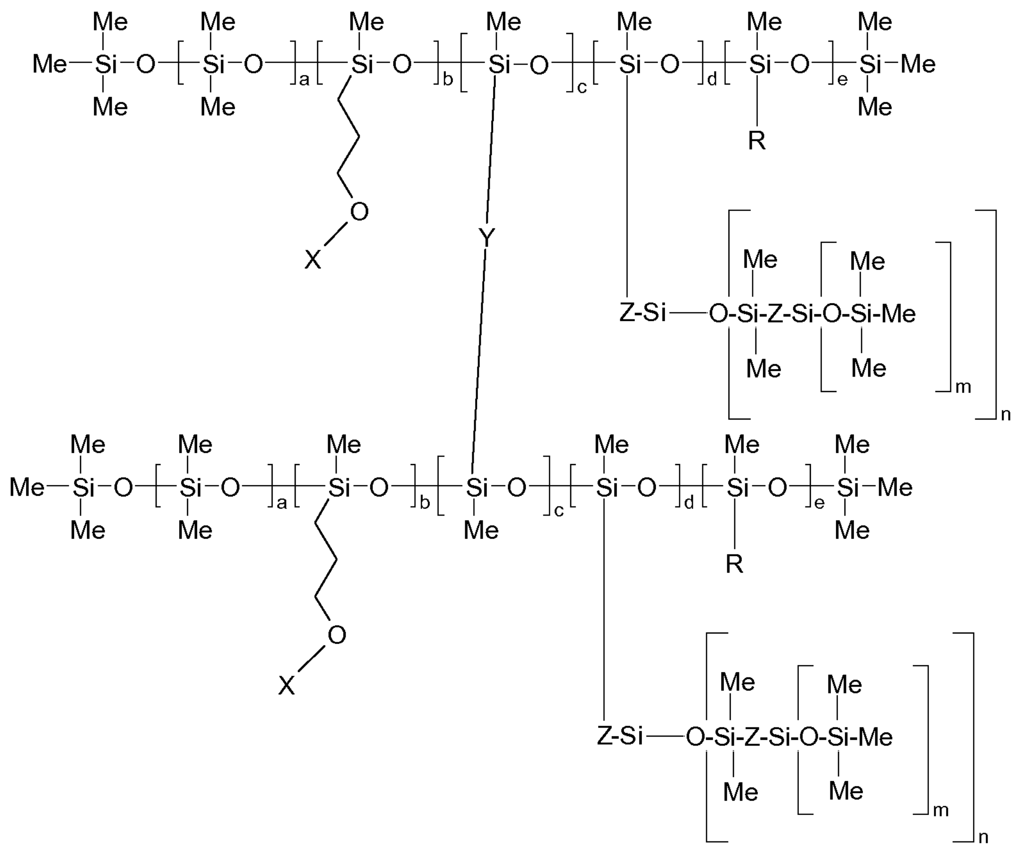

[Formula 56]

(In the formula, Me = methyl group, $Z=-CH_2CH_2-$ inside []n, $Z=-C_3H_6-COO-C_3H_6-$ outside []n, $R=-C_{16}H_{33}$, $Y=-(CH_2)_6-$, a=43.4, b=1.56, c=2.64, d=0.1, e=3.1, m=3, and n=3), $X=(C_3H_6O_2)_2H$

Comparative Example 3

<Preparation of comparative composition RE-3 containing glycerin derivative-modified silicone No. 2>

The grayish brown liquid with substantial turbidity obtained in Production Example 2 (reaction mixture containing

glycerin derivative-modified silicone No. 2 and caprylyl methicone as main components) was used directly as a sample.

Working Example 2

<Preparation of high-purity glycerin derivative-modified silicone No. 2>

First, 279 g of the light brown viscous liquid with substantial turbidity obtained in Production Example 2 and 8.4 g of a flaked product (organic wax) of PEG#20000 (polyethylene oxide with a molecular weight of 20,000, melting point: approximately 65°C) were charged into a flask, and heating was started while stirring under a nitrogen stream. When mixing and stirring were performed aggressively for one hour at 85 to 100°C, the appearance was a cloudy white, uniform dispersion. The composition was then left to cool (2.5 hours) while stirring until the temperature reached 40°C or lower, and treatment was ended. The appearance of the flask content was similar to before cooling. Next, the flask content was filtered with a pressure filter over the course of one hour at room temperature and an $N_2$ pressure of 600 kPa using 10 g of Hiflo Super Cell (Celite Corporation, flux calcined diatomaceous earth) as a filter aid and using ADVANTEC No. 424 (diameter: 110 mm, Toyo Roshi Co., Ltd.) as filter paper. As a result, a translucent, uniform, light yellow filtrate was surprisingly obtained from start to finish, and the total amount was 253 g. (Viscosity (25°C): 1,500 mPa. s) That is, with the technique of the present invention described in Working Example 2, it was possible to remove most of the turbidity from the opaque reaction mixture of Production Example 2 and to achieve translucence of the entire amount.

The contents of "high-purity glycerin derivative-modified silicone No. 1 and the high-purity glycerin derivative-modified silicone No. 2"of the working examples, which are the high-purity glycerin derivative-modified silicones of the present invention, and "comparative compositions RE-1 and RE-2 containing glycerin derivative-modified silicone No. 1 and comparative composition RE-3 containing glycerin derivative-modified silicone No. 2" of the comparative examples prepared with the methods described above are shown in the following Tables 1 and 2.

Table 2

Table 1

| Sample | Appearance | Diluent (concentration) | Chemical structure of the main component*1) |
|---|---|---|---|
| Working Example 1 | Light yellowish brown, translucent, uniform liquid | None | $MD_{47.5}R^{R*11}{}_{7.5}D^{R*31}{}_{1}D^{R*21}{}_{2}M$ |
| Comparative Example 1 | Grayish brown, opaque, uniform liquid | None | |
| Comparative Example 2 | Grayish brown, opaque, uniform liquid | None | |
| Working Example 2 | Light yellow, translucent, uniform liquid | FZ-3196 (50%) | $MD_{43.4}D^{R*32}{}_{0.1}D^{R*22}{}_{1.56}D^{R*11}{}_{3.1}D^{H}{}_{2.64}M$ Crosslinking reaction product with $CH_2=CH(CH_2)_2CH=CH_2$ |
| Comparative Example 3 | Grayish brown liquid with substantial turbidity | FZ-3196 (50%) | |
| Note*1) The chemical structure of the glycerin derivative-modified silicone serving as the main component is expressed by an average composition formula. | | | |

In the table, the structures and types of the functional groups are as follows.

< Group having a siloxane dendron structure: $R^{*3}$>

$$R^{*31} = -C_2H_4Si(OSiMe_3)_3$$

$R^{*32}$ =

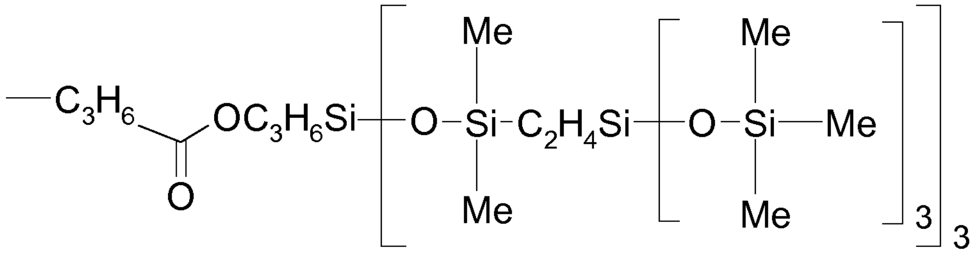

[Formula 57]

<Glycerin derivative group-containing organic group>

$R^{*21}$ =-$C_3H_6O\{CH_2CH(OH)CH_2O\}_2$-H

$R^{*22}$= -$C_3H_6O$-X {X=$(C_3H_6O_2)_2$-H;

in this example, the diglycerin portion is not a simple composition with only a chain structure.)

<Other organic groups: $R^{*1}$>

$R^{*11}$ = -$C_{16}H_{33}$

Table 3

Table 2

| | Light transmittance *2) | Viscosity *3) | Effect of purity increasing treatment |
|---|---|---|---|
| Working Example 1 | 52 | 8500 | ○ |
| Comparative Example 1 | 0.3 | 8400 | × |
| Comparative Example 2 | 0.8 | 8400 | × |
| Working Example 2 | 75 | 1500 | ○ |
| Comparative Example 3 | 0.2 | 1400 | × |

Note *2) Expresses the light transmittance T% of the sample at room temperature (wavelength: 750 nm, cell thickness: 10 mm).
Note *3) Value of the viscosity (mPa. s) of the sample at 25°C; expressed as a numerical value measured with an E-type rotary viscometer.

[Stability tests] First, 40 g of each of the samples of Working Examples 1 and 2 and Comparative Examples 2 and 3 was placed in a 100 ml glass vial and stopped tightly. These were left to stand for four months at room temperature. After the sample appearance was then recorded, the light transmittance and viscosity were measured. The results are shown in Table 3.

Table 4

Table 3

| | Appearance | Light transmittance *4) | Viscosity (25°C) (Rate of change%) *5) | Odor *6) |
|---|---|---|---|---|
| Working Example 1 | Light yellowish brown, translucent, uniform liquid (No change) | 48 | +1.1 | ○~◎ |

(continued)

| | Appearance | Light transmittance *4) | Viscosity (25°C) (Rate of change%) *5) | Odor *6) |
|---|---|---|---|---|
| Comparative Example 2 | Grayish brown, opaque liquid (slightly non-uniform feel) | 1.4 | -4.5 | × |
| Working Example 2 | Light yellow, translucent, uniform liquid (No change) | 73 | +0.3 | ○~◎ |
| Comparative Example 3 | Three-phase separation into a translucent liquid, an opaque, turbid liquid, and a grayish brown precipitate | - (separation) | - (separation) | Δ |

Note*4) Expresses the light transmittance T% of the sample at room temperature after the stability test (wavelength: 750 nm, cell thickness: 10 mm).
Note*5) Expresses the rate of change% in viscosity from the initial value.
Note*6) The degree of odor when the sample was unsealed was evaluated by sense of smell in accordance with the following criteria after the stability test.

Odor test evaluation standards:

◎: no odor is detected whatsoever.
○: there is no aldehyde odor whatsoever, but a slight substrate odor is detected.
Δ: a slight aldehyde odor is observed.
×: an unpleasant aldehyde odor which bothers the nose is clearly detected.

It can be seen from the above results that the samples of the working examples are far superior to the samples of the comparative examples from the perspectives of high purity and odorlessness.

Hereinafter, formulation examples of the cosmetic composition and the external use preparation according to the present invention are described, but the cosmetic composition and the external use preparation according to the present invention are not limited to the types and compositions recited in these formulation examples.

The high-purity glycerin derivative-modified silicone obtained by the present invention can be used in various external use preparations and cosmetics, for example. A specific formulation example thereof is one in which components corresponding to silicone compound Nos. 1 to 16 in Formulation Examples 1 to 43 of various cosmetics and external use preparations described in Patent Document 14 (WO2011/049248) and/or various polyether-modified silicones are substituted with the high-purity glycerin derivative-modified silicones of the present invention (high-purity glycerin derivative-modified silicone Nos. 1, 2, and the like) or solutions thereof.

Another formulation example is one in which components corresponding to silicone compound Nos. 1 to 14 in Formulation Examples 24 of various cosmetics and external use preparations disclosed in Patent Document 15 (WO2011/049247) and/or various polyether-modified silicones are substituted with the high-purity glycerin derivative-modified silicones of the present invention (high-purity glycerin derivative-modified silicone Nos. 1, 2, and the like) or solutions thereof.

Yet another formulation example is one in which components corresponding to the AB-type organopolysiloxane copolymers P1 to P6 contained in Formulation Examples 1 to 10 of various cosmetics and external use preparations disclosed in Patent Document 16 (WO2011/049246) (Synthesis Examples 1 to 12) and/or various oxyethylene-modified silicones are substituted with the high-purity glycerin derivative-modified silicones of the present invention (high-purity glycerin derivative-modified silicone Nos. 1, 2, and the like) or solutions thereof.

In addition, another formulation example is one in which components corresponding to silicone compound Nos. 1 to 8 contained in Formulation Examples 1 to 14 of various cosmetics and external use preparations disclosed in Patent Document 17 (Japanese Unexamined Patent Application Publication No. 2012-046507A) and/or various polyether-modified silicones are substituted with the high-purity glycerin derivative-modified silicones of the present invention (high-purity glycerin derivative-modified silicone Nos. 1, 2, and the like) or solutions thereof.

The high-purity glycerin derivative-modified silicone of the present invention has the advantage that, since an organic modifier with a polarity substantially differing from that of the silicone is removed, problems related to poor compatibility at the time of the addition of various starting materials are unlikely to occur when designing a formulation for a cosmetic or external use preparation, so the scope of formulation design widens. At the same time, it is also possible to reduce the risk or concerns related to the stability of the final product. Since the composition has high purity, it is

advantageous from the perspectives of the tactile feel improving effect, moisturizing effect, minimal degradation phenomena such as odorization over time, surface active effect, emulsification performance, powder dispersion stability, powder surface treatment effect, or the duration of these effects in comparison to typical organosilicon compounds with large impurity content. In particular, in formulations containing a powder or formulations with a small water compounding ratio, the characteristics of the high-purity glycerin derivative-modified silicone obtained by the manufacturing method of the present invention make it possible to finely disperse medicinal ingredients or powders into a cosmetic or external use preparation more stably than with conventional methods. As a result, application irregularities are eliminated, which yields the substantial advantage that the original effects of the formulation such as improved cosmetic durability or coloration or improved skin care or UV filtering effect are enhanced. In addition, in a formulation not containing a powder, the characteristics of the high-purity glycerin derivative-modified silicone obtained by the manufacturing method of the present invention make it possible to easily obtain a stable product with excellent transparency, even if the composition has low viscosity.

## Claims

**1.** A manufacturing method for a liquid high-purity glycerin derivative-modified silicone, the method comprising:

a step of adding, to a mixture containing a glycerin derivative-modified silicone and impurities, an organic wax having affinity with the impurities and having a higher melting point than the glycerin derivative-modified silicone, melting and mixing while heating, and introducing the impurities into the melted organic wax;
a step of obtaining a solidified product of the organic wax by cooling the organic wax; and
a step of performing solid-liquid phase separation on the glycerin derivative-modified silicone and the solidified product of the organic wax.

**2.** The manufacturing method according to claim 1, wherein the impurities are impurities originating from the glycerin derivative.

**3.** The manufacturing method according to claim 1 or 2, wherein the glycerin derivative-modified silicone is a liquid at least at 100°C.

**4.** The manufacturing method according to any one of claims 1 to 3, wherein the organic wax has a melting point of from 45°C to 150°C.

**5.** The manufacturing method according to any one of claims 1 to 4, wherein the organic wax has an average molecular weight of at least 900.

**6.** The manufacturing method according to any one of claims 1 to 5, wherein the organic wax contains a (poly)oxyethylene site.

**7.** The manufacturing method according to any one of claims 1 to 6, wherein the organic wax is a glycerin derivative containing a (poly)oxyethylene site.

**8.** The manufacturing method according to any one of claims 1 to 7, wherein silicon atoms of the glycerin derivative-modified silicone bond with glycerin derivative group-containing organic groups via Si-C bonds or Si-O-C bonds.

**9.** The manufacturing method according to any one of claims 1 to 8, wherein the glycerin derivative-modified silicone is a glycerin derivative-modified silicone expressed by the following general formula (1):

[Formula 1] $R^1_aR^2_bL^1_cQ_dSiO_{(4-a-b-c-d)/2}$ (1)

(wherein $R^1$ represents a monovalent organic group (however, excluding $R^2$, L, and Q), a hydrogen atom or a hydroxyl group; and $R^2$ is a substituted or unsubstituted, straight or branched monovalent hydrocarbon group having 9 to 60 carbon atoms, or the chain organosiloxane group represented by the following general formula (2-1):

[Formula 2]

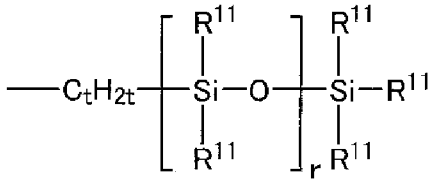

(2-1)

(wherein $R^{11}$ are each independently a substituted or unsubstituted monovalent hydrocarbon group having from 1 to 30 carbons, hydroxyl groups, or hydrogen atoms and at least one of the $R^{11}$ moieties is the monovalent hydrocarbon group;t is a number in a range of 2 to 10; and r is a number in a range of 1 to 500); or the general formula (2-2) below:

[Formula 3]

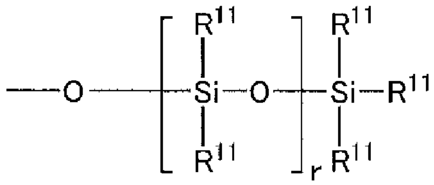

(2-2)

(wherein, $R^{11}$ and r are synonymous with those described above); and $L^1$ represents a silylalkyl group having a siloxane dendron structure expressed by the following general formula (3) when
i=1;

[Formula 4]

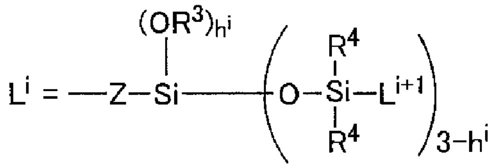

(3)

(wherein, $R^3$ each independently represents a substituted or unsubstituted, straight or branched monovalent hydrocarbon group having 1 to 30 carbon atoms; $R^4$ each independently represents an alkyl group or phenyl group having 1 to 6 carbon atoms; Z represents a divalent organic group; i represents a generation of the silylalkyl group represented by $L^i$ and is an integer of 1 to k when k is a number of generations that is a number of repetitions of the silylalkyl group; the number of generations k is an integer from 1 to 10; $L^{i+1}$ is the silylalkyl group when i is less than k, and $R^4$ when i=k, and $h^i$ is a number in a range from 0 to 3); Q represents a glycerin derivative group-containing organic group; and
a, b, c, and d are numbers within the respective ranges $1.0 \leq a \leq 2.5$, $0 \leq b \leq 1.5$, $0 \leq c \leq 1.5$, and $0.0001 \leq d \leq 1.5$)

**10.** The manufacturing method according to any one of claims 1 to 8, wherein the glycerin derivative-modified silicone is an organo-modified silicone obtained by reacting:

(A) an organohydrogenpolysiloxane;
(B) a glycerin derivative group-containing organic compound having one or more reactive unsaturated groups in each molecule; and
(C) one or more types of organic compounds selected from the group consisting of (C1) an organic compound having a number of reactive unsaturated groups greater than 1 on average in each molecule and (C2) an organic compound having one or more reactive unsaturated groups and one or more epoxy groups in each molecule (however, the use of the component (B) is optional when the component (C) contains a glycerin derivative group-containing organic group); the glycerin derivative-modified silicone having a silicon-bonded glycerin derivative group-containing organic group and having a crosslinked structure containing a Si-C bond in a crosslinking portion.

**11.** The manufacturing method according to any one of claims 1 to 8, wherein the glycerin derivative-modified silicone is a glycerin derivative-modified silicone in the form of a straight-chain glycerin derivative group-containing alternating copolymer obtained by reacting at least:

(D) an organopolysiloxane having reactive functional groups at both terminals of a molecular chain; and
(E) an organic compound having two reactive functional groups capable of reacting with the reactive functional groups positioned at both of the molecular chain terminals of the organopolysiloxane (D) in the molecule.

**12.** The manufacturing method according to any one of claims 1 to 11, wherein the mixture further contains a solvent of the glycerin derivative-modified silicone.

**13.** The manufacturing method according to any one of claims 1 to 12, wherein the mixture containing the glycerin derivative-modified silicone and the impurities are treated by an acidic aqueous solution, and water and odor-causing substances produced by treatment with the acidic aqueous solution are removed by heating or depressurization.

**14.** An external use preparation, a cosmetic, or an industrial material containing the high-purity glycerin derivative-modified silicone obtained by the manufacturing method of any one of claims 1 to 13.

## INTERNATIONAL SEARCH REPORT

International application No.
PCT/JP2013/085005

A. CLASSIFICATION OF SUBJECT MATTER
*C08G77/34*(2006.01)i, *C08G77/38*(2006.01)i, *A61K8/89*(2006.01)i

According to International Patent Classification (IPC) or to both national classification and IPC

B. FIELDS SEARCHED

Minimum documentation searched (classification system followed by classification symbols)
C08G77/00-77/62, C07F7/02-7/21, A61K8/00-8/99

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2014 |
| Kokai Jitsuyo Shinan Koho | 1971-2014 | Toroku Jitsuyo Shinan Koho | 1994-2014 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| Y | JP 11-181096 A (Dow Corning Corp.), 06 July 1999 (06.07.1999), claims; paragraph [0012]; examples & US 5789612 A & EP 905171 A1 | 1-13 |
| Y | JP 11-240951 A (Dow Corning Corp.), 07 September 1999 (07.09.1999), claims; paragraph [0012]; examples & US 5847180 A & EP 924238 A1 | 1-13 |
| X<br>Y | WO 2002/055588 A1 (Shin-Etsu Chemical Co., Ltd.), 18 July 2002 (18.07.2002), claims & US 2003/0158363 A1 | 14<br>1-13 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 April, 2014 (01.04.14) | 22 April, 2014 (22.04.14) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2013/085005

C (Continuation). DOCUMENTS CONSIDERED TO BE RELEVANT

| Category* | Citation of document, with indication, where appropriate, of the relevant passages | Relevant to claim No. |
|---|---|---|
| A | US 2383521 A (Sowa Frank J.), 28 August 1945 (28.08.1945), claims (Family: none) | 1-14 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2012289017 A
- JP S6234039 A
- JP S62195389 A
- JP 2583412 B
- JP H06089147 B
- JP 1956013 B
- JP 2613124 B
- JP H04188795A B
- JP 2844453 B
- JP H02228958 A
- JP 3976226 B
- JP 2002179798 A
- JP 4485134 B
- JP 2004339244 A
- JP 5037782 B
- JP 2005042097 A
- JP 4357909 B
- JP 2005089494 A
- JP S63202629 A
- JP 3172787 B
- JP H05186596 A
- JP H05156019 A
- WO 2002055588 A
- WO 2011049248 A
- WO 2011049247 A
- WO 2011049246 A
- JP 2012046507 A